# EUROPEAN PATENT SPECIFICATION

(11) **EP 3 038 516 B1**
(45) Date of publication and mention of the grant of the patent: **31.01.2024**
(21) Application number: 14748098.2
(22) Date of filing: 30.06.2014
(51) Int. Cl.: A61B 5/361, A61B 5/332

(54) **LIVE HOLTER**
ÜBERWACHUNGSVORRICHTUNG
HOLTER DIRECT

(30) Priority: 29.06.2013 CZ 20130513; 18.10.2013 CZ 20130808; 18.12.2013 CZ 20131031; 15.05.2014 CZ 20140338
(43) Date of publication of application: 06.07.2016
(73) Proprietor: Kranz, Vladimir, 14700 Praha 4 (CZ)
(72) Inventor: Kranz, Vladimir, 14700 Praha 4 (CZ)
(86) International application number: PCT/CZ2014/000072
(87) International publication number: WO 2014/206382

(56) References cited:
- WO-A1-2004/038942
- WO-A2-2012/022276
- GB-A- 2 452 158
- US-A1- 2011 270 112

## Description

### Technical Field

Subject of invention is holter.

### Background Art

The American engineer Norman J. Holter is author of Holter Register of Heart Signals within 24 hours, shortly called holter. Holters are used for more than 20 years and in the frame of current status of technology they are the electronic units which the monitored person has borrowed for certain time, usually from one day until a week, which are not making possible long distance transmission of data which can be read only after its giving back, mostly namely by cable to PC. During sensing of data there is no view on data either of doctors or monitored person. Course of curve of pulsation frequencies is not projected by normal pulse limits neither curve and/or stage of arrhythmia. Holters require glued electrodes which are not esthetic or comfortable for wearing especially because the wires leading to them are loosely hanging on chest.

The unit itself is in the form of box which is fastened on chest by glue band or is hung on neck or side whereas the wires of leads are led to it, loosely hanging on chest which is not esthetic and practical. Holters are of large dimensions and expensive. 20 years after discovery of holter revolutionary breakthrough comes in its development and applicant of this patent presents new solution " Live Holter" described in Disclosure of Invention.

### Disclosure of Invention

The indicated imperfections of similar known concepts eliminates solution accordingly the invention in which holter is adapted for long distance transmission of data wireless to evaluation block and/or locally to control module where the view has not only personnel and professional, especially medical staff but also patient. Holter is adapted for permanent monitoring of monitored and sensed data and figures and data are transmitted without interruption of monitoring.

Holter is adapted for live projection or from record, locally or from distance, not only curves of ECG but also curve of pulsation frequency, with arrhythmia limits, with values of degrees and curve of arrhythmia which is beneficial for monitored person who, by observation of indicated data especially live, has feedback for his subsequent physical activity which can preferably adjust to optimum for mineralization of arrhythmia, and thus to achieve prevention effect, therefore avoiding of serving of medicaments and/or non-increasing of dosage of medicaments and/or to fix dosage in optimal way.

Checking personnel and monitored person, due to the uninterrupted monitoring, know exactly when monitored person has arrhythmia and of what kind and can set optimum dosage of antiarrhythmic and colagual medicaments for blood solution.

Holter is adjusted for screening of heart signals by means of chest strap of relatively small dimensions which preferably does not contain glued electrodes in case that multilead ECG is not applied, its applying is easy, comfortable and esthetic, contrary glued electrodes. These are used only for increasing of number leads of chest strap when it is needed. Chest strap is connectable with displaying module preferably wireless but with possibility of wire connection. Holter is adapted for transmission of data by wireless communication with remote units preferably by means of network of mobile operator, especially GSM, 3G, LTE and/or WIFI, to evaluation block preferably formed by server and/or PC participants and further to control module by wireless transmission for communication with near units especially accordingly standards BT, BLE, ANT, in band 5,3 kHz, 2,4 GHz and/or data are transmitted by SD card or downloading by means of wire connection and/or transmission of figures from display by means of MMS.

Holter is equipped by display for monitoring of data, live or recorded, by checking personnel and preferably also by monitored person for whom is display optionally placed preferably on control module, preferably placed on wrist band with relatively small display for immediate monitoring of data and/or on standard projecting module, preferably consisted of mobile phone and/or tablet and/or PC with relatively larger display for detail monitoring of data. Checking personnel follows data and curves on PC of evaluation block.

Holter is formed by basic blocks, modules and units which are performing its functions. Chest strap has contact areas on it and preferably further contacts of external electrodes, par example glued, which are used only in case that multilead ECG is necessary than the contact areas of electrodes on chest strap are making possible. Chest strap is sensing heart signals and is wireless connected preferably by means of bluetooth with control module where is transmitting it in digital form. There they are further processed for displaying on display of holter. Control module is preferably placed on wrist band of holter and is of relatively small dimensions, than standard mobile phone for to be comfortably worn on wrist, with respective relatively smaller display, suitable for immediate observation of data and curves. Control module is wireless connected preferably by bluetooth with displaying module, preferably formed by mobile phone standard, where on the relatively larger display are monitored data and curves in details. With evaluation module which preferably consists of server and/or PC, is control module connected preferably by means of network of mobile operator and/or WIFI. On display is preferably projected ECG, arrhythmia in curves or level of arrhythmia and/or % of irregular pulses of total number of pulses for certain time period, variability and further data and curves. Chest strap is preferably controlled from control module and/or displaying module. In case of exceeding of set limits of chosen parameters holter sends warning signal from selected unit which preferably can be reset by reset button otherwise the data that exceeded limits are transmitted to evaluation block. Control module checks also standard mobile phone in displaying module standard and preferably is downloading out of it data and displays them as par example number of calling subscriber and it is preferably adapted for receiving of call or SMS message which displays preferably. Therefore holter becomes universal, with possibility to ensure also phone or data operation like standard mobile phone.

It comprises of set of modules, blocks, units and parts optionally usable for chosen functions of Live Holter. Basic function secures holter for basic functions with monitor of heart signals general with external electrodes and memory - SD card. This holter is able to be completed by chest strap general and elements of evaluation device i.e. control module general, displaying module and evaluation block. Accordingly choice is optionally adjustable holter for communication with control module with displaying element or holter for communication also with near cooperating units, completion of displaying module. Further optional enlargement for evaluation block is achievable structure of holter for communication between remote cooperating units. Communication between chosen groups runs live or from record, wireless, connections medias for remote and also for near communication SW of holter is unified in all units and supports this communication in all selected combinations.

Control module consists of control unit or mobile phone adapted or auxiliary device with mobile phone standard preferably of smaller dimensions on wrist band for emergency monitoring of data, curves and further data.

With preference it communicates by means or wireless connection with displaying module which is provided by larger display for detail projection. Displaying module preferably consists of standard devices as it is mobile phone and/or tablet and/or PC.

Control module is independent and to control of chest strap does not need further units. In this case is provided with sufficiently large display of holter for simultaneous projection but preferably it is not fastened on wrist band for to be able to apply larger dimensions of display.

Also displaying module alternatively functions independently, as operation without control module and preferably communicates directly with chest strap by wireless connection of holter and in case that displaying module is formed by mobile phone or tablet especially when data are processed on background of SW program for operation of mobile phone or tablet, it is preferably used maxi chest strap with monitor of heart signals where the signals are completely processed and then in displaying module are only projected and controlled additionally. Alternatively data are processed in displaying module but less data processed in chest strap, result of displaying module formed by standard device is less exact, because simultaneously effects also other programs , par example for serving of telephone operation and further applications, having currently preference. Control module and displaying module are preferably mutually controlled and displayed and displaying module is preferably adapted for control of chest strap. Control module preferably operatively displays data of displaying module, especially when formed by mobile phone, par example data of phone calls and SMS which can be taken over and have telephone call or display SMS, telephone number or further data.

Holter is usable with maxi chest strap and monitor of heart signals and sensors. Processing of analogue heart signals and preferably signals and data of sensors is made in module "front end". After digitalization of heart signals in monitor it comes by means of communication unit of chest strap to wireless transmission in mini of control module provided preferably by display. This control module is preferably formed by mini control unit preferably provided with display. Communication unit receives data and in digital form is passing over them to control unit which works for touch display. From there it is possible to control maxi chest strap. Mini control module can be preferably further formed by mobile phone adapted or auxiliary device with mobile phone standard. In maxi chest strap is placed removable memory SD card for record of data of monitor which is possible to replay/process/evaluate in PC of evaluation block.

Displaying module is preferably formed by mobile phone and/or tablet and/or PC and communicates wireless with communication units preferably through standard mobile phone and/or WIFI also with evaluation block preferably formed by server and PC operator and PC participants.

Holter with maxi chest strap with monitor of heart signals and sensors preferably in unit of electronics for processing of analogue heart signals and preferably signals and data of sensors which are processed in module " front end" and after digitalization processed in monitor of heart signals and preferably sensors form which communication unit of chest strap sends them wireless to mini control module adapted preferably by display which is preferably formed by mini control unit provided preferably with display, communication unit receiving data in digital form which is handling over them to control unit which processes them for touch display from which is possible to control chest strap. Mini control module can be preferably further formed by mobile phone adapted or auxiliary device with mobile phone standard.

In maxi chest strap is placed removable memory SD card for record of data of monitor which is possible to replay in PC of evaluation block.

Holter is usable with mini chest strap and without ECG monitor where heart signals and preferably signals of sensors are processed in "front end" of ECG unit and "front end" of sensor unit. The signals processed in such a way are led to digitalization unit and afterwards just in digital form sent wireless preferably bluetooth from communication unit to cooperating communication unit in maxi control module. This is preferably formed by maxi control unit provided preferably with display with ECG monitor of heart signals preferably placed in unit of electronics. There are alternatively brought also signals of heart signals and/or from sensors which are processed in "front end" unit and ECG and "front end" unit of sensors and further then in monitor of heart signals of sensors with control unit and microprocessor. In maxi control unit is also placed removable SD card for replay of data of monitor to PC in evaluation block. Sensors and electrodes are connectable by wire directly with ECG monitor respectively with its " front end " blocks.

Holter in execution of mini chest strap is without ECG monitor where heart signals and preferably signals of sensors are processed in "front end" unit of ECG and "front end" unit of sensors and led to digitalization unit and in digital form sent wireless preferably bluetooth from communication unit to cooperating communication unit in maxi control module preferably formed by maxi control unit adapted preferably with display with ECG monitor of heart signals preferably placed in unit of electronics. There are alternatively brought also heart signals and/or signals from sensors which are processed in "front end" unit and ECG and "front end" unit of sensors and further then in monitor of heart signals of sensors with control unit and microprocessor. In maxi control unit there is also placed removable SD card for replay of data of monitor to PC in evaluation block.

Control unit with module of auxiliary units preferably contains module of auxiliary units which contains monitor of heart signals and enables monitoring of heart signals and also quantities for which is adapted. Control unit of control unit is data interconnected with control unit of control module of auxiliary units.

Preferably is chest strap completed by electronics for evaluation of pulsation frequency and/or ECG and/or variability meter and two couples of contact areas for continual sensing of electric activity of heart muscle. Sensed physiologic and bioelectric signals are led preferably to two independent control units which are comparing continuously results and in case of detection of difference, contrary set limits are activating source of sound signal preferably speaker or vibrator or piezo changer, for sending of warning signal. Signals are further wireless transmitted to further processing and graphic imaging preferably to mobile phone standard by wireless connection preferably transmitter 5,5 5Hz and/or bluetooth and/or wireless connection 2,4 GHz. With preference it is also possible to transmit results to watch with receiver 5,5 kHz.

With further preference the results obtained in one control unit are transmitted in block of electronics of auxiliary device and results obtained in second control unit are transmitted to mobile phone and consequently both results are compared preferably in block of electronics of auxiliary device and/or in electronics of mobile phone with auxiliary functions. Control unit of chest strap preferably fastened on wrist band, contains buttons and display is reserved for control of electronics of chest strap and projection. If values of valuated parameters exceed selected limit, warning will be activated by starting of vibrator and/or source of sound signal. Advantage of both independent complete systems valuating heart pulsation and/or ECG is that system is not only backed up which is important for monitoring of vital functions but by comparison of them it is possible to verify resulting values or curves processed from physiologic and bioelectric signals.

Monitor of heart signals besides other things preferably contains reset button, panic button, unit of wireless communication, comparison unit and is communicating by means of wireless connection 5,5kHz or wireless connection 2,4GHz with watch equipped with control unit of watch and wrist band. Monitor communicates with chest strap and preferably contains unit of strap sensors which preferably contains besides other things breath sensor and sensor of body temperature.

Monitor communicates preferably with mobile phone through wireless connection in band of 5,5 kHz for receiving of signals of heart pulses or through bluetooth or ANT. Sensed physiologic or bioelectric signals from contact areas are led into control unit which is evaluating signals and in case of their deviation out of set limits is sending warning signal preferably through source of sound signal or some of near cooperating unit. Alternatively are physiologic and bioelectric signals preferably sensed from two contact areas and are led preferably into two independent control units which ensure their independent processing and results are currently compared and in case of difference revelation and/or their deviation out of adjustable standard are activating warning signal preferably by means of warning signal preferably acoustic of speaker, of some of kinds of acoustic changer and/or vibration of vibrator and/or light of light source or wireless connection through cooperating units preferably formed of watch, mobile phone with auxiliary functions, auxiliary device with mobile phone standard, control unit and in case that selected cooperating unit preferably is transmitting alarm to selectable remote unit preferably formed by server and/or suitable device at other participants preferably PC or mobile phone standard.

The results of minimum one control unit are preferably transmitted by wireless connection for graphic and numeric processing and projection into nearby cooperating units of mobile phone standard and/or module of auxiliary units in auxiliary device connected with mobile phone standard and/or watch and/or PC and/or projecting and control units preferably on wrist band and/or bluetooth low energy and/or wireless connection 2,4 GHz and/or wireless connection 5,5 kHz and/or ANT and/or WIFI whereas projection on indicated units preferably includes values of arrhythmia and/or variability and/or pulsation frequency and/or other medical data with respective curves and numeric values preferably ECG, preferably in control units evaluated and processed of heart signals and respectively in indicated units further processed for projection.

Further alternative is to transmit results obtained in one control unit par example control unit, through one communication channel, by means of wireless connection, into module of auxiliary units preferably into unit of wireless communication preferably formed by bluetooth unit and results obtained in the second control unit par example to transmit by the second channel into mobile phone standard preferably into unit of wireless communication preferably formed by bluetooth unit and subsequently preferably to compare both results preferably in module of auxiliary units of auxiliary device and/or mobile phone standard and to display preferably the results independently and in case of disharmony to initiate warning signal . Alternatively are used signals of both channels in indicated units independently without comparison as backup when one of them falls out or in simplified form is preferably used only one channel and only this one is processed and displayed. Also alternatively is preferably used only one couple of contact areas led into two control units therefore backup is going on just from them. Preferably monitor is together connectable units, especially mobile phone and/or auxiliary device adapted for full backup, processing and projection of physiologic and bioelectric signals preferably heart ones as par example curve and value of pulses, ECG arrhythmia and other ones - from contact areas, electrodes just up to display with projection of both channels independently and the whole system is doubled whereas mobile phone standard with display is doubled by module of auxiliary units and electronics of mobile phone working independently or is doubled in module of auxiliary units with two or more independently working microprocessors or alternatively system is backed up partially when only one part of it is doubled or is not backed up and monitor of heart signals is provided with only one couple of contact areas and one control unit.

Control unit is preferably fastened on wrist band and contains buttons, unit for data processing and display for data projection preferably of respective curves including ECG and which one, besides processing and projection, is set preferably for control of monitor of heart signals and forms with it the independent complex working without another units. With preference the control unit is of small dimensions and has only emergency functions necessary for operation of monitor of heart signals and simple projection contrary to mobile phone with auxiliary functions having more functions and detailed projection.

If the values of evaluated parameters exceed some of set limits, preferably is activated warning by starting of vibrator and/or source of sound signal preferably speaker or piezo changer in monitor of heart signals and/or option preferably in some of nearby cooperating units. Preferably in case that the warning signal is not reset by reset button on monitor of heart signals or on further nearby cooperating units until adjustable time, alarm is started which is preferably transmitted to remote cooperating units preferably surveillance center or other participants, by data or phone.

With preference monitor of heart signals functions independently without mobile phone with auxiliary functions or without auxiliary device with mobile phone standard whereas is operated preferably by buttons and/or further ones and in its control unit there are processed the indicated signals which in case of deviation out of set limits are setting off warning signal generated by source of sound signal or vibration by means of vibrator. In case that warning signal is not reset by reset button in adjustable time, monitor of heart signals is launching alarm through network of mobile operator to remote cooperating units. Warning signal is adjustable and preferably coded and thus is informing about the kind of alarm and how is caused. Setting of functions of monitor of heart signals is preferably made by control buttons on monitor of heart signals without necessity of connection of other device or control unit, par example mobile phone. In such a case monitor of heart signals informs users about values of measured quantities by sound signals by means of speaker or acoustic changer, as indicated above.

In case of connection of auxiliary device the operation is made by buttons or touch display of auxiliary device which preferably displays the curves and values on display preferably independently on projection on mobile phone standard for parallel projection on display and/or backup. Monitor of heart signals preferably contains display for projection of data in numeric form and/or in form of curves for projection of the most important data, preferably arrhythmia, heart pulsation, ECG, variability and other data and/or touch display for operation and projection, preferably of curves of pulsation, arrhythmia, ECG and other ones. With preference monitor of heart signals contains WATCHDOG watching its correct activity and launching alarm, in case that some of control units fails and does not send "refresh signal". Monitor of heart signals preferably observes correct activity of mobile phone with auxiliary functions and/or control unit and/or watch for sportsman and other units in such a way that indicated units are sending by wireless connection optional code or result of set task preferably mathematic into monitor of heart signals. In case that code or result is correct, control unit will send "refresh signal" to "watchdog". If this does not come in adjustable time or is not correct, monitor of heart signals will not send refresh signal and alarm is started, initiated in watchdog. Alternatively monitor of heart signals sets off alarm independently on watchdog in case that does not receive correct code. Signal lights or kind of acoustic and/or vibration alarm indicate cause of alarm. Besides they signal preferably level of charging of battery, heart pulses and further pieces of information.

Memory card, preferably removable, with preference records data of monitor of heart signals which can be brought to PC or another suitable electronic device with display preferably by its transfer to reading device of these electronic devices or by means of wireless connection or USB interconnection by means of connection. In such a way monitor of heart signals is adapted for being used as "Holter" with preference of non-interrupted monitoring simultaneously preferably for live evaluation on display and further data as pulsation and/or arrhythmia and/or variability in numeric form or expressed by curve and/or ECG, preferably it may be used instead of memory card in monitor of heart signals the memory card in auxiliary device and/or mobile phone standard and/or mobile phone with auxiliary functions.

Monitor of heart signals is preferably on chest strap or out of it preferably on wrist band or in module of auxiliary units of mobile phone with auxiliary functions or in module of auxiliary units and electrodes ECG preferably glued are connected cables on monitor of heart signals in sufficient number for ECG sensed in it would be preferably optionally adjustable for 1 to 12 leads and would be backed up. Connection to the remote units as server, par example, is realized preferably by means of network of mobile operator. To server there is preferably access from surveillance center and further participants. Connection through network of mobile operator has auxiliary device and mobile phone preferably independently for backup.

Alternatively there is used, instead of system of mobile phone standard with auxiliary device or simultaneously with it, mobile phone with auxiliary functions which has same functions as system and replaces it in full. Advantage of system comprises in fact that auxiliary device is removable and is applied in case of need and only with such kind of auxiliary where its function fulfils momentary need and in equipment preferably with optional sensors from set of auxiliary device thus mobile phone standard is enlarged for optional function only in case of need, it means that mobile phone is of smaller dimensions and less expensive. Auxiliary device contains module of auxiliary units, is removable with it out of mobile phone standard whereas in mobile phone with auxiliary functions there is module of auxiliary units firmly built in. Electronics of mobile phone with auxiliary functions is contained also in mobile phone standard as electronics of mobile phone standard with the same functions. Auxiliary device with mobile phone standard preferably contains unit of wireless communication placed in module of auxiliary devices, preferably formed by unit bluetooth or ANT for communication of nearby cooperating units.

Communication through network of mobile operator ensures connection of nearby cooperating units which are monitor of heart signals, mobile phone with auxiliary functions , mobile phone standard with auxiliary device and control unit, with remote cooperating unit preferably with server, on which are stored pieces of information, connected preferably on PC of operator, whereas pieces of information are accessible to remote cooperating units and authorized participants as par example to medical staff, relatives and other ones and preferably to surveillance center. Indicated communication is enabled by communication module with components for data and/or voice communication by means of network of mobile operator.

Mobile operator with auxiliary functions preferably on wrist band, of smaller dimensions than mobile phone with auxiliary functions, without band, communicating with monitor of heart signals, preferably simplified without backup. For backup mobile phone with auxiliary functions preferably communicates with monitor of heart signals on two channels by means of communication wireless connection on module of auxiliary units and wireless connection to electronics of mobile phone. Backed up communication is possible alternatively, instead of communication with module of auxiliary devices and also with electronics of mobile phone realize only with module of auxiliary devices where the second channel of wireless connection is preferably processed by the second microprocessor in control unit of module of auxiliary devices. Mobile phone with auxiliary functions on band of smaller dimensions processes data in control unit of module of auxiliary devices separately from control unit of mobile phone and therefore working reliably and with preference passes processed data to mobile phone standard, of bigger dimensions, which ensures detail projection on larger display than mobile phone of auxiliary devices on wrist band. Preferably mobile phone with auxiliary functions on wrist enables operative immediate view on smaller display and on mobile phone standard the detail view on larger display which is not immediate in case it is put, par example in pocket. Mobile phone with auxiliary functions on band ensures reliability of data processing and immediate view on emergency smaller display, projection with detail view ensures bigger mobile phone for detail projection. Mobile phone with auxiliary functions works independently on mobile phone for detail projection even when this is not connected. In case of non-backed up communication only on one channel of wireless connection is alternatively instead of monitor of heart signal classic, used monitor of heart signals in economic execution which is not adapted for backup and therefore it is simplier and smaller, with lower consumption of energy.

Mobile phone with auxiliary functions preferably displays optional data of mobile phone for detail projection, par example data about incoming calls or SMS which preferably takes over. Similarly is preferably for described communication with mobile phone for detail projection in case of monitor of heart signals used standard mobile phone with auxiliary device or displaying and control unit in case of connection of displaying module preferably formed by mobile phone, due to economic and dimensional reasons mobile phone or control unit or system preferably are not equipped by parts for communication with mobile operator , they are therefore smaller and less expensive and for communication in network of mobile operator is preferably used mobile phone which preferably forms displaying module.

Connection between mobile phone with auxiliary functions or system is not backed up and connection by means of wireless connection is only with module of auxiliary units which are processing data and send them into electronics of mobile phone from where are transmitted to mobile phone for detail projection or connection is effected by means of wireless connection on control unit and from it directly to mobile phone.

One of substantial parts of holter accordingly the invention represents and some imperfections of known status of technology eliminates mobile phone with auxiliary functions which is enabling contrary existing status of technology without increase of thickness of mobile phone the auxiliary functions as it is easy replacing of basic accumulator of mobile phone , through which necessity to charge mobile phone is removed, preferably by means of mechanism for replacement of accumulator which is possible preferably to realize within operation of mobile phone and thus to ensure its uninterrupted operation preferably by means of source of bridging energy preferably formed by bridging battery or condenser or accumulator and bridging electronics. Further important auxiliary function is preferably data and applications in block of auxiliary modules processing, independent on unit of electronics of mobile phone. With preference the results are displayed on auxiliary display, independent on display of mobile phone and/or part of "split screen" of module of units and results from electronics of mobile phone on part split screen electronics of mobile phone and/or alternatively are gradually displayed, once of electronics of mobile phone and afterwards of module of auxiliary units of mobile phone on display of mobile phone. Interface divides both parts of "split screen".

Bridging accumulator is preferably of substantively smaller dimensions and with lower capacity that the basic one but sufficient for keeping of operation of mobile phone during the replacement of basic accumulator when relatively small source of bridging energy backs up operation of mobile phone only for the time of replacing of basic accumulator, through which the many times smaller dimensions of mobile phone are achieved, contrary to execution with auxiliary accumulator securing not only bridging but also operation of mobile phone and/or charging of basic accumulator and thickness of mobile phone will not be increased because to the basic accumulator is not added auxiliary accumulator about the basis accumulator of devise is replaced. Basic accumulator preferably with substantively higher capacity than bridging accumulator one corresponding to current accumulator par example in mobile phone, is charging bridging accumulator if it has enough of energy whereas is easily and quickly replaceable for other one preferably from the set of accumulators charged in advance in group charger and namely thanks to storing mechanism of accumulator preferably enabling to place the same basic mechanism in mobile phone and charger as well. Replacement of accumulators in mobile phone with auxiliary functions takes only some seconds preferably during operation and without removal of cover, cotrary to existing technology where lengthy replacement under condition of interruption of operation and necessity to activate again mobile functions with renewal of respective application is on the top of it, burdened especially by necessity to remove at first cover of accumulator and then to reinstall it.

Alternatively mobile phone is preferably fastened by band of mobile phone forming wrist band for fastening of mobile phone to wrist. Contrary to mobile phone which is not fastened on wrist, mobile phone which is fastened on wrist, preferably of smaller dimensions and is set for operative monitoring of display on wrist, above all.

Basic accumulator of bigger dimensions than is bridging one, which is being replaced preferably for charged one, instead of charging , preferably during operation, thanks to source of bridging energy, which supplies energy to electronics of mobile phone and module of auxiliary units from supply module of central electronics of mobile phone during replacement of basic accumulator, is preferably for supply of bridging energy in time of replacement of basic accumulator, connectible charger modular or external or source of bridging energy or casing which source of bridging energy preferably replaces. With preference it is then instead of internal source of bridging energy for replacement of auxiliary accumulator usable external source of bridging energy, in such a case bridging accumulator with internal source preferably drops.

Connection of module of auxiliary units and electronics with external devices is preferably realized wireless by means of wireless connections formed by bluetooth or by wire through connector for such connection of module and/or through connector for data connection of electronics of mobile phone.

Independent processing of data of block of auxiliary modules on unit of electronics of mobile phone is realized preferably by placing of minimum one processor, working independently on electronics of mobile phone, at control unit in module of auxiliary units in block of auxiliary modules communicating with data connected by independent connection or independent connector to connector for electronics of mobile phone which is working independently as well. By such a mean the reliable operation is to be reached and processing of applications and coming data processing in independently working module of auxiliary units and less reliable processing of applications and data in electronics of mobile phone is to be removed, which is done because more programs are simultaneously processed, especially preferentially the programs securing operation of mobile phone. In case that does not depend on reliable processing of data and applications, preferably is not applied module of auxiliary units and the described processing of applications and data is realized in electronics of mobile phone where these applications and data are led directly.

With preference the mobile phone is used, with auxiliary functions with mechanism for replacement of basic accumulator and block of auxiliary modules for realization of these auxiliary functions, especially easy removal of basic accumulator without necessity of cover removal of mobile phone or auxiliary device preferably without interruption of operation and preferably on unit of electronics of mobile phone independent on processing of data and applications in block of auxiliary modules. Preferably the results are displayed on auxiliary display, independent on display of mobile phone and/or on part of "split screen" of module of units and results of electronics of mobile phone on part of "split screen" of electronics of mobile phone and/or alternatively are projected gradually, by one of electronics of mobile phone and afterwards of module of auxiliary units of mobile phone, on display of mobile phone. Interface separates both parts of "split screen".

Mobile phone with auxiliary functions contains electronics of mobile phone with control unit with microprocessor of electronics of mobile phone, mechanism for replacement of auxiliary accumulators with tiltable casing, closure of door and contacts for connection of accumulator and block of auxiliary modules including module of supply of electronics of mobile phone source of bridging energy, preferably formed by bridging accumulator and module of auxiliary units of mobile phone with control unit with microprocessor. Interconnection of modules and units is realized by connections of data and connections of voltage. Alternatively there is preferably fastened to mobile phone band of mobile phone forming wrist band for fastening of mobile phone on wrist. There is used source of bridging energy preferably of bridging condenser or accumulator which is preferably of smaller dimensions contrary basic accumulator because it secures operation of mobile phone only for a short time when replacement of basic accumulator takes place. The herein generalized term Source of operation energy is basic accumulator which is preferably with higher capacity amp/hour and of bigger dimensions as bridging accumulator, secures operation of mobile phone and is replaced for charged one preferably during operation, thanks to source of bridging energy, which supplies energy to electronics of mobile phone and module of auxiliary units via supplying module of supply to electronics of mobile phone during the replacement of basic accumulator. For saving of space for source of bridging energy preferably formed bridging accumulator, by connector and cable of charging, is connected charger or modular charger or external source of bridging energy or preferably casing with charged accumulator which source of bridging energy preferably replaces. With preference it is then instead of internal source of bridging energy placed in mechanism for replacing of auxiliary accumulator used external source of bridging energy connected externally by cable and connector. In such a case bridging accumulator falls off. Data connection of module of auxiliary units and electronics with external devices is preferably realized wireless by means of wireless connections preferably formed by bluetooth or through connector for data connection of module and through connector for data connection of electronics of mobile phone. Independent processing of data of block of auxiliary module on unit of electronics of mobile phone is done, preferably by placing of minimum one microprocessor to electronics of mobile phone, working independently, to control unit in module of auxiliary units in block of auxiliary modules that communicates with data connected by independent wireless connection or independent connector on connector for electronics of mobile phone, operating independently as well.

One of substantial parts of holter accordingly invention represents and some imperfections of current status of technology by its using, removes module of auxiliary units with independent control unit working preferably independently on control unit of mobile phone. It contains internal monitoring units in block of monitoring units internal and enables connect external monitoring units in module of monitoring units external via interface connector and/or electronic. Internal units preferably include unit of wireless communication, unit of watchdog checking continuously trouble-free operation, unit of detection of position containing preferably acceleration meter or gyroscope, further units forming block of monitoring units internal, including unit of testing of health functions , receiver of pulses 5,5 kHz, unit ECG preferably processing heart signals on ECG curve and/or curve of pulsation and/or curve of arrhythmia and/or calculated degree of arrhythmia, variability, monitor of heart signals, unit of alcohol test and/or drugs and other units.

Via interface connector and/or electronic are connected modules of monitoring units external which preferably include probe of health functions, unit of testing of health functions external, further unit of alcohol test and/or drugs external with probe, units of detectors external. Further advantage is connection of electrodes and sensors placed on wheel of car through connector ECG enabling continuous monitoring of health conditions of driver preferably of driver of public traffic, buses and lorries.

Reliable processing of data or applications in module of auxiliary units is given by use of minimum one independently working microprocessor, on electronics of mobile phone, control unit placed in module of auxiliary units which has data and signals led directly from monitoring units external or internal independently on bringing of data into electronics of mobile phone. Electronics of mobile phone does not guarantee reliable processing of data because simultaneously more programs and applications especially programs and application for operation of mobile phone which are preferential.

Module of auxiliary units contains watchdog which produces independent warning signal in case of dysfunction of mobile phone.

Module of auxiliary units preferably processes data from individual monitoring units, sensors and detectors which is economic because in existing status of technology these data are processed in individual monitoring units individually where in every unit there is display and microprocessor, while in module of auxiliary units is used display preferably of mobile phone and independently working microprocessor together for all monitoring units.

In case that is control unit preferably adapted for direct communication with module of monitoring units internal or external, preferably is this communication and processing realized in electronics of mobile phone. In this case module of auxiliary units is not preferably applied which is economic but less reliable. To module of auxiliary unit is preferably connected also unit of alcohol test and/or drugs external or internal which is especially advantageous in case that simultaneously are used contacts or sensors on wheel of car so continuously health conditions of driver are tested. In case that the results are shown, which are not supporting good conditions of driver, driver preferably will use chest strap or electrodes, preferably glued, by which is sensed ECG, curve of pulsation, arrhythmia, variability more exactly than from contacts on wheel, by hand touch. Device will enable to check not only arrhythmia, ECG and variability but also it is possible to do test for drugs and alcohol. This is advantage especially in case of public traffic drivers or drivers of lorries. Auxiliary device with block of auxiliary modules and module of auxiliary units with mobile phone standard or in case that it contains unit of wireless communication and/or communication module also without standard phone is preferably built in transport mean in which takes place monitoring of person driving the mean and which communicates with cooperating units nearby and/or remote ones. Therefore falls off wearing of auxiliary device with standard phone or phone with auxiliary functions also in case of monitored person. In such a case monitored person wears only chest strap or electrodes for case of necessity of exact sensing of ECG or further physiologic signals. Preferably are strap and/or electrodes and/or sensors especially of alcohol testing and/or drugs also preferably built-in in transport mean and preferably connected by sufficiently long cable with auxiliary device in such a way for to be accessible for test or are loosely set preferably with wireless connection.

Module of auxiliary units of auxiliary device to mobile phone standard with auxiliary functions is provided with the same units and functions as module of auxiliary units of mobile phone described above, with difference, that connector of module of auxiliary units enables connection to mobile phone, the second connector of module of auxiliary units ensures connection of external devices and the third connector of module of auxiliary units connection to further external devices.

Alternatively the bridging energy is obtained of module of charging by stand by condenser.

Module of auxiliary devices contains unit of wireless communication and/or bluetooth and/or ANT and/or WIFI and/or network of mobile operator and other suitable wireless medias, further module of auxiliary units contains watchdog which is producing warning signal in case of disfunction of mobile phone, unit of detection of position preferably acceleration meter or gyroscope, control unit with minimum one microprocessor which is adapted to function independently on microprocessor of mobile phone and is adapted on independent and parallel processing not only of applications processed in electronics of mobile phone but also further applications preferably medical ones, module of auxiliary units further contains receiver of pulses 5,5 kHz and other electronic units internal preferably adapted for processing of medical applications. ECG unit processes heart signal from electrodes external, placed preferably on chest strap or glued and connected by wire by connector of ECG connection or from electrodes internal, fastened on surface of mobile phone. Alternative is the connection by means of wireless connections and unit of wireless communication to external source of ECG signals, preferably to monitor of heart signals. ECG unit preferably formed by monitor of heart signals preferably processes heart signals on ECG curve and/or pulsation curve and/or curve of arrhythmia and/or calculated degree of arrhythmia, variability and data and signals of detectors placed on surface of mobile phone and/or of detectors external connected via detector of probes. Probes are taking soundings of quantities preferably medical ones, par example doing test on alcohol or drugs, saturation of blood by oxygen, diabetic values, blood solution INR, breath frequency, blood pressure and further functions, accordingly probes attached in connectors. External unit of detectors enlarges functions of internal detectors and is connected through auxiliary connector. Wireless connection inductive preferably 5,5 kHz intermediates connection of module of auxiliary unit's par example with sportsman watch with receiver of pulses preferably on 5, 5 kHz. By wire module of auxiliary units is connected through connector of mobile phone independently on electronics of mobile phone so as also through independent wireless connections. Preferably control unit is adapted for direct communication with module of monitoring units internal and module of monitoring units external and in this case module is realized without control unit. Connection of modules is effected directly into control unit of mobile phone by means of connections of modules of monitoring units and into data connection. Advantage of processing of data in module of auxiliary units is the reliability which is bigger than in case of processing of data in electronics of mobile phone because also function of mobile phone and further applications are processed while module of auxiliary units is processing only selected applications and in case of need is provided with more microprocessors for further increase of reliability. Via interface connector and/or electronic are connected modules of monitoring units external which preferably include probe of medical functions, unit and testing of medical functions external, further units of alcohol test and/or drugs external with probe, unit of detectors external. Further advantage is connection of electrodes and sensors placed on wheel of car through ECG connector enabling continuous monitoring of health conditions of driver, preferably driver of public transport.

For components of holter accordingly invention there is preferably usable principle of mobile phone with auxiliary functions. It is applicable preferably on existing standard mobile phone which has not indicated auxiliary functions, by means of auxiliary device connected to it, when the existing basic accumulator of mobile phone is used for operation, when by means of mechanism for replacement of basic accumulator is easily replaceable for charged one, without removal of cover or auxiliary device of mobile phone in such a way that in auxiliary device which preferably replaces original cover of mobile phone is wall of auxiliary device provided by mechanism for accumulator replacing preferably with door enabling this replacing. After its opening it is possible to remove accumulator by hand and replace it by charged one which is not possible in the frame of existing technology.

Source of bridging energy is preferably used also in auxiliary device which is adapted for its fastening on mobile phone by means of affixing mechanism of auxiliary device. Source of bridging energy for block of electronics of auxiliary device is possible to get either of connector charging and communicating preferably USB mobile phone when it is enabled or from contacts of basic accumulator which is preferred in case that mobile phone does not make possible replacement of basic accumulator not even when there is led respective voltage on its charging connector. In this case at moment of replacing of basic accumulator without interruption of operation source of bridging energy supplies energy directly to mobile phone on contacts for connection of basic accumulator. Switcher, manual or automatic preferably enables switching over of source of bridging energy for time period when the replacement of basic accumulator is not done, from supply of energy to mobile phone on supply of auxiliary device and charging of source of bridging energy from mobile phone, with exception that it is formed by battery. In case of exhausting of energy from contacts of basic accumulator between its contacts and contacts of mobile phone are preferably installed contact springs which are pressed by basic accumulator on contacts of mobile phone and are connected with auxiliary device which supplies together with charging source of bridging energy which is preferably of dimensions as small as possible and has capacity which still is able to ensure replacing of basic accumulator without interruption of operation. Auxiliary device with source of bridging energy, reduced in such a way, is preferably of smaller dimensions than auxiliary device with auxiliary accumulator from which is supplied energy not only during time of basic accumulator replacing but also for operation of mobile phone and charging of basic accumulator and preferably keeps original thickness of mobile phone by realization of auxiliary device where block of auxiliary module sis placed into to place of auxiliary device by which is body of mobile phone only extended or enlarge instead of its placement above it. Source of bridging energy is part of block of auxiliary modules placed in auxiliary device, which is enabling further functions and preferably contains module of auxiliary units with control unit with microprocessor, which is enabling to process applications independently on mobile phone. Energy for auxiliary device and mobile phone for the time of accumulator replacing is preferably supplied by replaceable battery or bridging accumulator which is placed in storing mechanism of bridging accumulator and then is easily replaceable, which is to be replaced for charged one instead of its charging par example from springs on contacts of basic accumulator preferably formed by door, placed in wall of auxiliary device and other executions further described.

Mobile phone standard with auxiliary functions in auxiliary device which is provided with mechanism for replacing of basic accumulator and block of auxiliary modules, for realization of auxiliary functions especially easy removal of basic accumulator without necessity of removal of auxiliary device preferably without break of operation of mobile phone and preferably on unit of electronics of mobile phone independent on processing of data and application is in block of auxiliary modules of auxiliary device. Through connector, the second, of auxiliary device is module of auxiliary units interconnected with external device and through connector, the third, with further external device and module of auxiliary units is adjusted to intermediate communication between external device and mobile phone by which is replaced data communication of mobile phone from connector which is blocked at the moment of putting on of auxiliary device. Auxiliary device is adapted for easy replacement of basic accumulator for the charged one preferably without interruption of operation of mobile phone without enlargement of its thickness. Auxiliary device is connected to mobile phone as and removable contains upper wall which is extending it only as far as length is concern and/or width whereas thickness remains unchanged or is preferably becomes thicker only for wall of auxiliary device which contains source of bridging energy which is preferably formed by bridging accumulator , in this case drawn, or battery or bridging condenser and module of supply of auxiliary device which supplies of basic accumulator through connector of mobile phone , connector of auxiliary device and connection of supply auxiliary device and at the time of replacement of basic accumulator supplies from source of bridging energy mobile phone (standard). Switching over of module of supply of auxiliary device for supply of energy to mobile phone is done by switch. In case of mobile phones which are not enabling supply of energy to auxiliary device through connector of mobile phone and/or replacement of basic accumulator without break of operation even at charging though connector of energy for supply of auxiliary device is preferably obtained of contact springs and bridging cable of basic accumulator, contacts of basic accumulator of which fit on contact spring which by its second side fits on contacts of mobile phone whereas bridging cable is preferably connected on contacts, par example by soldering. Supply of mobile phone at the time of replacement of basic accumulator then preferably goes through connector and bridging cable, module of supply is switched of charging of external source of bridging energy over on supply of mobile phone standard during replacement of basic accumulator, preferably by manual switch or electronic one, automatic operated preferably by switch at the moment of opened door during replacing of basic accumulator interconnected by cable. Alternatively is switch controlled by mobile phone standard through connecter by data signals, which module of auxiliary units processes and operates switch in module of supply.

Supply of mobile phone of auxiliary device is alternatively effected preferably through connection and connectors preferably USB supplying and communicating, in case of mobile phones, that are enabling it after respective switching off. For to save place necessary for external source of bridging energy of charger is preferably, for its replacement, led bridging energy of external source of bridging energy preferably formed by basic accumulator placed preferably par example in charger, interconnected for bridging of replacement of basic accumulator in mobile phone by external bridging cable. Source of bridging energy is preferably formed by bridging accumulator preferably placed in mechanism for replacement of bridging accumulator which is preferably applied for supply of auxiliary device and supply of mobile phone through connectors at time of replacement of basic accumulator. In case that mobile phone does not enable supply of energy through connector of mobile phone, connection for connection of basic accumulator through contacts takes place. Similarly alternatively replacing of source of bridging energy is preferably made, for external source of bridging energy. Auxiliary device contains block of auxiliary modules of auxiliary device with module of auxiliary unites with control unit with microprocessor of block of auxiliary device.

Further substantial part of holter represents using of module of auxiliary units to mobile phone standard accordingly invention. Its substance is that is containing same monitoring units internal, enables connection of external monitoring units like module of units in mobile phone with auxiliary functions. On difference of it however it does not enable using of mobile phone standard.

Module of auxiliary units of auxiliary device is provided by the same units with functions as module of auxiliary units of mobile phone. Connector of module of auxiliary units of auxiliary device enables connection to mobile phone; the second connector of module of auxiliary units secures connection of external devices and the third connector of module of auxiliary units connection to further external devices. Control unit with microprocessor is provided with minimum one microprocessor and is adapted for data processing in application independently on control unit of mobile phone.

With preference control unit is adapted for direct communication with module of monitoring units internal and module of monitoring units external and in such a case is module realized preferably without control unit. Connection of modules is realized directly into data connection of mobile phone through connector of modules of auxiliary units on which the respective sensors are alternatively connected by means of connection instead of connection of control unit. Advantage of data processing in module of auxiliary devices is reliability which is bigger than in case of data processing in electronics of mobile phone, because also function of mobile phone is processing it as well as further applications while module of auxiliary units is processing only selected applications and in case of need is fitted by more microprocessors for further increase of reliability.

Further advantageous arrangement for holter accordingly invention is placing of probes for internal sensors either as parts of surface of mobile phone and/or auxiliary device or they are connectible by wire and/or wireless.

Further advantage is also execution when probes are placed on internal side of garments fitting closely on body which ensures contact with body and simultaneously is their wearing comfortable for monitored person.

Preferable execution is also, in case of probes glued on surface of human body, their connection with mobile phone and/or auxiliary device placed on wrist band by thin conductors in such a way that i tis not disturbing for monitored person and not noticeable by subjects in environ, whereas monitored person has permanent overview thanks to pieces of information displayed on displaying unit and/or on display of mobile phone placed on wrist band. Preferable is also execution when there are external sensors placed on operation devices of means with human service, preferably on wheel of car, handlebar of motorcycle, bike, joystick of aircraft, control levers of transport, building and other machines.

Mobile phone with module of auxiliary devices or auxiliary device with module is adjustable, preferably contains module of internal sensors provided preferably with sensors of body temperature, detector of pressure, detector of sleep phases, light detector preferably with blood oxygenation and detector of acoustic signals. To module is affixed set of external sensors preferably completing internal sensors which are preferably detector of alcohol level, detector of presence of addictive drugs, sensor of body temperature, sensor of breath, detector of pressure, ECG detector, EEG detector, EMG detector i.e. electromyography, detector of sleep phases , light detector preferably with blood oxygenation and sensing of pulsation, detector of acoustic signals preferably completing or replacing module of internal sensors which is connected on sensors placed on mobile phone or outside it, as sensors. With preference there are sensors placed on T-shirt for sensing of signals and values from selected places on upper part of body of monitored person whereas are connected by wire or wireless to module and completing or replacing sensors in set of internal or external sensors and/or are placed on multifunctional chest strap connected by wire or wireless. With preference are sensors and/or external electrodes placed on control device of transport or other mean with human service preferably wheel of car and signals from them are led to mobile phone by wire or wireless connection. With preference they are sensed also signals of ECG led further to detector of ECG.

Watchdog unit will activate alarm signaling preferably acoustic and/or visual and/or vibration with preferably acoustic changer or LED or vibrator if par example mobile phone does not indicate correct code within certain time. In case of watchdog placed in mobile phone with auxiliary functions, this one is programed preferably in such a way that its microprocessor unit effects in set time intervals suitable time undemanding activity, preferably par example product of several numbers and if result is identical with correct result stored in memory then will send refresh signal into timer of watchdog which resets timer measuring permanently time passed since the last reset. If reset does not occur sooner than preset time passes, timer will activate acoustic changer, preferably speaker or piezoelectric changer.

In case of watchdog placed in auxiliary device, mobile phone will send result of calculation in auxiliary device which will evaluate it and if result is correct, will send refresh signal into timer of watchdog.

For activity detection is used watchdog unit placed in auxiliary device which activates acoustic signaling by acoustic changer if mobile phone does not indicate correct course. Mobile phone is programed in such a way that its microprocessor unit effects in set time intervals suitable time undemanding activity, par example product of several numbers and if result is identical with correct result stored in memory then through connector and connector will send code corresponding to release of signal refresh into control unit with microprocessor of auxiliary device signal which after processing of signal resets timer reset for permanent measuring of time passed since the last reset. If reset does not happen sooner than preset time passes, timer will activate acoustic changer (preferably speaker or piezoelectric changer and/or vibrator and/or visual indication preferably with LED. Watchdog placed in mobile phone will receive refresh signal directly from electronics of mobile phone by bridging connection and control unit and USB connectors are not used.

Data produced by monitor of heart signals are displayed on display of monitor of heart signals and/or on display of mobile phone with auxiliary functions and/or auxiliary device with mobile phone and/or watch and/or control unit and/or other units. Displaying part preferably formed by display, in case of numeric projection preferably displays value of arrhythmia, value of variability, value of breath, value of pulsation and cause of alarm if alarm situation occurs. The displaying part is preferably universal and displays course of curves, par example curve of ECG, is with touch control, par example control of sensibility, control of speed of shift, further control of movement filter preferably for calmness, walking run and control og lultiple lead displaying adjustable preferably on display of ECG curve which is adjusting for to see immediately results of setting. With preference it displays curve of pulsation, curve of arrhythmia and curve of variability whereas it is possible to change displaying preferably by touch operation directly on display then it is possible to follow change immediately at the moment of realization and set suitable value. These quantities and/or curves are preferably displayed individually or in groups, on one displaying unit. Value of arrhythmia is preferably expressed in % of irregular pulses out of the total number, for optional time period or in degrees of arrhythmia preferably in values from 1 to 10 taken as 1/10 from above indicated % of irregular pulses or in another adequate scale preferably expressed in words as arrhythmia : none, low, middle, high, very high. As irregular pulses are usually understood these ones which time distance of them exceeds value of 1,15 of distance corresponding to average pulsation set by calculation taken out of adjustable number of last pulses, preferably 3 pulses, for filtration of disturbance mostly to the detriment of exactness of projection.

In execution of holter accordingly invention is used lot of components, modules, sensors and probes. Substantial for sensing and projection of data and values about heart activity are advantageous the arrangements enabling comfortable wearing by monitored person. System (multimedia device, central control unit, auxiliary device) with displaying unit is preferably placed on wrist band, in pocket or on belt in such a way that sensed signals are in system led in necessary number by wire or wireless or in combination, whereas probes are glued , on buckle or clothing part and then pressed to body due to being implemented into surface of monitor, in clothing, belt, strap preferably of flexible material and in combination with metal pressure bands placed vertically to straps for to get double pressure force and then contact in various body positions. By wire interconnection with monitor it is selected track and kind and number of conductors for conduction in such a way for to be as discreet as possible and conductors in no way limiting the monitored person.

Further preference of execution of invention is the sensing of physiologic and bioelectric signals out of external sensors glued and/or placed on T-shirt and/or internal sensors on chest strap and/or directly on mobile phone and/or wrist band and it is enabling their wireless or wire transmission to processing and display on mobile phone and/or auxiliary device.

Monitor is attachable preferably on belt by hanging or in pocket or on wrist band or arm band. Electrodes glued on various parts of body are connected by various cables and to monitor's band. In this case are the electrodes preferably glued on skin or they are taken from chest strap or preferably glued electrodes combined with electrodes on chest strap preferably realized accordingly need for more than 12 lead ECG.

For interconnection of electrodes is preferably used one core cable taken from lower electrode to upper electrode where it is modified in two core cable for easy installation and esthetic arrangement whereas cables are preferably as thin as possible and with transparent isolation, eventually in body color for not to draw attention and not to be psychological obstacle for wearing. To such a purpose is helping wearing of cable in sleeve, thanks to which it is not visible, preferably on right hand because for the first ECG curve it is necessary to place upper electrodes to the right shoulder.

Alternatively the upper electrode is placed as high as possible by practical test of quality of heart signals ideally up to shoulder or slightly behind it and lower electrode as low as possible and on side, ideally on level of belt, thus even by eventual removal of shirt they had not been seen as if they would have been placed on chest. In this case cable is taken preferably along the belt on which it is fastened by buckle and up on side or back to upper electrode and from this place by two core cable to monitor on band again for to reach lower visibility in case of shirt removal. Lower electrode is preferably alternatively, instead of being glued, placed on clasp hung on belt of trousers or skirt or just on them and by which is pressed on body for better contact with skin or alternatively is the lower electrode placed on band on body and cable is preferably led through band and preferably clasp is wearing monitor placed near belt on body and from which the cable from electrode in interconnected with monitor thus preferably falls off cable of two cores because cable is in this case preferably led just into monitor. Adequately preferably alternatively more electrodes are fastened on clasps or belt accordingly the need.

Watch serving besides other thing as display projecting information about heart pulsation and preferably ECG sensed wireless or cable of chest strap, are preferably fastened on wrist.

For to avoid that any cables should be on chest is possible to reach leading of signals by cable from lower glued electrode through belt and later on alongside up to place in area of shoulder to conductor from upper electrode or directly just to upper probe from which they are led together through sleeve to monitor on wrist.

Alternative of glues probes are the probes pressed to skin by flexible parts of clothing, rubber-textile bands namely also in combination with electrodes fastened cross to belts namely on flexible metal bands pressed by rubber-textile bands/belts to body, thus pressure force is doubled and contact with skin preserved in various positions of body of monitored person. Adequately is solved the attachment of probes on shoulder namely by rubber-textile band - suspensor fastened to belt from front and back.

Next advantage of invention is the way enabling by means of holter to follow and instruct sportsman during training in individual phases warm up, performance phase of training and cool down phase. Also it enables to follow curve of pulsation live, during training, as well as after it of stored record. At the organization of training it is preferably chosen regime of automatic observation when trainer will press only once the control element, preferably button start and afterwards training is automatically brought from phase warm up to performance phase of training, after then to calming cool down phase. In case of manual pressing of button the trainee activates button at start of warm up, afterwards again at start of time measuring of performance section of training, then at ending of performance section of training, through which is stopped the measuring of time and ended phase cool down. In regime of time measuring after reaching of target value of pulsation frequency is sportsman led only in performance phase of training on target pulsation frequency where by activation button at start is not led in phase warm up but preferably sound indication is giving him information that he has not yet reached target pulsation frequency and thus also not the operation temperature. When sound signal stops, he will receive the information that he can start performance phase of training there preferably by activation of button start and stop is measuring time of training. In phase cool down again is not trainee led and record is stopped by button cool down.

In regime of simple graphic illustration of course of pulsation frequency is recorded course of pulsation frequency live and simultaneously is stored in memory of device or on server to immediate or consequent information. At all these regimes preferably sportsman follows courses of pulsation frequency live or later on, after finishing of training.

In principle sportsman from the calm level of pulsation frequency in warm up phase is increasing his pulsation frequency until suitable target value, mostly 70% of individual pulsation frequency and on it is suitable to remain during the intensive performance phase of training. After finishing of performance phase is suitable the load of organism decrease in defined way again onto the calm level. The invention is optimizing such process in such a way that the sportsman is guided by the devise whether to increase or decrease the load. This is be reached when device on base of entry data of sportsman or trainer will create the mask in which the pulsation frequency during the training may be found namely for all phases of training.. Mask is preferably interactive and sportsman can influence it that by pressure of buttons especially places all individual phases of training.

Part of mask there is tolerance field in which deviation of ideal curve, alongside which the actual pulsation frequency should move in time, should be kept. After exceeding of tolerance field the sportsman will get usually acoustic warning whether is under or above limit. Width of tolerance field is also adjustable.

The indicated way is suitable also for monitoring of pulsation frequency of cardiac during the physical activities recommended by doctor which are one of the best preventions measures against illness of heart and vessels. Primarily it concerns the dynamic sports as running, swimming, biking, under condition of continuous monitoring of heart frequency. Also in case of these activities is course of pulsation frequency similar as in case of training of sportsmen, although with substantial lower load at warming up of muscles, main part of exercise also in the end including taking of breather and following relaxation.

Device provides various illustrations namely as the whole shape of set mask for planned or current training therefore also its actual part in required detail in dynamic regime when the picture on display is moved.

Optional is possibility of change of starting setting of time distinguishing of projection.

Sportsman optionally influences various evaluated phases of training par example to start them sooner or, at the contrary, to postpone accordingly momentary situation.

Par example he has not to follow the device for warm up phase only he lets to indicate that he reached the required pulsation frequency, afterwards he will press the button for start of performance phase of training, similarly in the end of this phase.

For further illustration there is on display displayed also course of pulsation frequency in time scale selected in such a way that i tis possible to distinguish the individual pulses and then also arrhythmical pulses which are preferably marked graphically. Numbers which are illustrating the individual pulses are giving possibility clearly to monitor irregular pulses and are marked if being irregular. The irregular pulse is such one that exceeds adjustable limit of deviation of average pulse measured within set number of pulses. Arrhythmia is illustrated preferably simultaneously in scale and/or verbally where level of arrhythmia is percent of irregular pulses from the total number of pulses regularly divided in individual degrees and/or percent's. Preferably they are indicated percent's of arrhythmia from zero to hundred percent's when at hundred percent's are hundred percent's of pulses irregular. Preferably they are indicated also the degrees of arrhythmia par example 1/10 percent of irregular pulses that means scale from one to ten.

Preferably is possible to display on display of some of devices which holter contains in real time during training also later from record with possibility of setting time axis floating graph of pulsation frequency which is hifted to left in the direction from right side of display, where starts, to left end of display where it disappears. It is projected with actual value of time. On displaying unit or display of monitor is placed in upper part of projection and is drawing the adjustable time interval preferably of last 30 seconds of course of pulsation frequency in real time. Position of every vertical segment illustrates time moment of occurrence of corresponding individual heart pulse and level corresponds with its pulsation frequency.

Besides it is continuously monitored each occurrence of arrhythmia at detection of arrhythmic pulse which is drawn preferably with different thickness and color of corresponding vertical segment. Value of arrhythmia is further drawn graphically by column and by verbal indication of level of arrhythmia as indicated in following degrees : "None" (arrhythmia not detected), further degree "Low" and "Trivial" marked by column, degree "Medium" and degree "High" and "Very High". Preferably is numerically projected actual value of pulsation frequency, par example 82 degrees per minute and continuously are during training numerically marked the time data on display. Evaluation of training is controlled preferably by big round button which is generally marked and its inscription illustrates what will happen after its pressing in dependence on current application, preferably "Start", "Pause", "Stop" etc. Fixed graph of pulsation frequency placed in lower part of display is drawing course of pulsation frequency during the whole training with marking of time intervals. Before the start the user has the possibility to make option of regime of monitoring of training. Regime of automatic monitoring is using the preset parameters without their corrections, whereas as value of pulsation frequency is inserted in certain moment the actually measured value. Regime of automatic monitoring can be modified in such a way that the sportsman before starting of training will use possibility of change of all or only selected preset parameters before start of training. By pressing of button with function Start (after pressing the notice and therefore the function is changed on Pause) on panel of electronic device, the training starts and the short beep is given. The floating graph will be continuously shifted which will enable to display value of pulsation frequency for the last 30 seconds with good distinguishing. Fixed graph of pulsation frequency placed in lower part of picture will be drawing value of pulsation frequency recorded during the whole training. After start of training will grow the value of pulsation frequency until the reaching of end of zone. At this moment the actual pulsation frequency of user will reach optimum value of target pulsation frequency and training comes to further zone and such stage will be signaled by short beep. After ending of load part of training at the end of zone the user comes to other zone, transit is again acoustically signaled by short beep. In this zone user will reduce the tempo of running and actual value of his pulsation frequency will be coming down until the reach of value of original pulsation frequency before start and thus also ending of zone.

On graph of course of pulsation frequency are visible the moments during the training in which occurred the exceeding of set limits as deviation of tolerance field.

Graph of course of pulsation frequency is floating and enables to sportsman to follow wherever phase of training in various scales on time axe by placing of change of scale or shift of graph.

When the scale is placed on time axe in such a way that the whole training can be displayed, the graph will be fixed, only the momentary value of pulsation frequency is changed in graph, the scales will remain fix.

Projection can be stopped by function "pause" and renew the function "continue" eventually finish by function "stop".

Curves characterized heart activity of monitored person displayed by display of holter which is preferably part of control module or displaying module or evaluation block. Advantage is simultaneous projection of curve of pulsation frequency and curve of number of occurrence of arrhythmic and regular pulses expressing number of occurrence of arrhythmic pulses of monitored person. Curve of upper limit of deviation from average pulsation and curve of lower limit of deviation from average pulsation are the limiting curves of regular pulses for determination of arrhythmia. Upper bar displayed on display is drawing two lines of text messages, lower bar is drawing time axe. In upper part of display is placed the curve of pulsation created as connection of points drawing values of pulsation one by one displayed on picks of segments of values of pulsation the position of which is indicating on time axe the moments in which the value of pulsation was determined and their height indicates value of pulse. Degree of arrhythmia expressing number of occurrence of arrhythmia pulses is in indicated case stipulated with the use of average pulse which is calculated as average out of values of optional number of preceding pulses, preferably of three. From this curve is derived curve of upper limit of deviation from average pulse corresponding preferably to values of curve of average pulses increased for optional number of pulses corresponding preferably with values of curve of average pulsation increased for optional number of pulses preferably 10 pulses and similarly curve of lower limit of deviation from average pulsation corresponding preferably with values of curve of average pulsation decreased for optional number of pulses, preferably 10 pulses. If actual value of pulsation expressed by curve of pulsation deviates out of limits set by curve of upper limit of deviation from average pulse and curve of lower limit of deviation from average pulse the occurrence of arrhythmia is detected. Curve of number of occurrence of arrhythmic and regular has preferably 10 degrees (0 to 10) whereas degree No. 1 0 is the highest. Curve of number of occurrence of arrhythmical and regular pulses 12 is connecting bar of degrees of arrhythmia which are not displayed. Initial degree of arrhythmia is 0. At detection of occurrence of arrhythmic pulse the curve of number of occurrence of arrhythmic and regular pulses is preferably increased for one degree with every arrhythmic pulse. If in case of actual pulsation occurrence of arrhythmia is not detected, curve of number of occurrence of arrhythmic and regular pulses is decreased for one degree or alternatively on 0. Time course of arrhythmia depicts curve of number of occurrence of arrhythmic and regular pulses placed in lower part of projection of curves. Scale of arrhythmia has division on segments corresponding to percent's namely par example 10 segments i.e. 1 segment corresponds to 10 % of arrhythmia. Value of arrhythmia is drawn on axe Y, by curve number of occurrence of arrhythmic and regular, which is continuously actualized. Time scale drawn on axe X for curve of number of occurrence of arrhythmic and regular pulse sis common with curve of pulsation. Value of arrhythmia related to chosen time segment can be expressed by several ways. Numeric value of arrhythmia for time segment of preferably 30 seconds is displayed in upper part of display where indication with preference 4.2 decrees of arrhythmia taken as 10 % out of arrhythmic pulses then means occurrence of arrhythmia 42 % which is fixed of perceptual relation of number of pulses with detected arrhythmia out of whole number of pulses in evaluated time segment. Values of pulsation and time depicted by curve of pulsation in evaluated time segment and on tine axe expressed as number of pulses per minute which is placed on lower bar with marked indication of real time. Actual value of pulsation is calculated of time distance of segments of pulsation value generated in monitor of heart signals general depicting actual pulse in preceding time interval. Time is fixed by numbers of time interval preferably with distance of 10 seconds which are depicted by time marks. For increase of distinguishing are the segments of pulsation value and time marks distinguished by colors. Storing of whole or partial courses of pulsation, arrhythmia or ECG into memory of device is possible whenever by use of buttons preferably touch and/or automatically in case of exceeding of preselected limit and/or automatically in set time intervals. Setting including choice of actually projected curves and data as well and way of their projection controls preferably group of touch buttons formed on display the number of them, marking and function is subject of change in dependence on actual setting. Further buttons can have various functions. Volume of acoustic alarm par example for night and day is changed by button. Brightness of display is possible to change by button M. Curve of pulsation is possible to shift vertically by buttons. Further button controls remote change in setting of group of filters in electronic s of attached detecton device. Stop and restart of continuous projection of curves controls button 1-/II. Increase/decrease of horizontal scale of curve of pulsation control buttons H+/H-.

Reading of coordinates of the actual pulsation is easy. Start of curve of pulse on right side depicts actual value of pulse in actual time and its placing several millimeters from right edge of display makes easy reading of its coordinates. After evaluation of every next pulse the while displayed curve of pulsation is shifted for one pulse to left and on the eree place is displayed its value. Simultaneously also curve of number of occurrence of arrhythmic and regular pulse is shifted and completed.

Expression of arrhythmia is preferably simple in words or by column graph. For orientation expression is possible to use the verbal description of arrhythmia and there are values divide in several groups, preferably None, Low, Trivial, Medium, High, and Very High and similarly are possible to use for the depiction of arrhythmia also several various heights of orientation column graph of arrhythmia.

Curve of average of pulsation is calculated of chosen number of preceding pulses which has not to be displayed and was calculated of curve of pulse. Curve of upper limit of deviation from average pulse and curve of lower limit of deviation from average pulse are the limiting curves of regular pulses for determination of arrhythmia.

Parameters of setting of filters of external disturbance and setting of limiting value arrhythmia are adjustable.

Button N is set for evocation of offer or gradually groups of offers for insertion of parameters of projection and evaluation of curves. Filters 0, 1 and 2 of external disturbance are decreasing influence on external disturbance on evaluation of pulses. Filters are possible to switch over during operation by button marked as F0 the marking of which is changed on F1 or F2 in dependence on chosen filter. Degree of filtration sets parameter marked as "Upper edge"of pulses per minute the exceeding of which is taken as failure caused by external disturbance. Source of alarm status and therefore also release of signal by exceeding of set limit of arrhythmia is set by parameters "Arrhythmia above" expressing limit value of arrhythmia expressed in % and "for the time " for which the arrhythmia is calculated. Also is possible to set delay between exceeding of limits and release of sound signal of alarm. There are available also the offers namely for setting of parameters of detection of arrhythmia and setting of limits of alarm the exceeding of which activates the alarm stage. Detection of arrhythmia is using parameters for calculation of values of average pulsation and from it derived curves - curve of upper limit of deviation from average pulse and curve of lower limit of deviation from average pulse which are setting items "Average Pulse per X Pulses" and "Upper and Lower Limit " expressing deviations of curves of upper limit of deviation from average pulse and curve of lower limit of deviation from average pulse from calculated average value of pulses expressed by number of pulses par example +/-10 or percent's.

Parameter "Ignoring of multiple of average" is limiting activation of alarm status in cases of evaluation of abnormal deviation of pulse caused by breaks of signals.

Remaining items of offer are set for setting of further limits of alarm.

Limits of alarm of pulses are setting parameters " Minimum permitted pulse", per example 40 depicted by line of upper limit of pulse for Alarm how is depicted on " indicating value of pulse, under which the decrease of pulse for more that in item "Alarm at N pulses one by one above/under limit" per example N is equal to 3, one by one going pulses causes alarm status. Similarly item "Maximum permitted pulse" per example 80 is setting position of line of upper limit of pulse for alarm of upper limit for alarm indicating value of pulse the exceeding of which by pulse for N( par example N is equal to 3) or more one by one going pulses inserted as second item marked "Alarm at N pulses one by one above/under limit" will cause alarm status.

Level of arrhythmia is followed for individual pulses in optional time limits in respective example 0,5h. Degrees of arrhythmia are depicted in area of projection of curves and it is possible to switch over by button S of display of pulsation. Imaging of values of arrhythmia is using the whole area od curves projection. Function of buttons N, S and F0, are not changed in case of switching over. Simultaneously with switch over of projection also the marking and function is changed as well as some other touch buttons in comparison with preceding example. By pressing of button of range is possible to select time range of degrees of arrhythmia. Under degrees of arrhythmia is in each of depicted segments marked time on begin and end of segment as well. Also there is indicated occurrence of arrhythmia during the whole segment. There is also depicted cursor as thicker segment line the position of which on curve up to place of interest is shifted by buttons < > ^ v. By pressing of button D is cursor shifted into the place of actual time, button Z increases the time detail in place of position of cursor.

Imaging of degrees of arrhythmia is formed by numeric values of arrhythmia which are not connected by curve and in case of selection of longer time segment par example 8 hours are strained in structure which has no gaps between the individual numeric values of arrhythmia and thus optically creates curve appearance.

Curves of ECG are par example in time range of 5 seconds. On background of curve ECG is illustrated millimeter grid enabling deduct values of amplitude of impulses and also time data of course. Scales of projection of ECG curve in horizontal and vertical direction are possible to be changed by buttons of group of touch buttons formed on display and set also for all further indicated operations. Actual scales, per example 25 mm/sec. On horizontal axe and 20mm/mV on vertical axe are indicated on lower side of ECG graph. Besides this is on the beginning of ECG curve illustrated calibration impulse the height of which indicates scandalized voltage 20mm/mV normally used for evaluation of amplitudes of impulses of ECG curve. Above every complex QRS is mark of pulse preferably formed by small star indicating that in monitor of heart signal general to every QRS complex was handled over pulse drawn in curve of pulse. Touch button of the group of touch buttons is enabling switch over of remote movable filters to monitor of heart signals. The movable filters are set preferably in régime calm, walking and run. Filters for sensing ECG and curves of pulse in movement are distorting curves so that is difficult to get from them wave "P ". For recording of undistorted curve in calm holter produces warning signal acoustically, by vibrator or display in adjustable periods in which the monitored person is brought in calm and by button marker is made record and marked time which is depicted on display in case of curves preferably of pulse or ECG, by mark. The monitored person can use holter also without warning signal in order to indicate time in certain situations or activities par example, if the record was made, if he/she is not feeling well or at increased load. Simultaneously voice record is preferably on control module preferably initiated by button. Record is replayed upon request preferably in case of marking by marker. In regime pause is preferably possible to travel through record par example ECG or pulse only following the markers at pressing of button "image marker" preferably always for one marker further. For saving capacity of accumulator is preferably prepared record only at pressing of marker.

In imaging also the missing is displayed, with missing impulses, which indicates that the detection of complex QRS has not passed in a correct way and therefore the value of segment of pulse value will not be achieved correctly, there will be gap in curve of pulse. In case that complex QRS as it is displayed will be additionally detected which is to be recognized on marks following each other, the value of segment of pulse value will be not contained correctly and on curve of pulse will be recorded one pulse more.

Curve ECG can be simultaneously projected with curve of pulse whereas time scale of projection of both curves differs. Time scale used for projection of curve of pulsation can be also bigger which is enabling to follow course of curve of pulsation. Width of rectangle forming ECG cursor is marking on curve of pulsation time period corresponding to length of segment displayed by curve of ECG. In regime PAUSE when the imaging in real time does not takes place it is possible by moving of cursor on recorded curve of pulsation to browse through records and thus to display respective segments of stored curve of ECG. The invention is:

Live Holter comprising a holter (351) with a control module (357') and/or with imaging module (358) and optionally an evaluating block (359) wherein the holter (351) comprises a chest belt (749"), a front end unit (362), a monitor of heart signals (349"), a communication unit (275 '), and the live holter is adapted for sensing analog heart signals from the chest belt (749") and processing the heart signals in the front end unit (362) into data sent to the monitor of heart signals (349") and from the monitor of heart signals (349") to the communication unit (275') for transmission for Holter evaluation in at least one of: the control module (357'), the imaging module (358), the evaluating block (359) and for transmission for displaying live or from record on at least one of: the control module (357'), the imaging module (358), the evaluating block (359), and the evaluating block (359) is comprising server (806) and further comprising operator's PC (839) and/or participant's PC (839'), wherein the monitor (349") of heart signals comprises a memory for data recording of heart signals, advantageously formed by a SD card (888), and the communication unit (275') communicates locally with the control module (357 '), for displaying on a display (363) of control module and/or the communication unit (275') communicates locally with the imaging module (358) and the local displaying is for patient and/ or medical staff, wherein the holter (351) is further adapted for long distance transmission by means of the communication unit (275') through a network of mobile operator (898) and/or through a WiFi (131) to evaluation block (359) for displaying to medical staff and for permanent monitoring of monitored data live or monitoring of recorded data.

### Clarification of drawings

**Fig. 1** is drawing the main principles of holter and basic blocks, modules and units from which consists and their functions.
   **Det. 1** is drawing overview of communication of chest strap with control module.
**Fig. 2** is drawing holter with maxi chest strap with ECG monitor preferably in unit of electronics.
**Fig. 3** is drawing holter with mini chest strap without ECG monitor.
   **Det. 1** is drawing control unit with module of auxiliary units.
**Fig. 4** is drawing connection of mobile phone to monitor of heart signals and further data about human body.
   **Det. 1** is drawing remote communication of nearby cooperating units through network of mobile operator.
   **Det. 2** is drawing mobile phone on wrist, communicating with monitor preferably simplified, without backup.
   **Det. 3** is drawing simplified version of detail 2 where connection is not backed up.

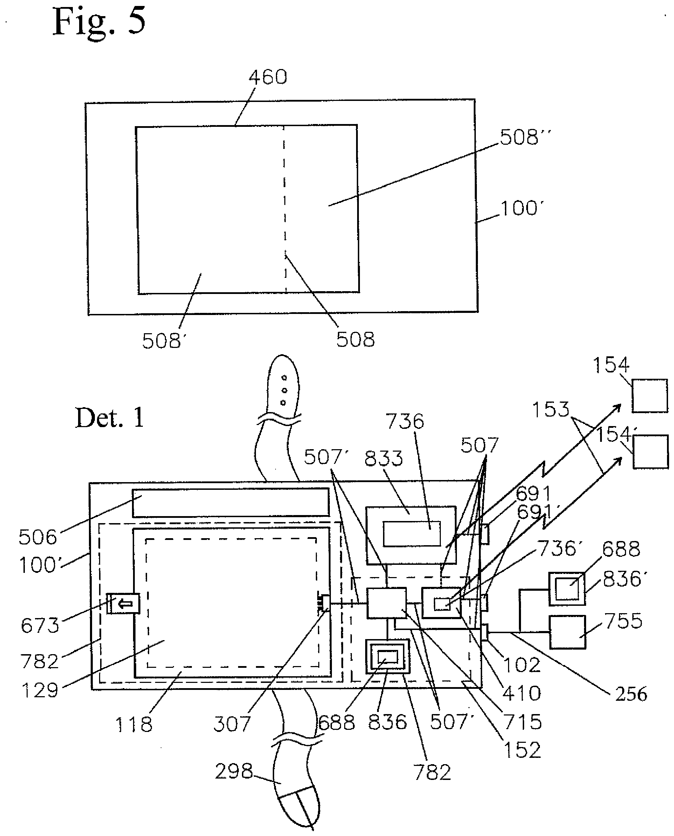

**Fig. 5** is drawing mobile phone with auxiliary functions, easy removal ot basic accumulator preferably without interruption of operation.

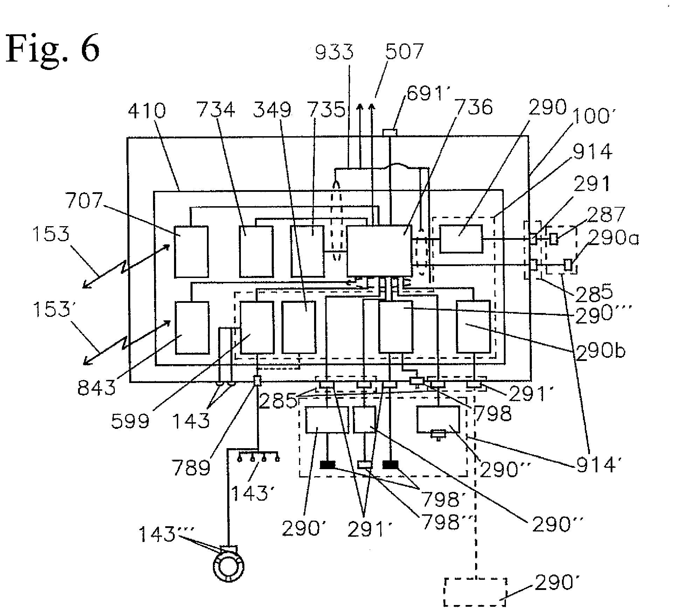

**Fig. 6** is drawing module of auxiliary units with control unit placed in mobile phone with auxiliary functions.

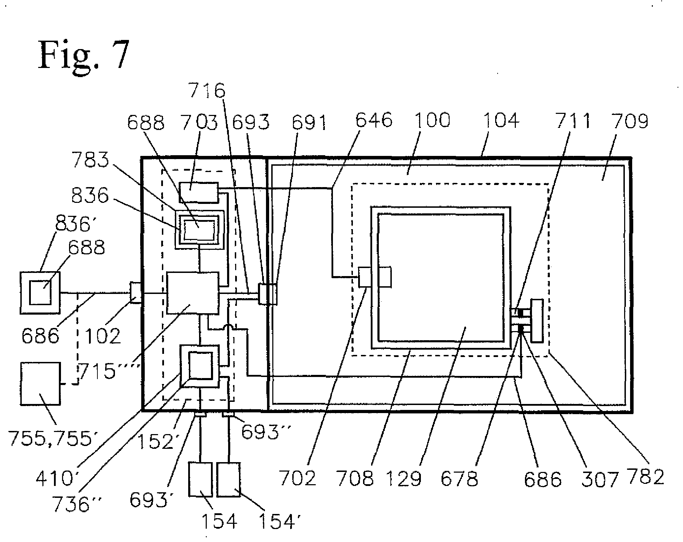

**Fig. 7** is drawing mobile phone with removable auxiliary device adapted for easy replacement of basic accumulator for charged one.

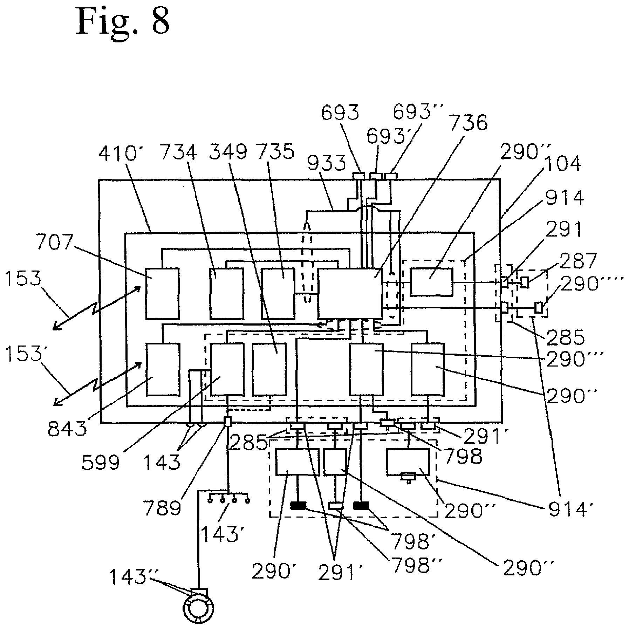

**Fig. 8** is drawing module of auxiliary units for auxiliary device to mobile phone standard.

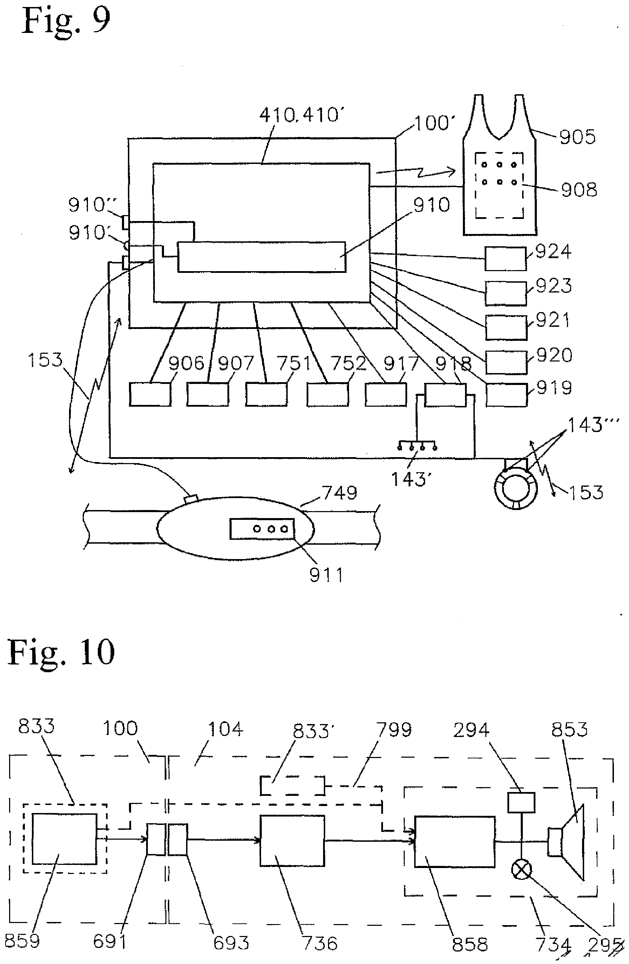

**Fig. 9** is drawing connection of sensors to mobile phone.
**Fig. 10** is drawing block diagram of detection of activity by WATCHDOG unit.

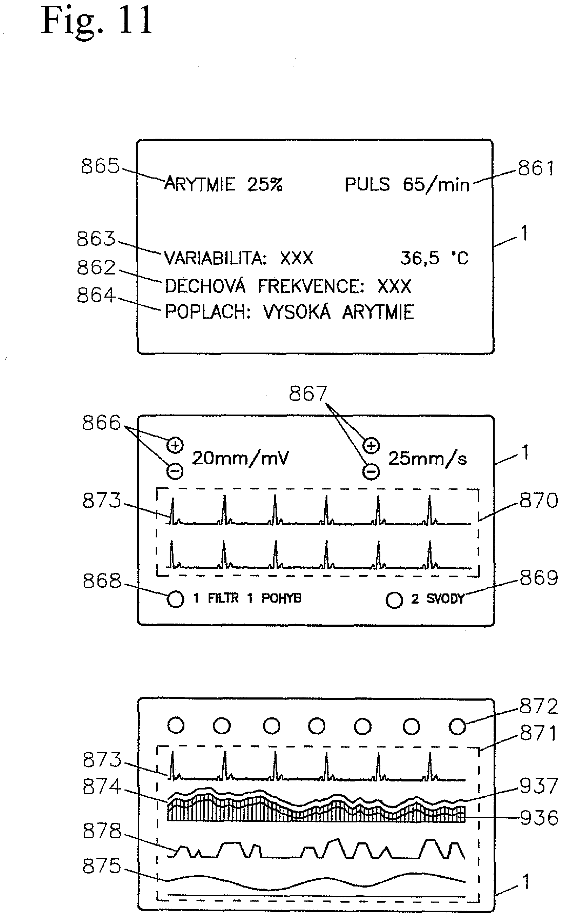

**Fig. 11** is drawing the examples of projection of data produced by monitor of heart signals on display of monitor.

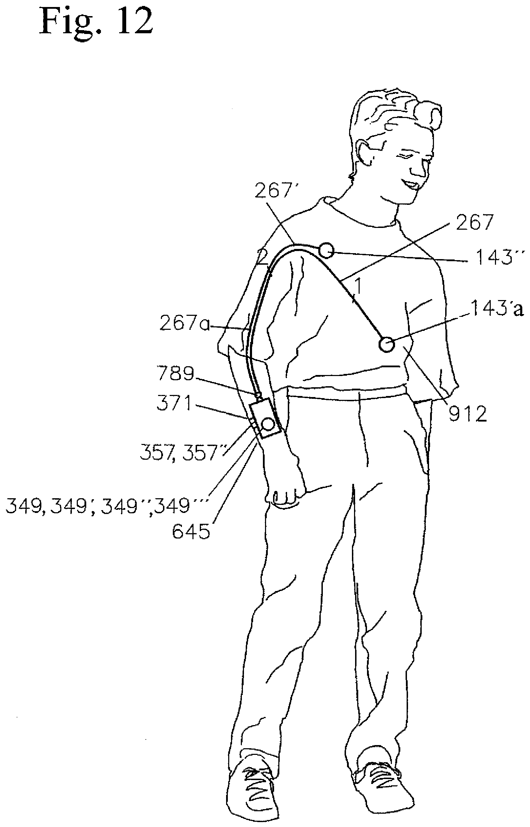

**Fig. 12** is drawing attachment of glued electrodes to mobile phone.

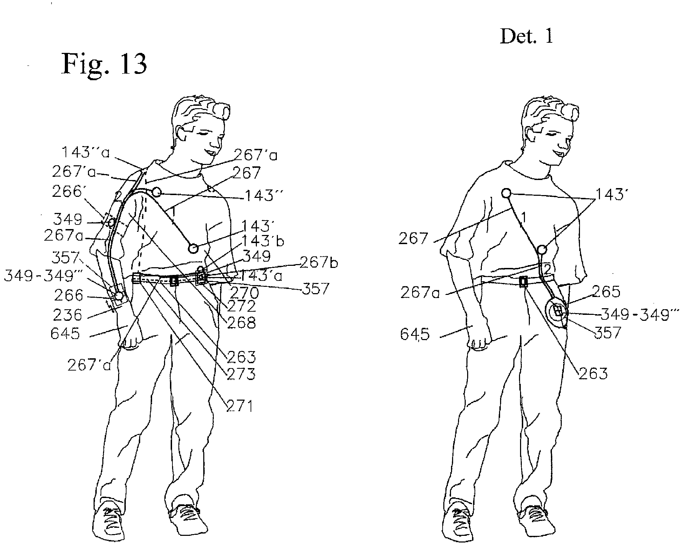

**Fig. 13** is drawing monitor preferably fastened on band.

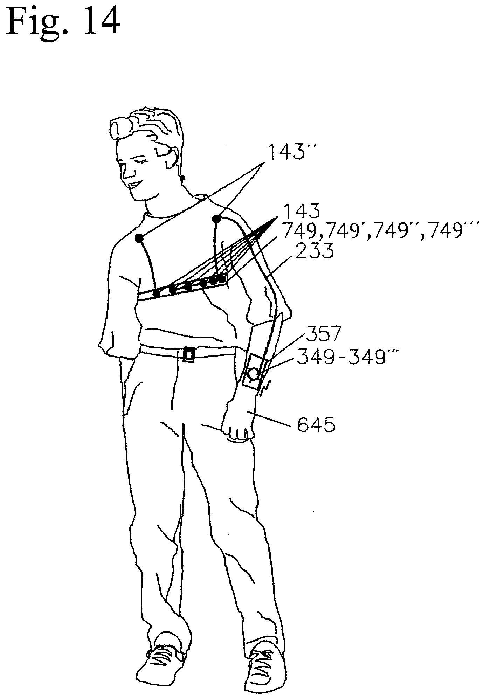

**Fig. 14** is drawing combination of glued electrodes with electrodes on chest strap.

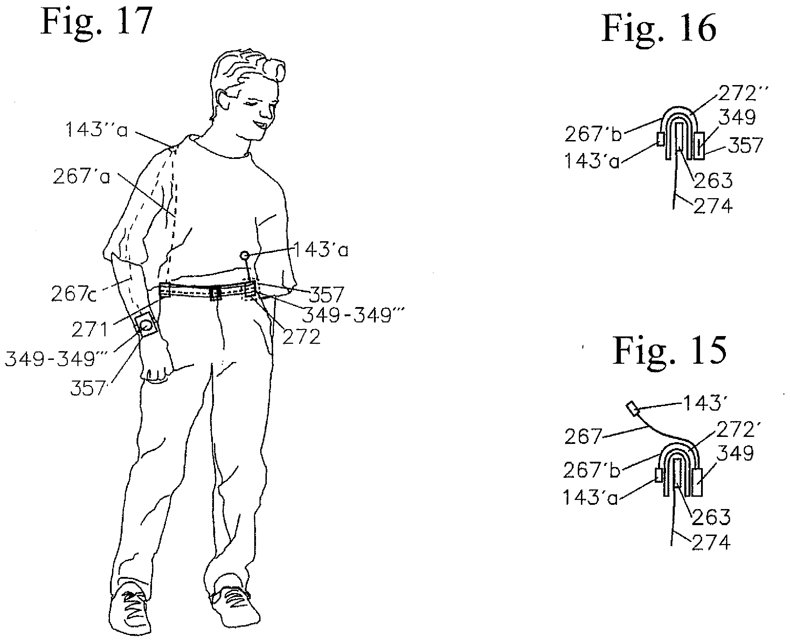

**Fig. 15** is drawing attachment of clasp on band and interconnection of electrodes.
**Fig. 16** is drawing clasp fastened on band.
**Fig. 17** is drawing cable brought directly to monitor.

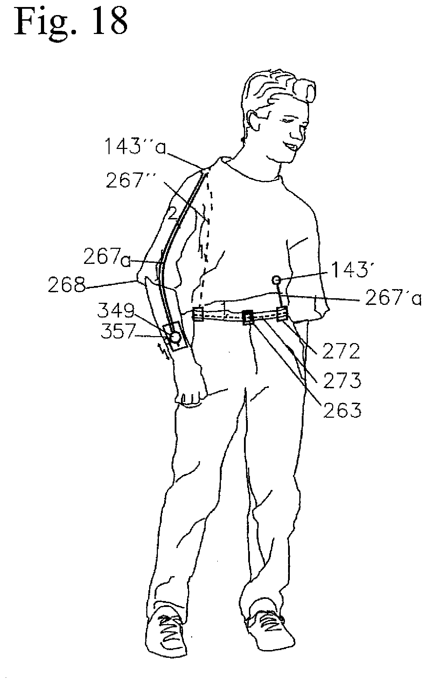

**Fig. 18** is drawing electrode connected to monitor by cable led through sleeve therefore there are not any cables or bands on chest.

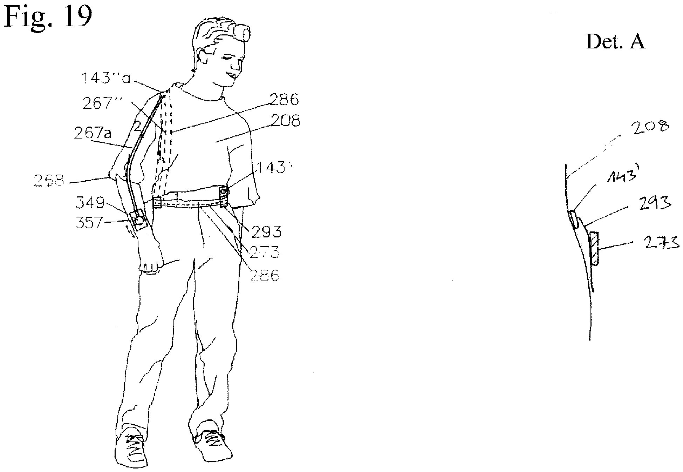

**Fig. 19** is drawing placement of electrodes, without glue pressed on body.

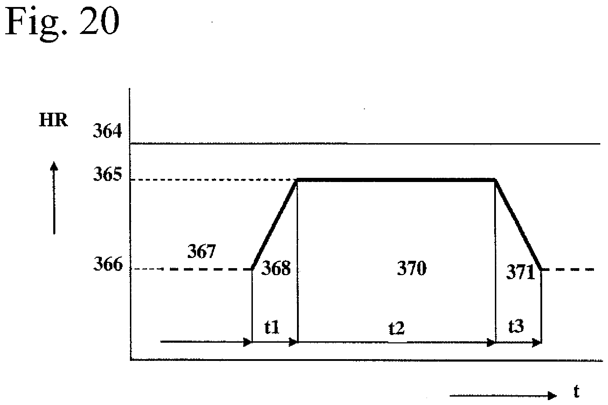

**Fig. 20** is drawing general time course of value of pulsation frequency in dependence on time and physical activity.

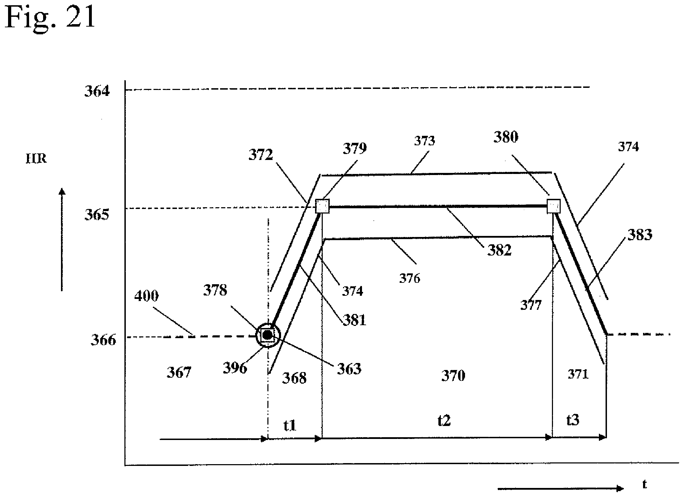

**Fig. 21** is drawing example of automatic monitoring and evaluation of measured values of pulsation frequency during training.

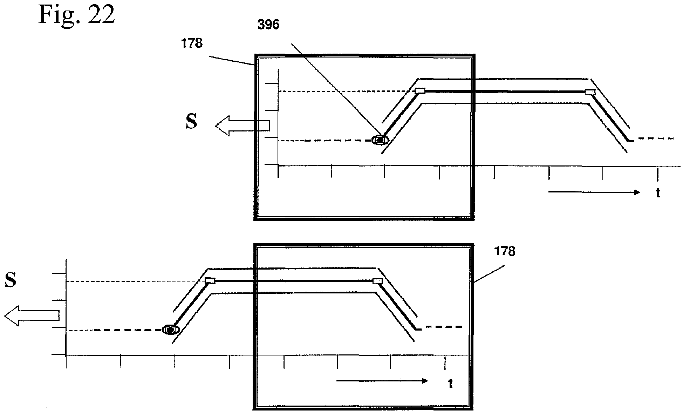

**Fig. 22** is drawing dynamic shift of displayed graph.

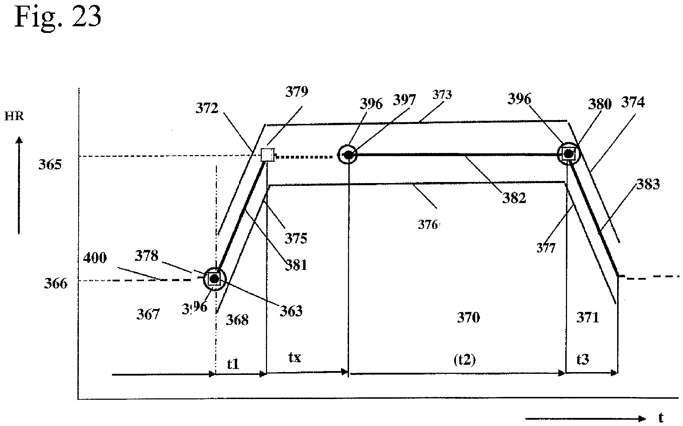

**Fig. 23** is drawing example of manually started monitoring and evaluation of measured values of pulsation frequency during training.

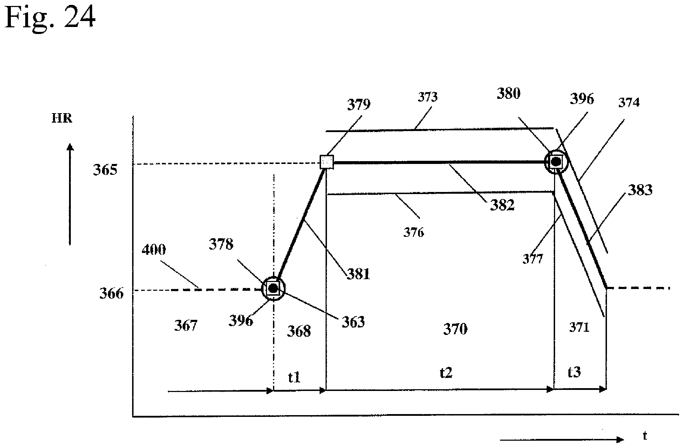

**Fig. 24** is drawing measuring of time necessary for the increase of pulsation frequency onto target value.

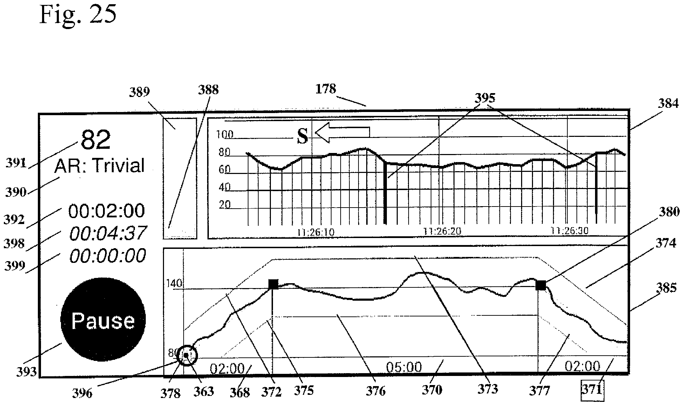

**Fig. 25** is drawing example of projection of values of pulsation frequency during training on display of electronic device.

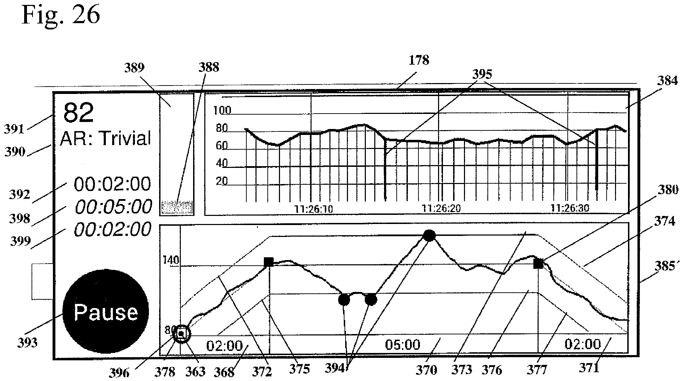

**Fig.26** is drawing example of deviation of values of frequency of heart pulsation out of set limits.

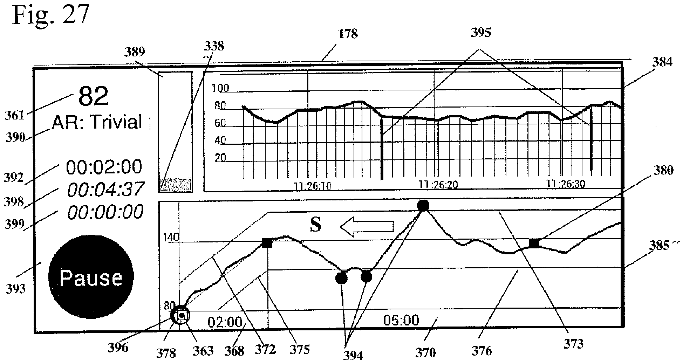

**Fig. 27** is drawing example of dynamic shift of displayed graph on monitor for increase of time distinguishing of imaging of graph.

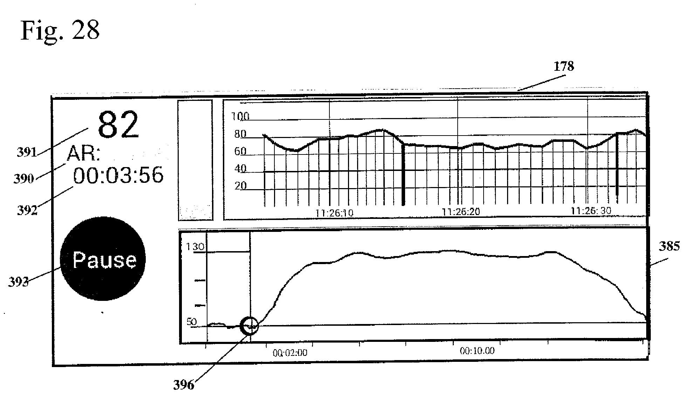

**Fig. 28** is drawing example of simple graphic projection of course of pulsation frequency during the whole training.

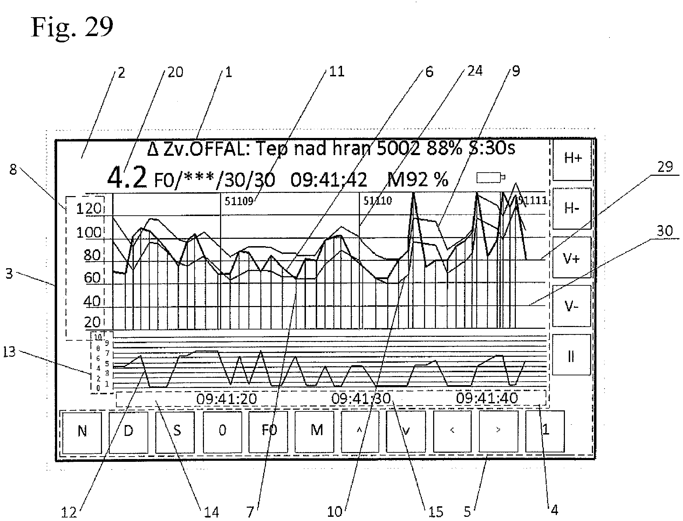

**Fig. 29** is drawing example of simultaneous projection of curve of pulsation frequency and curve of arrhythmia.

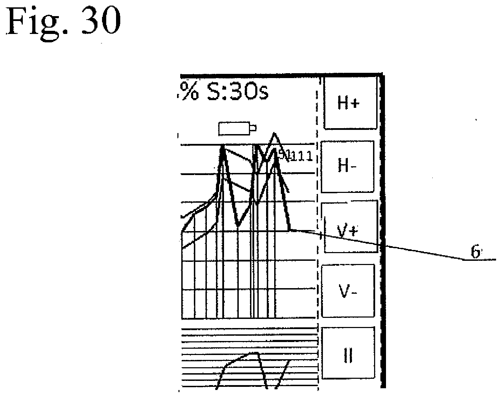

**Fig. 30** is drawing example of imaging of actual values of pulsation frequency and arrhythmia.

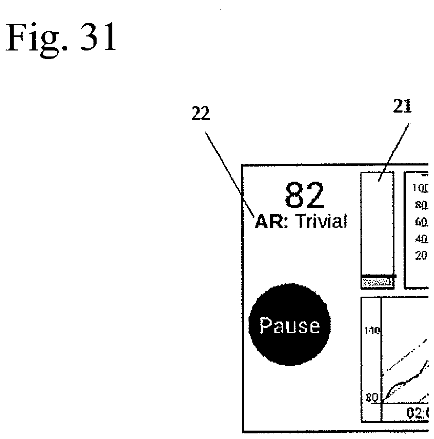

**Fig. 31** is drawing expression of arrhythmia by column graph and verbally.

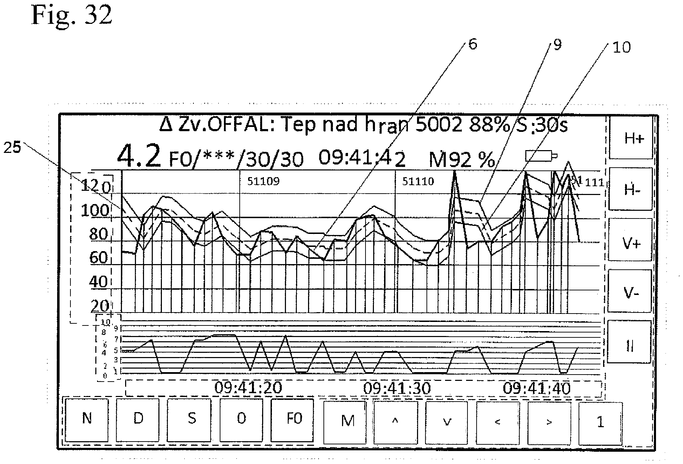

**Fig. 32** is drawing curve of average of pulsation frequency.

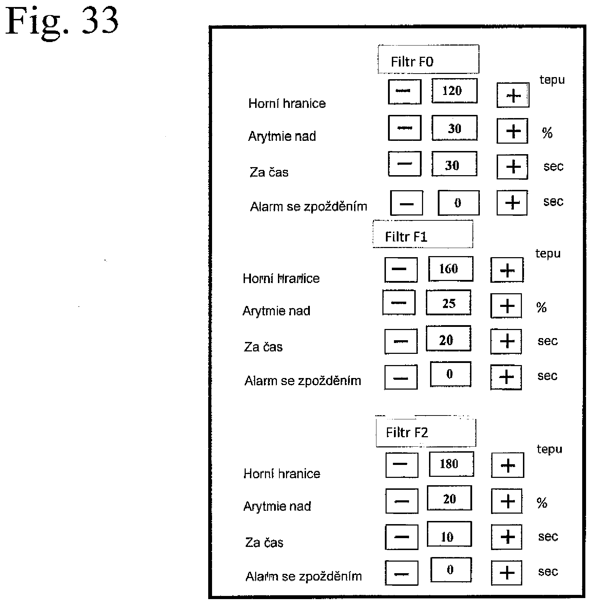

**Fig. 33** is drawing offer of parameters of setting of filters of external disturbance and limit of arrhythmia for starting of alarm.

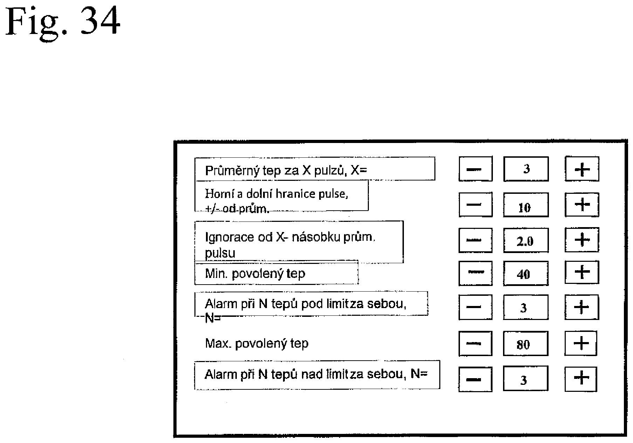

**Fig. 34** is drawing offer of setting of parameters of pulsation frequency for evocation of alarm.

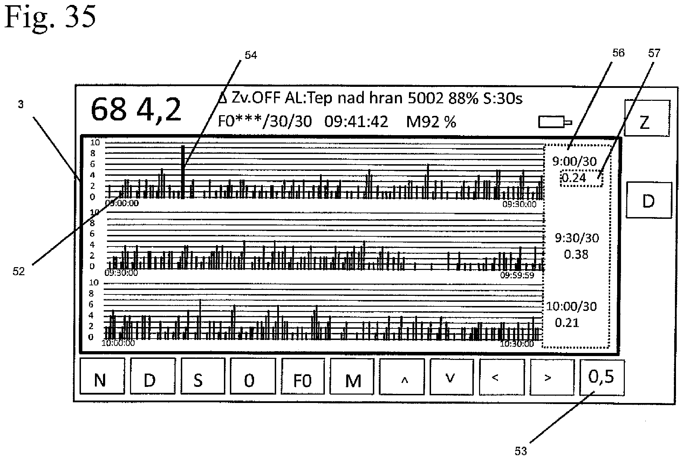

**Fig. 35** is drawing example of monitoring of level of arrhythmia in half an hour periods.

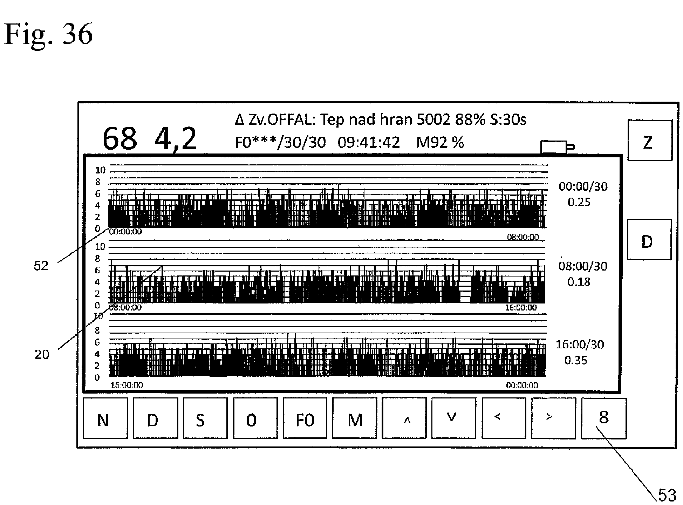

**Fig. 36** is drawing example of monitoring of level of arrhythmia in eight hours periods.

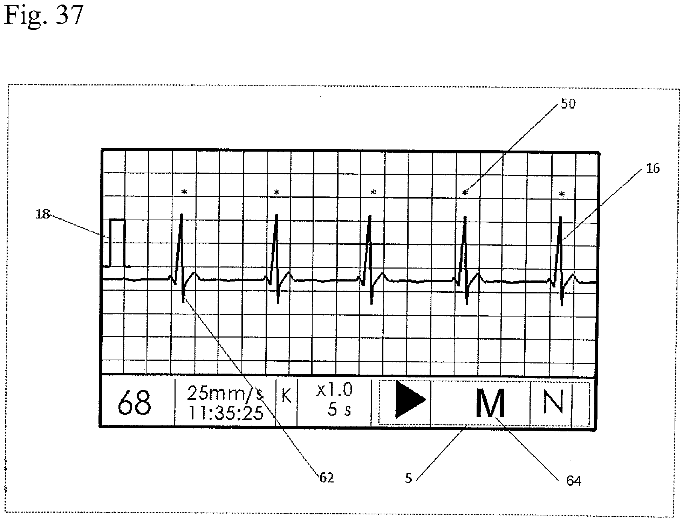

**Fig. 37** is drawing imaging of curve of ECG in time segment of 5 seconds.

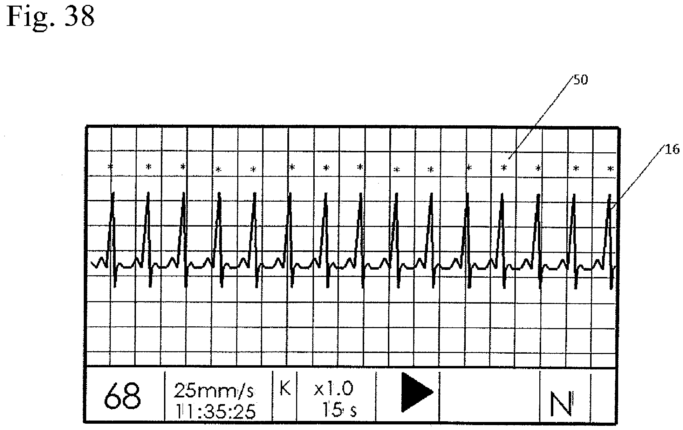

**Fig. 38** is drawing imaging of curve of ECG in time segment of 15 seconds.

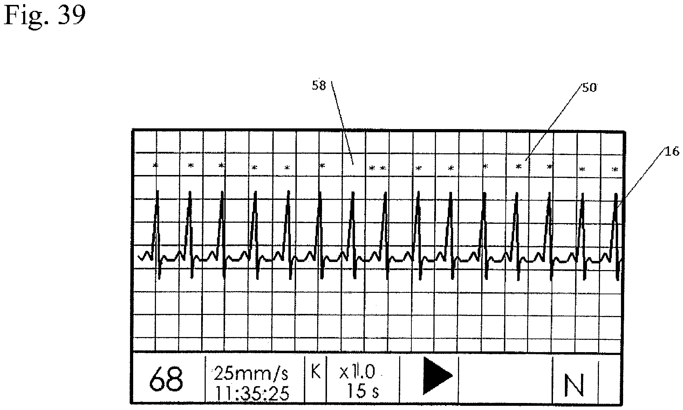

**Fig. 39** is drawing imaging of curve of ECG in time segment of 15 seconds with example of missing pulse.

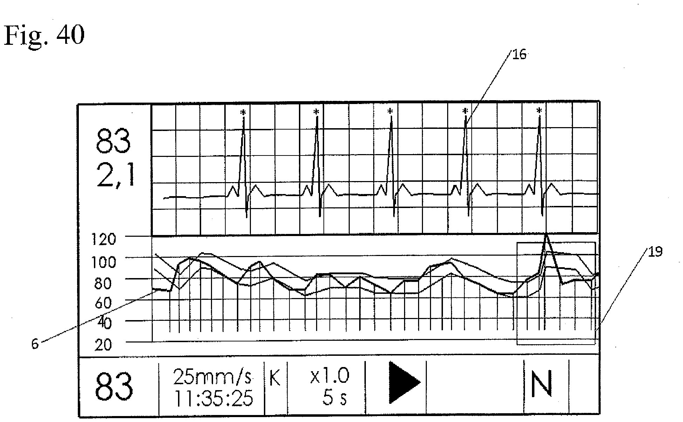

**Fig. 40** is drawing example of simultaneous imaging of curves of ECG and pulsation frequency in various time scales.

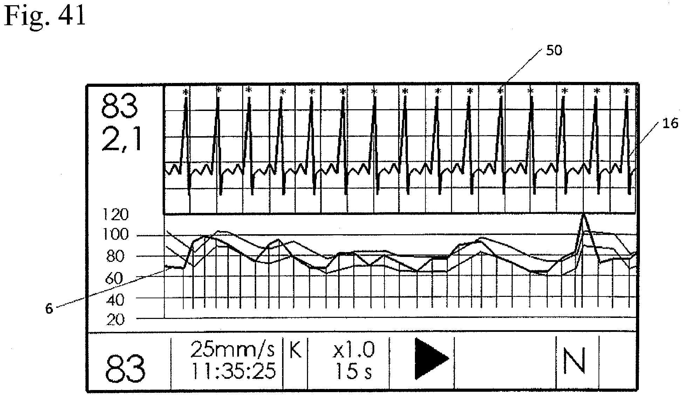

**Fig. 41** is drawing example of simultaneous imaging of curves of ECG and pulsation frequency in the same time scale.

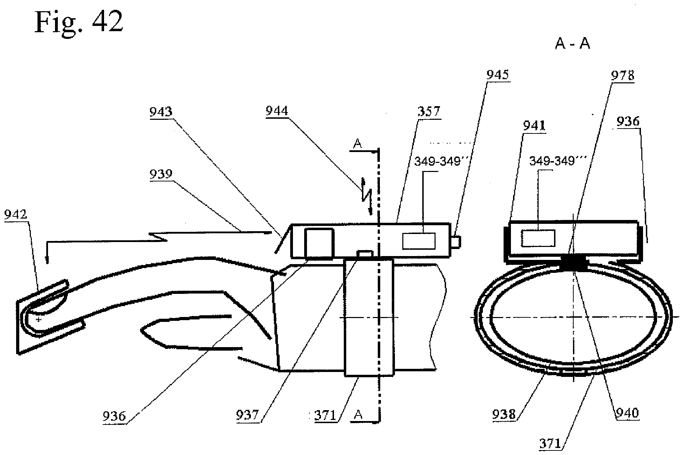

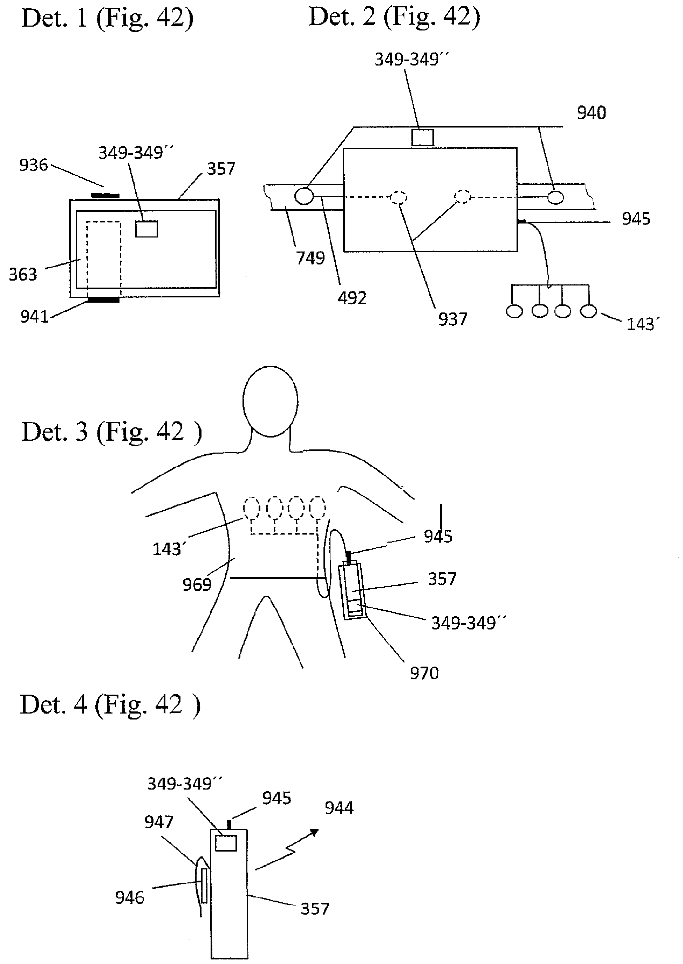

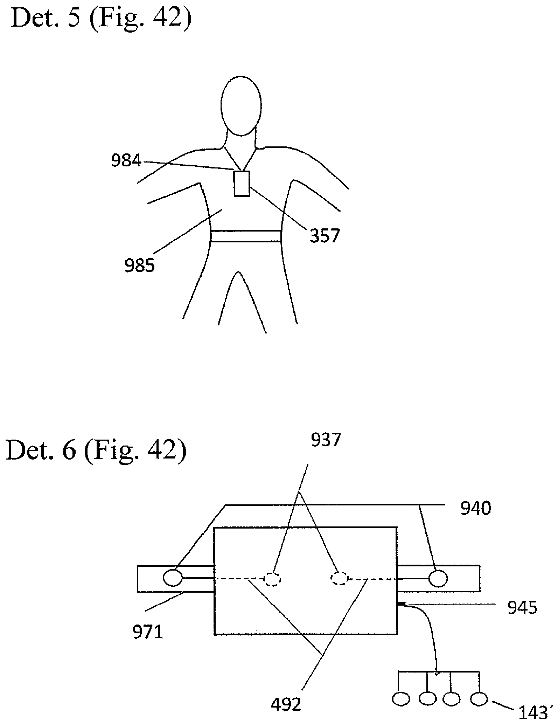

**Fig. 42** is drawing placement of control module.
   **Det. 1** is drawing control module in a freeway.
   **Det. 2** is drawing control module on chest strap.
   **Det. 3** is drawing control module in purse.
   **Det. 4** is drawing control module on belt.
   **Det. 5** is drawing control module on neck.
   **Det. 6** is drawing control module on shortened belt.

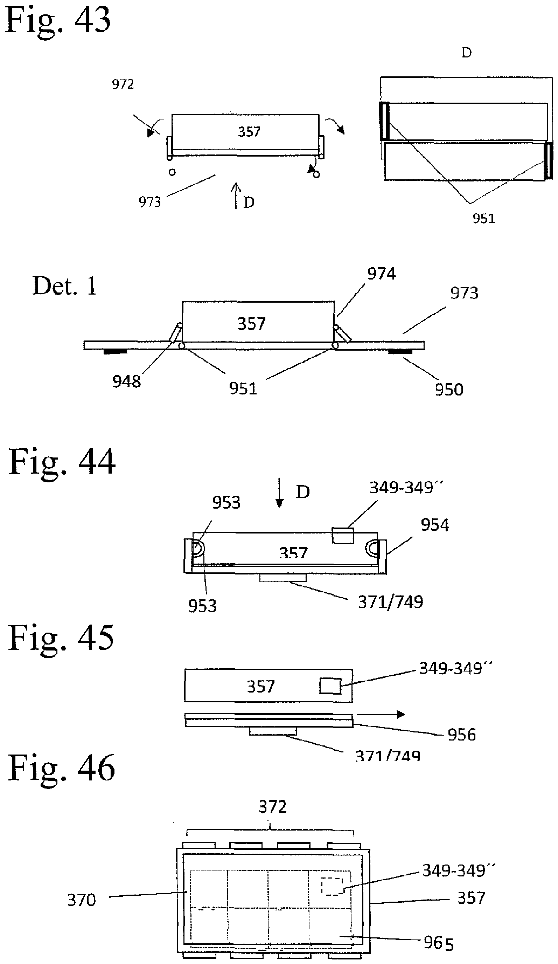

**Fig. 43** is drawing control module with tiltable electrodes.
   **Det. 1** is drawing control module with tiltable electrodes.
**Fig. 44** is drawing control module in click-on holter.
**Fig. 45** is drawing control module in slide-onto holter.
**Fig. 46** is drawing control module with button.

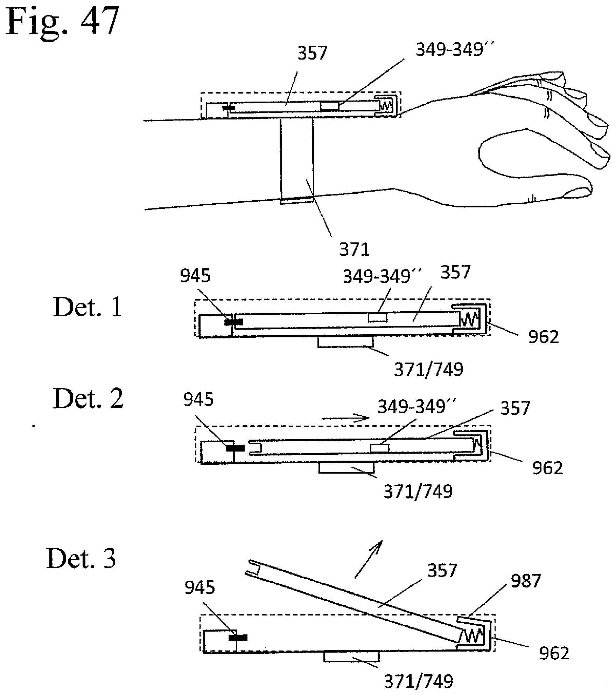

**Fig. 47** is drawing control module in storing mechanism.
   **Det. 1** is drawing control module inserted.
   **Det. 2** is drawing control module slided out.
   **Det. 3** is drawing control module removed.

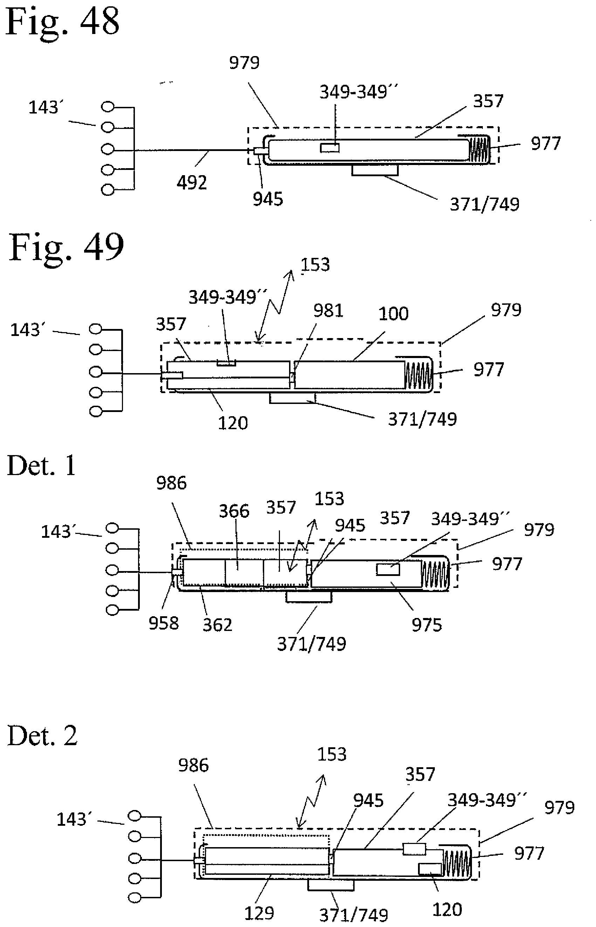

**Fig. 48** is drawing control module in holter with spring.
**Fig. 49** is drawing control module with mobile phone.
   **Det. 1** is drawing control module with further units.
   **Det. 2** is drawing control module with removable accumulator.

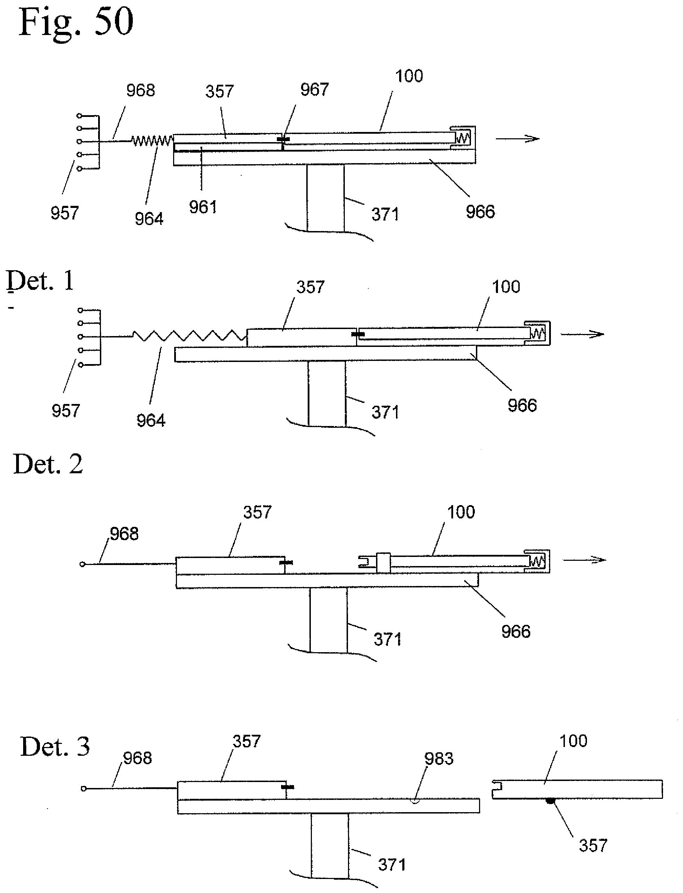

**Fig. 50** is drawing control module with sliding out skids.
   **Det. 1** is drawing sliding out of control module with auxiliary device of control module.
   **Det. 2** is drawing sliding out of control module.
   **Det. 3** is drawing slide out control module.

### Best Modes of Carrying Out the Invention

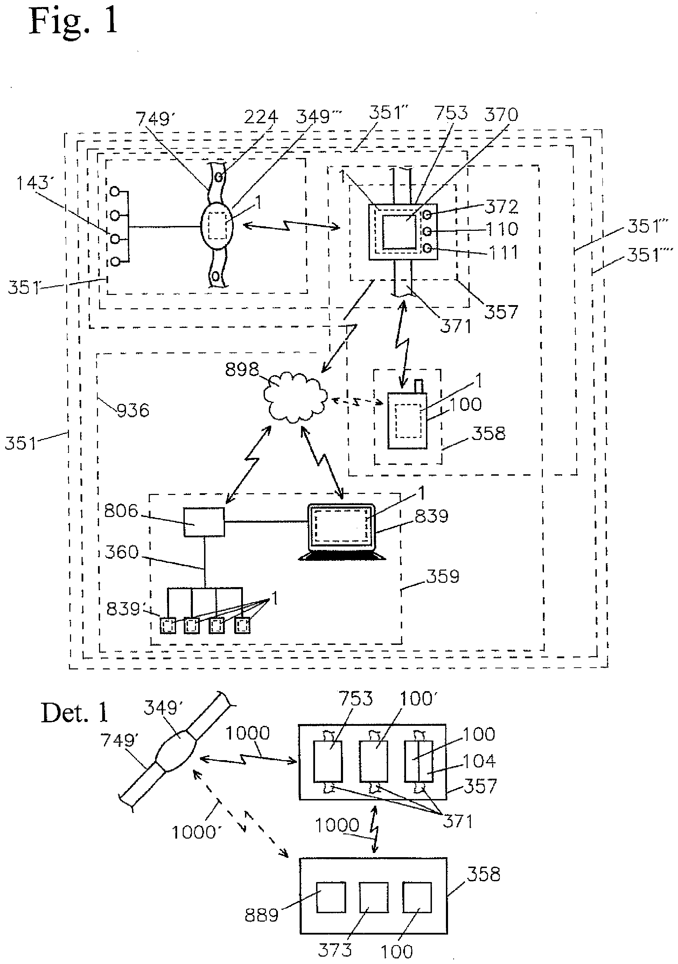

**Fig. 1** represents clearly the main principles of a holter 351, generally, which is abbreviated name, used in medical practice for a Holter's device for long/term registration of heart beats and their evaluation, which is used in some embodiment's examples. There are presented its parts such as blocks, modules and units of which holter 351 is composed and are described its functions. Holter 351 is generally equipped preferably with parts which are necessary for chosen function and mode and differ according to holter's application. The basic function of a holter is to download, process and store heart signals and deliver them into chosen evaluating and imaging device 936 made up of an conventional control module 357 , or/and displaying module 378 and/or evaluating block 359.

For basic function is an conventional holter 351 preferably made up of a holter 351' for basic function with a heart signals monitor 349‴ with a basic equipping, namely with a memory made up of SD chart, which is adapted for downloading data into evaluating device 936 made up preferably of PC 889, 839.. A heart signal monitor 349'" , controlled preferably by pushbuttons located on itself is preferably placed on an conventional chest strap 749' equipped preferably with contact areas 224 of chest strap, which make electrodes for heart signal sensing, or with external electrodes 143'. For operational displaying from memory or live and for control in details is holter 351' extended and preferably constituted of a holter 351" for communication with control module 357 made preferably up of a conventional control unit 753, which is equipped with displaying element of 1 holter, preferably made up of a display 134 of control module, preferably on bracelet 371 of control module.

For local displaying in details I the conventional holter 351 extended and preferably formed of holter 351‴ for communication among close cooperating units preferably with imaging module 358 and imaging element 1 of holter and made up with bigger display 134' of imaging than display 134.

For long range displaying is holter 351 extended and preferably made up of holter 351ʺʺ for communication among remote cooperating units, preferably with evaluating block 359 with imaging unit 1 holter, preferably formed of display units comprised inside .Blocks, modules and units of holter 351 have software of the holter for mutual communication, imaging and evaluation and the results are preferably imaged from the memory or LIVE on displaying elements 1. holter on displays with which is the holter equipped.

An conventional control module 357 preferably controls an conventional heart signal monitor 349‴, which is placed remotely, for instance on the chest strap 749' or in situ, for instance in control module 357 . It controls namely filters for application of holter for different breathy activity, for instance resting, walking, running etc. so as the resulting curves were not by these activities affected and distorted. Filters are preferably analog in "front end " unit ECG 362 , or are software processed in control unit of an conventional monitor 349"" of heart signals. For instance further are selected types of some data such as ECG, heart rate pulses and others.

Heart signal monitors are preferably equipped with appropriate parts for performing tasks as described further and for conventional equipping which is not specified is mentioned conventional heart pulses monitor 349‴ , and likewise as other units with attribute conventional means any selected parts.

The conventional chest strap 749' has contact areas 224 which form electrodes for sensing heart activity placed on it and preferably further external electrodes 143', for instance glued ones, which are used in case when a multi-lead ECG is needed which conventional contact areas 224 on conventional chest strap 749' does not enable.

The conventional chest strap 749' detects heart pulses which are processed in conventional heart signal monitor 349‴, which is wirelessly connected, preferably by means of Bluetooth with a conventional control module 357 made up of conventional control unit 753 , where it sends in digital form. There it is processed for imaging on the display 370 of control unit. Conventional control module 357 is preferably placed on bracelet 371 of control unit and is in terms of space smaller than standard mobile phone, in order to be comfortably carried on wrist with also appropriately smaller display 370 of control unit suitable for instantaneous watching data and curves. The conventional control module 357 is wirelessly connected preferably by means of Bluetooth with the imaging module 358, made up preferably of mobile telephone 100 , where data and curves can be seen in details. With evaluating block 359, which preferably consists of a server 806 and is a conventional control module 357 connected by mean of mobile operator 898 and/or WIFI. On displays of PC 839' of participants connected with the server 806 preferably by means of connection 360 are preferably imaged data of holter processed in monitor 349‴ preferably with imaging ECG, arrhythmia in curves or degree of arrhythmia and/or in percentage of irregular pulses from total number of pulses in definite time interval, variability, curve of heart pulses with curves of limits for regular pulses and other data and curves. PC 839 of operator is preferably also connected to server 836. The conventional chest strap 749' is preferably controlled from conventional control module 357 and/or displaying module 358. At exceeding of preset data limits, the conventional holter 351 sends of a warning tone from selected unit, which can be easily canceled by pushbutton reset 111, otherwise data that exceed limits is send into evaluation block. The conventional control module 357 controls preferably as well standard mobile telephone 100 in a standard imaging 358 and downloads from it data and displays such as phone number of calling person and is adapted for receiving a phone call or SMS coming to a standard mobile phone 100, which preferably displays. Thus holter becomes universal with a possibility to ensure also phone and data operation of standard mobile telephone. The conventional control unit 753 is preferably equipped with pushbuttons of control unit 372, from where controls jointly with conventional heart signal monitor 349‴. Alternatively control is realized from touch display 370 of control unit. The control unit 753 is as well equipped with panic button 110 for alarm initiation in case of emergency.

Detail 1 shows survey of examples of communication of conventional chest strap 749' provided with a conventional monitor 349' with control module 357 and displaying module 358. The conventional control module 357 is preferably made up of a conventional control unit 753 or an adapted mobile phone 100', or auxiliary device 104 with a standard mobile telephone 100 preferably of smaller size on a bracelet 371 of control unit for emergency watching data, curves and other quantities.

It communicates with display module 358 preferably by means of wireless connection 1000 which is provided with a bigger display for imaging details. The imaging module is made up preferably of standard devices such as mobile telephone 100 or a tablet 373 and/or PC 889. Conventional control, module 357 is standalone and does not need further units for control of conventional chest strap 749' and imaging. In this case is provided with sufficiently big display 370 for simultaneous imaging more graphs and is not on bracelet for achieving of more detailed information.

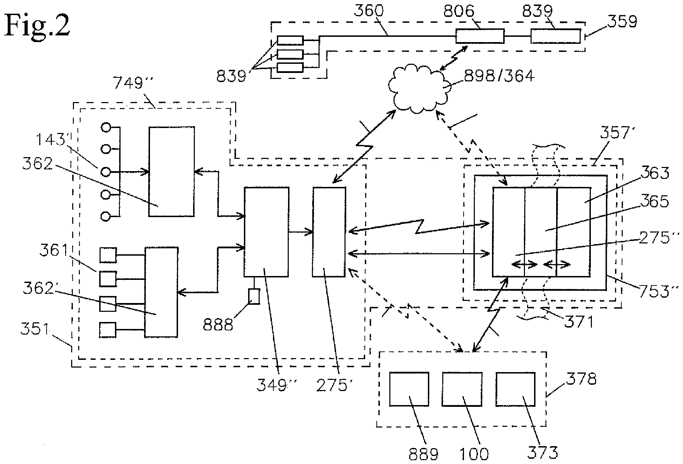

The imaging module 358 also alternatively works independently as the control without conventional control unit 357 and communicates preferably directly with a conventional chest strap 749' through wireless link 1000 of holter. In case the imaging module 358 is formed by a standard mobile telephone 100 or tablet 373, especially when data is processed on background of SW of mobile telephone 100 or tablet 373, than it is preferable to use maxi chest strap 749" with a heart rate monitor presented on Fig. 2, where signals are complex processed and inj imaging module 358 are only imaged and controlled. Data is alternatively processed in imaging module 358, but the less data is processed in monitor 349‴ on conventional chest strap 749' , the less accurate is the result of module 358 made up of a standard device, because simultaneously are processed other programs, for instance standard mobile telephone 100 for telephone calls and further applications which have currently priority.

The control module 357 and imaging module 358 control mutually each other and display and imaging module 358 is preferably adapted for control conventional chest strap 749'. The conventional control module preferably displays operatively data from imaging module 358 especially when is made up of standard mobile telephone 100, for instance data from phone calls and SMS which can take over and can realize a phone call or display SMS.

**Fig. 2** presents an embodiment according to the invention, showing a holter 351 with maxi chest strap 749" with a heart rate monitor 349" and sensors for processing of analogue heart signals and preferably signals and data from sensors 361 which are processed in "front end" unit 362 of heart signals and "front end "unit 362 sensors and after digitalization processed in heart signal sensors monitor 349", from where the communication unit 275' of the chest strap sends it wirelessly to a mini control module 357', which is preferably made up of a mini control module 753" preferably with touch display 363. The communication unit 275" receives data in digital form and hands them over to the control unit 365, which processes it and from touch display 363 is controlled maxi chest strap 749" with heart rate monitor.

Communication unit 275' and communication unit 275" communicate preferably with imaging module 358 made up of a mobile telephone 100, and/or a tablet 373, and/or a PC 889 and preferably through network of mobile operator 898 and/or WIFI 131 also with the evaluating block 359 made up of server 806, a PC 839 of operator and PC 839 of participants.

In maxi chest strap 749" with the monitor of heart signals, there is placed a removable SD memory chart SD 888 for data recording of heart signals monitor 349" and sensors which is easy to download into PC 839' of participants of evaluating block 359 or PC 859 and to display it.

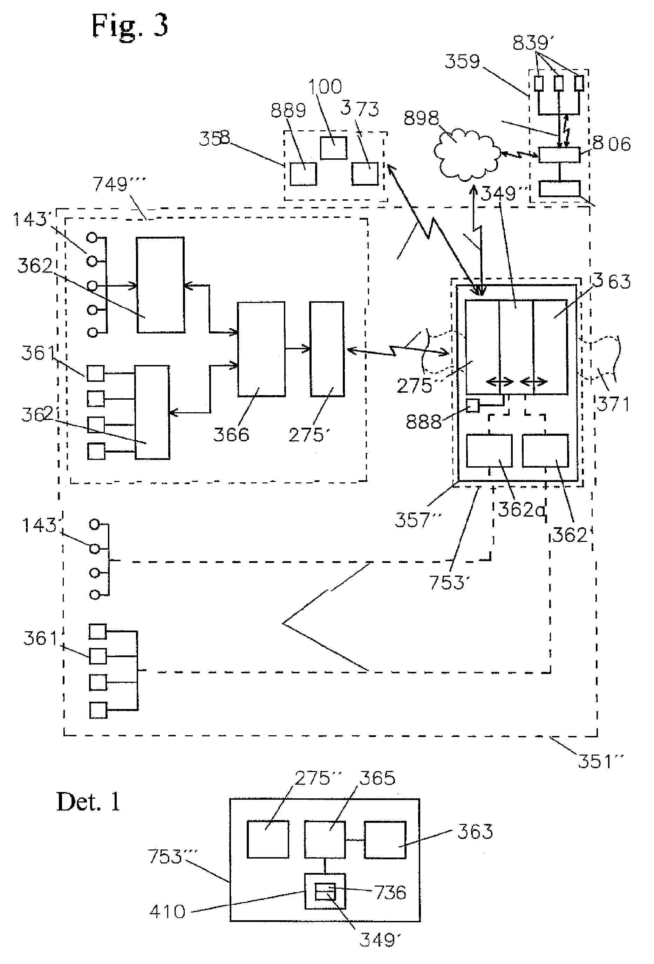

**Fig. 3** represents a conventional holter 351 with a mini chest strap 749‴ without monitor of heart signals, where heart signals and preferably signals from sensors 361 are processed in "front end" unit 362 of heart signals and a "Front end" unit 362' of sensors and led to digitizing unit 366 from where they are send as digital data wirelessly, preferably through Bluetooth from communication unit 275' to cooperating communication unit 275" placed in maxi control module 357' in maxi control module 357" made up preferably of maxi control unit 753' , preferably with a display 363 and heart signals and heart signals and sensors monitor 349" . Here lead heart signals and/or signals from sensors which are processed in "front end " unit 362a of heart signals and "front end" 362' and then in monitor 349" of heart signals and sensors and are in control unit and microprocessor processed. In maxi control unit 753' is placed also a removable SD chart 888 for playback monitor 349" data of heart signals and sensors data into PC 839' in displaying module 359.

**Det. 1** shows the control unit 753‴ which contains preferably the module of auxiliary units 410, heart signals monitor 349' and enables monitoring heart signals and quantities for which it was adapted. The control unit 365, controlling units is data connected with control unit 736 of module of 410 of auxiliary units.

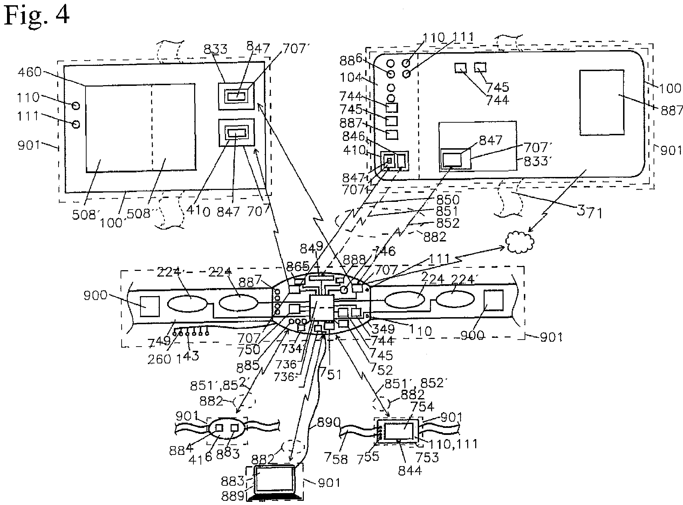

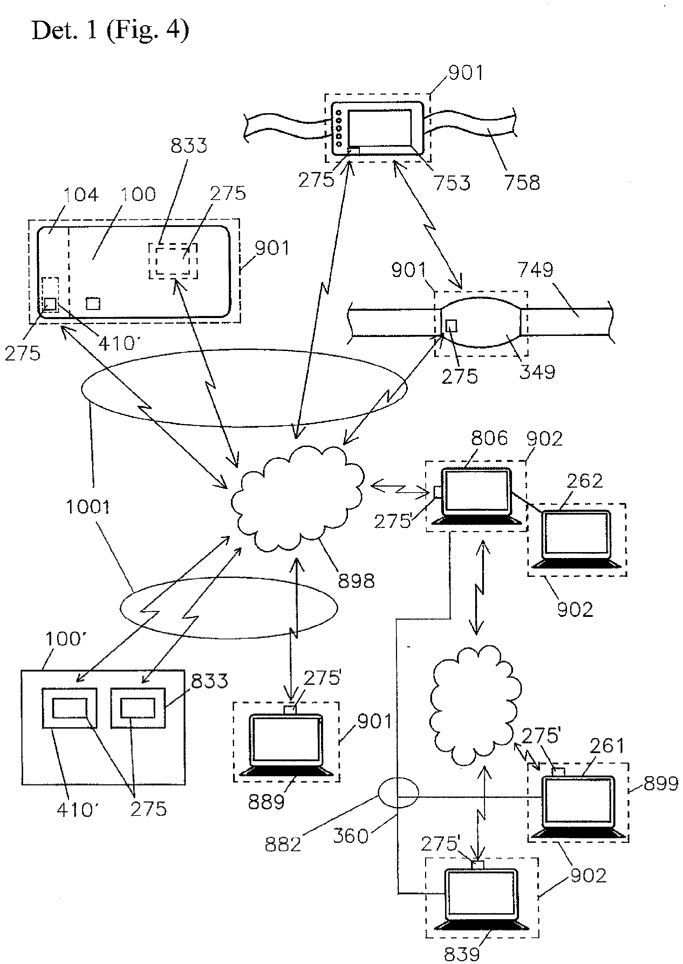

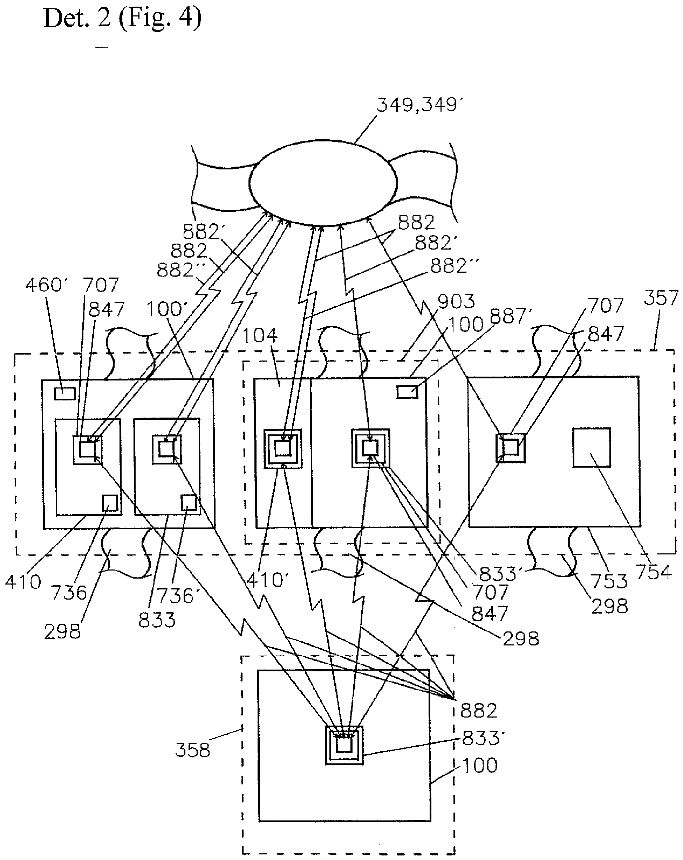

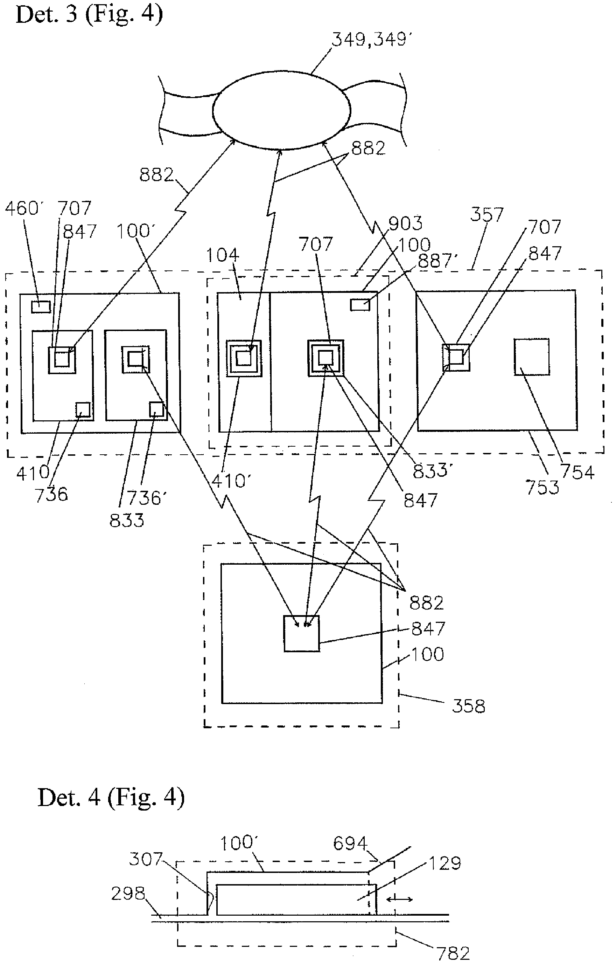

**Fig. 4** illustrates monitor of heart signals 349 self-sufficient and its communication with near cooperating units 901 by means of wireless connection 882, preferably realized by Bluetooth or ANT. It is also shown linking-up of the control unit 753 and/or mobile telephone 100 ' and/or auxiliary device 104 with standard mobile telephone 100 to self-sufficient heart rate monitor 349 , placed on chest strap 749, sensing and evaluating physiologic and bioelectric signals, which processes into numeric value or heart pulse with limit curves of arrhythmia and/or arrhythmia and/or variability and/or ECG curve and/or further particulars on human body with pairs of contact areas 224 and/or contact areas on 224' on chest strap 749 where are preferably placed electrodes 143, glued or further according to requirements, adapted for continual sensing of bioelectric signals. The self-sufficient heart signal monitor 349 is modified for stand-alone operation and serves fully as a holter 351 without necessity of connecting other units, whereas these units can be connected according to needs.

Self-sufficient heart signals monitor 349 contains amongst other pushbutton 111 reset, pushbutton panic 110, unit of wireless communication 707', comparing unit 750 and communicates by means wireless connection 851', 5.5kHz, or wireless connection 852' 2.4GHz for communication with clock 416 provided with clock control unit 884, bracelet 936.

Monitor communicates preferably with a mobile telephone by means of wireless connection 882 preferably provided with pulse receiver 843 in frequency band 5.5kHz for receiving of signals of heart beats. Sensed physiologic and bioelectric signals from contact areas 224 are led into control unit 736 which evaluates signals and if signal exceeds the preset limits, sends acoustic warning signal 745 or through some near cooperating unit 9010, monitor 349 is alternatively adapted for backup operation and physiologic and bioelectric signals are preferably scanned from two contact areas 224 and 224' and are led preferably led into two independent control units 736 and 736' which ensure independent processing and preferably continually compare results. In case of different results or in case of exceeding of preset limits a warning acoustic signal is triggered, preferably on a loudspeaker, piezo-converter, or vibrator 744 or by wireless connection 882 through cooperation units made up preferably of adapted mobile telephone 100' or auxiliary device 104 with standard mobile telephone, control unit 753 and in case that the warning signal is not canceled with the pushbutton reset 111 in selectable time, then chest strap or selectable units sends alarm into selectable remote unit preferably made up of server and/or other suitable device, at further participant preferably with PC 889 or by standard mobile telephone 100.

Results from at least one control 736 , 736 are preferably transferred by wireless connection 882 to graphic and numeric processing and imaging into near cooperating units 901 of standard mobile telephone 100 and/or to module 410 of auxiliary devices in auxiliary device 104 connected with mobile telephone 100 and/or watch 416 and/or PC 889 and/or imaging and controlling units 753 preferably on bracelet and/or further, whereas wireless connection 882 is preferably made up of Bluetooth and/or Bluetooth low energy and/or wireless connection 2.4 GHz, or wireless connection 5.5kHz and/or ANT and/or WIFI whereas image 883 on mentioned units comprises preferably values of arrhythmia and/or variability and/or pulse and/or further health values with appropriate curves with numeric values, preferably ECG, preferably in control units 736, 736' evaluated and processed from heart signal and appropriately in displayed units processed further for displaying.

Next alternative is the results obtained in one control unit, for instance control unit 736 transfer through one communication channel, without a help wireless connection 882 to module 410'of auxiliary units preferably to a unit of wireless communication 707' made up of unit Bluetooth 847 and results obtained in the second control unit 736' , for instance through second channel to mobile telephone 100 preferably to a unit of wireless communication 707 , made up preferably of a unit Bluetooth 847 and subsequently to compare both results preferably in module of auxiliary units 410'of auxiliary device 104 and/or in a mobile telephone 100 and preferably to display results independently and in case of different results ti initialize warning signal. Alternatively signals of two channels are used in the some units separately without comparison as back-upping, for case when one of them failed or in simplified operation is used only one channel an only this is proceeded and displayed. Alternatively is preferably used only one pair of contact areas 224 led to both control units 736 and 736' so backup starts only from them. Monitor 349 is preferably. together with described units contactable , namely mobile telephone 100 and/or auxiliary device 104 adapted for full backup, processing, and displaying of physiological and bioelectric signals, preferably curves and value of pulse ECG, arrhythmia, and other from contact areas 224, 224' electrodes 143 up to display with imaging of both channels and all system is doubled whereas mobile telephone 100 with display is doubled with auxiliary device 104 with display 887 or mobile telephone 100' is doubled with module 410 and electronics 833 of mobile telephone working independently or is doubled in module 410 with two or more independently working microprocessor or is system alternatively back-upped only partly, where is its part doubled, or is not back-upped and monitor 349 is equipped only with one pair of contact areas 224 and one control unit 736.

Control unit 753 is preferably fixed on bracelet 936 and contains pushbuttons 927, display 754 for processing and displaying data and preferably appropriate curves including ECG and which except processing and displaying is designed for monitor 349 of heart signal control and forms independent unit working without further units. Small control unit 753 is adapted for functions necessary for control of monitor 349 and preferably also further near units for simple imaging in contrary with mobile phones 100, 100', which is designed for more accurate imaging.

When values of evaluated parameters exceed some of selected limits warning is triggered by vibrator 744 and/or source of acoustic signal 745 preferably loudspeaker or piezo-converter in monitor of heart signals 349, and/or according to selection preferably in some of close cooperating units. In case when warning signal is not cancelled by reset button 111 on monitor 349 or further close cooperating units 901 within the preset time interval, the system sends off alarm, which is preferably transferred automatically to remote units 902, surveillance center 899 or further PC 839 as data or voice.

A self-sufficient, separate, heart signal monitor 349 without mobile telephone 100', or auxiliary device 104 with mobile telephone 100 , whereas is preferably controlled by pushbuttons 885, 927, 926 and/or further, and in its control unit are mentioned signals proceeded , which in case of exceeding limits triggers a warning signal 745 generated or vibrations by means of vibrator 744. In case when the warning signal is not cancelled by pushbutton reset 111 within pre settable time period, monitor 349 sends off alarm through the network of mobile operator 898 to remote cooperating units 902 . Warning signal is settable and preferably coded which informs of kind of alarm and the reason of alarm. Setting functions of monitor 349 of heart signals are preferably done by control buttons 885 on monitor of heart rate 349 without necessity of connecting further device or control unit 753, or for instance mobile telephone 100, 100'.In this case monitor 349 informs the user about measured quantities by voice signals by means of s loudspeaker 694 or acoustic converter 853 as mentioned above.

In case of connecting the auxiliary device 104, control is performed on buttons 138 or touch display 887 of auxiliary device, which preferably displays curves and quantities on display 887, preferably independently on mobile phone 100 for parallel imaging on display for parallel imaging on display 887' and/or back-upping.

Monitor 349 of heart signals contains preferable a display 865 numeric and/or curves for imaging most important statements , preferably arrhythmia, heart rate, numeric or bar chart of heart pulses with limits of arrhythmic pulses of ECG, variability and further data and/or touch display for simultaneous control and displaying mentioned values and curves.

Heart signal monitor 349 contains preferably WATCHDOG 734 watching its proper activity and triggering alarm as described on Fig. 12, in case when control unit 736 and/or control unit 736' fails, and doesn't send " refresh signal". Heart rate monitor 349 preferably supervises the regular activity of mobile telephone 100' and/or auxiliary device 104 and/or mobile telephone 100' , and/or control unit 753 and/or sport watch 416 and further units so, that mentioned units send on wireless 882 optional code or result of ordered task, preferably mathematic to heart rate monitor 349 . In case that code or result is correct, the control unit 736 and/or 736'sends "refresh signal" to watchdog 734. If refresh signal does not come in proper time, or is not correct, monitor 349 doesn't send the "refresh signal "and in watchdog 734' is initialized alarm . Alternatively monitor 349 triggers alarm independently of watchdog 734 in case when receives improper code. Signal lights 931 and/or the type of acoustic or vibrating alarm indicate the reason of alarm. Besides that signal lamps can provide information the charge of battery heart pulses and further information

A memory SD chart 888 , preferably removable , preferably stores data from heart rate monitor 349, which can be transferred into PC 889 or other suitable device, preferably relocating, or by means wireless connection 882 or USB connection by means of connection 890. In this way the monitor 349 is modified for use as a "Holter" with advantage of continuous monitoring and simultaneous live evaluation on display 460, 887, 754 and further information on heart pulse and/or arrhythmia and/or variability and/or ECG. Instead of memory chart SD 888 in monitor 349 can be used SD chart 888' in auxiliary device or mobile telephone 100, or in mobile telephone 100'.

Monitor 349 is placed preferably on chest strap 749 or out of it preferably on bracelet on wrist or in module of auxiliary units 410 for instance mobile telephone 100' or module 410' of auxiliary units, for instance auxiliary device 104 or in control unit.

Electrodes 143 ECG preferably glued are connected with cables 267 to heart signal monitor 349 in sufficient amount so as ECG would be adaptable for measuring fro 1 up to 12 electrodes with backup data.

Connection to remote units such as server 806 is realized preferably by means of network of mobile operators 898. Access to server has preferably surveillance center 899 and PC of participants 839. Connection through network of mobile operator has auxiliary device 104 and mobile telephone 100 preferably for data backup.

Alternatively is instead system 903 mobile telephone 100with auxiliary device 104 or simultaneously used a mobile telephone 100', which has the same functions as the system 903 and fully it replaces .Advantage of system 903 is in that, that auxiliary device 104 is removable and is applied in case of need and with such kind of auxiliary device 104 where its functions fulfill momentarily needs and in outfits preferably with selectable sensors from the set of auxiliary device 104, so the mobile phone extends with selectable functions only in case of need which means that mobile telephone 100 is smaller in terms of size and less expensive,. Auxiliary device 104 contains module 410' which is removable from mobile telephone 100, while in mobile telephone 100' is module 410 firmly installed. Electronics 833 of mobile telephone 100' is contained also in mobile telephone 100 as electronics 833' of mobile telephone with the same functions, auxiliary device 104 with mobile telephone 100 contain a unit of wireless communication 707 placed in module 410 410' preferably made up of unit 847 Bluetooth or ANT for communication of near cooperating units 901.

**Detail** 1 shows communication through network of mobile operator 898 , near cooperating units, which are preferably monitor 349 , mobile telephone 100' , mobile telephone100 with auxiliary device 104 and control unit 753 with remote cooperating units 902 preferably with the server 806 where is stored information connected with operator's PC 262 whereas information is available also for further remote cooperating units 902 such as PC 839 of authorized members, for instance to medical staff relatives and to others and surveillance centre. Mentioned communication is enabled by communication unit 275 with parts for data and/or voice communication by means of operator's network. Alternatively are remote members 902 namely PC 889 and PC 839 connected by internet.

**Detail 2** presents control module 357 formed preferably of mobile telephone of smaller size 100' preferably on a band 298 , on wrist in imaging module 358, whereas mobile telephone 100' communicates with monitor 349, or monitor 349' preferably simplified without backup ability. For backup the mobile telephone 100' preferably communicates with monitor 349 on two channels by means of wireless connection 882 to module 410 also with electronics 833 of mobile telephone. Backup communication can be alternatively instead of communication with module 410 and electronics 833 of mobile telephone realize only with module 410, where the second channel of wireless connection 882" is preferably processed by second microprocessor in control unit 736 of module 410. Mobile telephone 100' on band 298 of smaller size is processing data in control unit 736' of module 410 , separated from control unit 736 of mobile telephone working reliably and preferably provides with data a standard telephone 100 of bigger size, which ensures detailed imaging on bigger display then mobile 100' on band 298

Preferably is on mobile telephone 100' on wrist operatively instantaneous preview on smaller display 460', and in mobile telephone 100 detailed outlook preferably on bigger display, which is not instantaneous when put in the pocket. Mobile phone 100' on band 298 ensures reliability of data processing by separate control unit 736 and instantaneous preview on display 460 whereas mobile telephone 100' is of smaller size for carrying on wrist and instantaneous preview. Imaging with all details ensures a bigger mobile telephone 100. Mobile 100'works independently of mobile phone even if is not connected. In case of communication without storing only on one channel of wireless connection 882 is alternatively instead of monitor 349 used monitor 349' simplified in economic embodiment, which is not adapted for backing up , Is more simple with advantage of power consumption

Mobile telephone 100' placed preferably on bracelet 298 displays prompt preview and image of optional data from mobile telephone 100 placed mostly in the pocket, for instance data on incoming calls or SMS which preferably picks over. For described communication with monitor 349, 349'is control module 357 instead of mobile telephone 100' alternatively made of system 903 with auxiliary device 104 and mobile telephone 100 or control unit 753.

In case of connection of imaging module 357 preferably made up of mobile telephone 100 , from economical and dimension reasons the mobile telephone 100', or control unit 753, or system 903 are preferably not equipped with parts for communication in network of mobile operators is used mobile telephone 100, which preferably makes displaying module 357

**Detail 3** presents a simplified version of Detail; 2, where connection by means of wireless connection 882 between monitor 349, 349' preferably situated on chest strap 749 and mobile telephone 100' or system 903 or control unit 753 preferably with a module 410, 410' is not back upped and data from electronics 833 and 833' of mobile telephone 100', or system 903 or control unit 753 are sent to mobile telephone 100 for high resolution display.

**Detail 4** introduces mechanism 782 for removing the basic accumulator from lateral side, for instance at mobile phone 100' on a band 298 of mobile telephone by the help of door 694, which pushes the basic accumulator 129 to contact 307 and locks.

**On** **Fig. 5** is displayed a mobile telephone 100' with auxiliary functions with mechanism 272 for exchange of basic accumulator 129 and a block of auxiliary modules 152 , necessary for realization of auxiliary functions, namely easy withdrawing of basic accumulator without need of removing the back cover of mobile telephone or auxiliary device preferably without interrupting of operation and with advantage on unit of electronics of mobile telephone 833 independent data and application processing in block 152 of auxiliary modules and preferably results are displayed on display 506auxiliary, independent of display of mobile telephone 460 and/or on a part of screen (split screen) 508" electronic of mobile telephone and/or alternatively is displayed subsequently once from electronics of mobile telephone 833 and then from module of auxiliary units 410 of mobile telephone on display 460 of mobile telephone. Interface 508 splits both parts of split screen. On Fig. 5 is a mobile telephone 100' with auxiliary functions with mechanism 782 for basic accumulator 129 exchange and block 152 of auxiliary modules for realization of these auxiliary functions, namely easy withdrawal of basic accumulator without need of opening the back cover of the mobile telephone or auxiliary device preferably without interrupting the operation and preferably on unit of electronics 833 of mobile telephone independent data and application processing in block 152 auxiliary modules and results are preferably on displayed 506 and on display 460 of mobile phone and/or on split screen 508" module of units and result from electronics 833 of mobile telephone on a part of split screen 508' electronics of mobile telephone and/or alternatively is displayed subsequently, once from electronics 833 of mobile telephone and then from module 410 of auxiliary units of mobile telephone on display 460 of mobile telephone. Interface 508 splits both parts of this screen.

**On Detail 1** is from parts which mobile telephone 100' consists with auxiliary functions is presenter electronics 833 of mobile telephone with control unit 736 with microprocessor of electronics of mobile telephone, mechanism 782' for exchange of auxiliary accumulator with tipping casing 118 , lock of door 673 and contacts 307 for connection of accumulator and block 152 of auxiliary modules comprising module 715 supplying electronics of mobile telephone, source 836 of bridging energy made preferably by bridging accumulator 688 and module 410 of auxiliary units of mobile telephone with control unit 736 with microprocessor interconnection of units is carried out with data 507 connection and with energy connections 507'. Alternatively there is a belt 298 fastened to mobile telephone 100' creating a bracelet for fixing mobile telephone on wrist. The bridging accumulator 688 is smaller in size in comparison with the basic accumulator 129 because it ensures the operation of mobile telephone 100' only short time during the basic accumulator 129 exchange. The basic accumulator 129 which has higher ampere- hour capacity and is bigger than bridging accumulator 688 ensures operation of mobile telephone 100' and is exchanged with charger one during the operation, thanks the source 836 of bridging energy, which supplies electronics 833 of the mobile telephone and module 410 of auxiliary units through supplying module715 supplying mobile telephone during accumulator exchange. For saving the space for source 836 of bridging energy, preferably made by bridging accumulator 688, preferably for delivery of bridging energy during the basic accumulator 129 exchange through connector 102 and cable 256 of charging, is connected charger with 755 or modular charger 755' or external bridging energy source 836' not mentioned on picture, or case of bridging energy preferably replace 886 which can source 836 of bridging energy preferably replace. Instead of internal source 836" of bridging energy placed in mechanism 782' for exchange of auxiliary accumulator preferably used external source836' of external bridging cable 256 and connector 102. In this case the bridging accumulator 688 can be skipped out.

Data connection of module 410 of auxiliary units and electronics 833 with external devices 154, 154' is preferably realized by means of wireless links 153 ensured preferably by Bluetooth, or through connector 691' for data connection of module 410 and through connector 691 for data connection of electronics of mobile telephone. Independent data processing of block 152 of auxiliary modules on the unit of electronics 833 of mobile telephone is carried out by putting at least one microprocessor into control unit 736' in module 410 of auxiliary units in block 152 of auxiliary modules working independently of microprocessor in control unit 736 of mobile telephone, which communicates with data connected by independent wireless link 153 or through independent connector 691' on connector 691 for electronics 833 of mobile telephone working also independently .Independently working microprocessor is alternatively placed in control unit 736 of electronics of mobile telephone. Alternatively is also placing independently working microprocessor to chip of multi-core microprocessor of control unit 736 of mobile telephone, preferably as one of core, which is not influenced by remaining cores as much as can be affected its independence and reliability.

**Fig. 6** presents the module 410 of auxiliary units, preferably made up by a mobile telephone, which contains a unit of wireless communication 707 and/or Bluetooth and/or ANT and/or WiFi and/or network of mobile operators and other appropriate wireless media, further module 410 of auxiliary units comprising watchdog 734 which produces warning signal in case of malfunction of mobile telephone 100', a unit of position detection 735, preferably accelerometer or gyroscope, control unit 736 with at least one microprocessor which is adapted to work independently of microprocessor of mobile telephone 100' and is modified for independent and simultaneous processing of not only applications processed in electronics 833 of mobile telephone, but also other applications concerning health and sport, module 410 of auxiliary units, further contains a receiver 843 of pulses 5.5kHz and further electronic units 290b internal, preferably adapted for health applications processing. Unit 599 for hearth signals processing formed preferably by monitor 349-349‴ processes heart signal from external electrodes 143' located preferably on a chest strap or glued and wire connected through a connector 789 ECG or from internal electrodes 143' fixed on surface of mobile telephone 100'. Alternative is connection by means of wireless links 153 and unit of wireless communication 707 to external source of signals ECG preferably to chest strap 749 -749‴.

The unit 599 for heart signal processing preferably made up of monitor 349 - 346‴ of heart signals processes preferably heart signals to ECG curve and/or curve of heart pulse, and/or curve of arrhythmia; and/or calculated degree of arrhythmia, variability and data transfers to control unit 736 for further processing and imaging. The unit of internal detector 290 processes data and signals from detectors 788 located on surface of mobile telephone 100' and/or external detector 788' connected through detectors_of probes 861. Probes 798' ascertain health quantities, for instance test of ingestion of alcohol or drugs, saturation of blood with oxygen, level of diabetes, dilution of blood INR, breathing frequency, blood pressure and other functions according to connected probes in connector 291'. The external unit of detectors extends function of internal unit 290' of detectors and is connected through auxiliary connector 291'. Wireless inductive link 153' 5.5kHz mediates connection of the module 410' of auxiliary units with for instance sport watch with receiver of pulses preferably on 5.5kHz.

The module of auxiliary units 410 is connected through connector 691 of mobile telephone independently of electronics 833 of mobile telephone as well as through independent wireless connections 153. Control unit 736 is preferably adapted for direct communication with internal module 914 of monitoring units and external module 914' and in this case modules 914, 914' are carried out in control unit 736 of mobile telephone by links 933 of modules of monitoring units and to data link 507. The advantage of data processing in module 410 of auxiliary units is reliability, which is higher than at processing in electronics 833 of mobile telephone because this electronics processes process also other tasks of mobile telephone and further applications, whereas module 410 of auxiliary units processes only selected tasks and in case of need can be amended with more microprocessor for further increase of reliability. Through connector and/or electronic interface 285 can be connected modules 914' of monitoring external unit external units which preferably involve probe 287 of health functions, external unit 290a for testing health functions, further unit 290" for alcohol test and/or drugs external with the probe 798", unit of detectors external, the next advantage is connecting electrodes 143‴ and sensors located on steering wheel 934 of a car through connector 789 ECG enabling continuous monitoring of health condition of a driver, preferably in public transport.

**Fig. 7** illustrates standard mobile telephone 100 with auxiliary functions in auxiliary device 104 , which is provided with mechanism 782 for exchange of basic accumulator 129 and block 152' of auxiliary modules for realization of auxiliary functions, namely easy replacement of accumulator 129 without necessity removing of auxiliary device, preferably without interrupting function of the mobile telephone and preferably independent of electronic unit 833 of mobile telephone and so independent data and application processing in block 152'of modules in auxiliary device, which is realized similarly as described on Fig.1 Module 410 of auxiliary units is connected with external device 154 connected through connector 693' and through connector 693‴ the third with external device 154' further and module 410 of auxiliary units is adapted for mediate communication between external device 154'and mobile telephone 100, thereby replaces data communication of mobile telephone from connector 691, which is at setting of auxiliary device104 blocked. Auxiliary device 104 is adapted for easy exchange of basic accumulator 129 for charged preferably without interruption of operation of mobile telephone 100 without extension of the thickness. The auxiliary device 104is to mobile telephone 100 preferably removable and contains upper side 709 which lengthen it to longitude, or extend it to width whereas the thickness remains nearly the same or is preferably enforced by the side of auxiliary device 104, which contains source of bridging eneggy836, which is made by bridging accumulator 688 in this case illustrated, or accumulator or bridging capacitor 808 and module 715ʺʺ of supply of auxiliary device which feeds from basic accumulator 129 through connector 691 mobile telephone, connector 693 of auxiliary device and connection 716 feeding auxiliary device 104 and during the basic accumulator 129 exchange supplies from source 836 of bridging energy standard mobile telephone 100. Switching the module 715ʺʺ of supplying auxiliary device 104 for supplying mobile telephone 100 ensures change-over switch 703. At mobile telephones that do not enable supply energy to auxiliary device 104 through connector 691 of mobile telephone and/or exchange of basic accumulator 129 without breaking the mobile telephone 100 operation even at charging through connector 691 , energy for supplying auxiliary device 104 can be get through contact springs 678 and bridging cable 686 from basic accumulator 129 , where contacts 711 of basic accumulator abut against contact spring 678, where the opposite side abut against contacts 307 of mobile telephone, whereas preferably bridging cable 686 is fixed to contacts 307 for instance by soldering. Supplying of mobile telephone 100 at exchange of basic accumulator 129 goes through connector 307 and bridging cable 686 is supplying module 715" switched over from charging external source 386 is module 715' of supply switched over from charging external source 836' of bridging energy for supplying standard mobile telephone100 in time of basic accumulator 129 preferably with a change over switch 703 manual, or electronic, with automatically controlled switch 702 in time of opened door 708 at exchange of basic accumulator 129, connected with cable 646. change-over switch 703 is alternatively controlled from standard mobile phone 100 through connector 691 with data signals, which are processed in module 410 of auxiliary units and controls change over switch 703 in module 715"of supply. Supplying of mobile telephone 100 from auxiliary device is preferably carried out through connection 716 and by connectors 691, 693 preferably USB feeding and communicating, at mobile telephones 100 that enable it after switching over. For saving space, necessary for external source 836' of bridging energy of the charger 755 is preferably for its replacement the bridging energy is lead from external source 836' of bridging energy made up of basic accumulator 129 placed preferably in charger 755 connected for bridging exchange of basic accumulator 129 in mobile telephone 100 with bridging external cable 686' Source of bridging energy 836 is preferably made up of bridging accumulator 688 placed preferably in mechanics 783 for exchange of bridging accumulator 688 , which is preferably applied for supplying of auxiliary device 104 and mobile telephone 100 through connectors 693, 691 in time exchange of basic accumulator 129. In case when mobile telephone 100 is not able suck energy from connector 691 of mobile telephone, than it is realized connection for connecting by means of contact 307 basic accumulator 129. Accordingly is preferably done substitution of source 836 of bridging energy for external source 836' of bridging energy shown on Fig. 1, where it is alternatively substituted source of bridging energy 836'. Auxiliary device 104 contains block 152' of auxiliary modules of auxiliary device with module 410 of auxiliary units with control unit 736 with microprocessor in block of auxiliary device

**Fig. 8** illustrates module 410' of auxiliary units, which is provided with the same units with functions as module 410 of auxiliary units of mobile telephone 100 as mentioned above on Fig. 6 Connector 693 of module of auxiliary units of auxiliary device enables connection with mobile telephone 100, second connector 693' of module of auxiliary units ensures connection of external device and the third connector 693" of module auxiliary units for connection with further external devices. Control unit 736 with microprocessor is provided at least with one microprocessor and is adapted for data processing in application independently of control unit of mobile telephone100.. Control unit 736 is preferably modified for direct communication with module 914 of internal monitoring units and module 914' external monitoring , in this case is module 410' realized preferably without control unit 736. Connection of modules 914, 914' is carried out directly to data link 507 of mobile telephone 100 trough connector 693 of module of auxiliary units. On this data link 507 are connected alternatively appropriate sensors by means of link 933 instead of link 935 of control unit. The data processing in module 410 of auxiliary units brings advantage of reliability which is higher than data processing in electronics 833 of mobile telephone, because it is processing also functions and mobile telephone applications, while module 410' of auxiliary units processes only selected application and in case of need can be provided with more microprocessors for reliability increasing.

On **Fig. 9** is presented mobile telephone 100' with module 410 of auxiliary units or auxiliary device 104 with module 410', which_preferably contains module of internal sensors 910 provided preferably with sensors of body temperature 751, pressure detector 917 , detector of sleeping phase 921, light detector 923 , sensor of body temperature 751, detector of sleeping phase 721, light detector 923 for measuring oxygen amount in blood and acoustic signals detector 924.To module 410 is connected a set of external sensors which complete internal sensors such are sensor measuring alcohol level 906, sensor for detecting drugs 907, detector ECG 918, detector EEG, 919, detector EMG 920, heart pulse detector and module of internal sensors connected to sensors 910' located on mobile telephone 100' , or outside of it such as sensors 910". Sensors 908 are preferably located on T-shirt 905 for sensing signals and quantities on selected places on upper part of monitored person, where sensors with wire or wirelessly to module 410, 410; and complete or replace sensors in a set of internal or external sensors and/or are located at chest strap 749 , connected with wire or wirelessly. Sensors and/or external electrodes 143" are preferably located on control system of vehicular or other mean with man service, preferably steering wheel of a vehicle and signals of these sensors are led into mobile telephone on wires or wirelessly on link 153. Preferably also sensed in this way ECG signals led further to ECG detector 918. Modules 410, 410' of auxiliary units preferably in a transport mean or other facility, where driver or staff use chest strap 749 with sensors, or sensors that communicate remotely by radio link through in transport mean installed modules 410, 410', communicate remotely by means of radio connection in mean installed module410, 410', preferably in control module357 and through it are connected with network of mobile operator 898 with a server 806 and drivers and staff supervising center. Control and displaying for staff or driver of transport mean is realized preferably with firmly installed control module with touch screen, the described installed elements are not alternatively inbuilt but separate.

**Fig. 10** illustrates in block detection activity unit /watchdog 734 located in auxiliary device 104, which activates acoustic signalization by acoustic converter 853 when mobile telephone 100 doesn't perform correct course. The mobile telephone 100 is programmed so, that microprocessor unit 859 performs in definite intervals easy tasks, for instance product of several numbers and when the result is same as numbers saved in memory then through connector 691 and 693 sends a code responding to signal refresh to control unit 736 with microprocessor of auxiliary device 104 signal which after processing signal resets the timer 858 which measures time since last reset. If reset comes not earlier than preset time, timer 858 activates acoustic converter 853, preferably loudspeaker or piezo-electric converter and/or vibrator 294, or visual indication 295 preferably LED. Watchdog 734 located in a mobile telephone 100' receives refresh signal directly from electronics 833 of mobile telephone with bridging connection 790, control unit 736 and USB connectors 691 and 693 are not used.

**Fig. 11** present examples of data imaging produced by monitor 349 heart signal on display 875 of monitor 349 heart signals and/or on display of mobile telephone 100' and/or auxiliary 'device 104 with mobile telephone and/or watch 416 and/or control unit 753and/or further units. Imaging element 1 of holter preferably made up of same mentioned display 878, 178, 506, 754, 865, 887 at numeric displaying preferably displays value of arrhythmia, value 862 of breath, value of pulse 861, reason of alarm 864 if alarm situation emerges. Displaying element 1 of holter is preferably universal and displays course of curves, for instance curves 873 ECG with touch display, for instance control 866 of sensitivity, control 867 of speed of movement of curves on display, further control 868 of moving filter preferable for calm, walking, run, and control 869 of multi-electrode adjustable displaying, where touch buttons are on control display with curves 873 ECG, which adapts in order to display prompt results of adjusting. It preferably shows the curve 874 of pulse, curve 878 of arrhythmia and curve 875 of variability, where it is possible to change imaging by means of touch display 872 just on display so that is possible to watch changes during setting and so set the appropriate value. These quantities and/or curves are preferably displayed separately or by groups at one imaging element 1 holter. Value of arrhythmia is preferably expressed in % of irregular pulses of total number of pulses in time period, or in degrees preferably in values from 1 to 10 taken as 1/10 from above mentioned % irregular pulses or in other adequate scale such as none, low, middle high and very high, As irregular pulses are pulses which exceed value 1.15 average pulse measured from settable number of pulses, preferably 3 pulses /min for filtration of disturbance. The curve 874 preferably contains vertical abscissas 139 for each pulse for highlighting of possible irregular pulse. Further contains preferably curves of limits of regular pulse 937 far from average curve of pulse 874 by settable number of pulses or percents of pulses for to be clear, when irregular pulses exceed limits of pulse frequency selected from settable numbers of pulses. Heart pulses which exceed curves of limits are considered as irregular pulses for calculation of degree or % of arrhythmia as selectable interval as mentioned above.

**Fig 12** shows an example of wire connecting of external electrodes 797' preferably made up of electrodes 143', 143" glued, connected by means of cables 267 by help of connector 786 , electrodes and monitor 349 to 349‴ placed preferably in control module357, 357" made up of mobile telephone 100' or system 922 or control unit 753. Cable of one cord 267 led from glued electrode 143' is thin and in body color or transparent so that it should not be bothering for monitored person or attract undesirable attention. on arm is added on arm to one cord cable 267' from electrode 143", so since area of shoulder of the cable 267a contains two wires 267' and is led through sleeve below the shirt 912 to connector 786 ECG where are brought heart signals.

**Fig. 13** is drawing monitor 349-349‴ preferably placed in control module 357 fastened preferably on band 263 by hanging or in pocket 265 or on wrist as bracelet 266 or arm as armlet 266'. Electrodes 143', 143", 143"a, 143'b glued on various parts of body are connected by various cables 267'a to wrist band 266', monitor 349. In this case are the electrodes 143'preferably glued on skin or taken of chest strap 749 or preferably are glued electrodes 143", drawn on Fig.14 combined with electrodes 143 on chest strap 147 preferably viable with more electrodes accordingly need, up to 12 leads ECG. On Fig. 13 is preferably cable 267 of one core, taken from lower electrode 143' to upper electrode 143 ", where converts into cable 267a of two cores for easy installation and esthetic arrangement whereas cables 267,267a are preferably as thin as possible, with transparent isolation, eventually of body color, for to attract minimum attention and were not the psychological scruple against the wearing. It helps to this fact that the cable 267 is led in sleeve 268, where is not seen, preferably on right hand because for the first curve of ECG is upper electrode 143" placed near right shoulder.

Alternatively electrode 143" is to be placed, drawn as electrode 143"a upper, as high as possible, accordingly availability of heart signals, in ideal case up on shoulder or slightly behind and electrode 143'drawn as electrode 143'a as low as possible and sideways, ideally up to level of band 263, drawn as electrode 143b, therefore when shirt 270 is open they are not so visible as in case of their placement on chest. In this case is cable 267'preferably stretched along the band 263 where is preferably fastened by clasp 271a up on side or back to electrode 143" and from that place by cable 267a of double core to monitor 369 on wrist band 266 for worse look in case shirt 270 is open. Electrode 143'a is preferably alternatively, instead of glueing , placed on clasp 272 hung on belt 263 of trousers or skirt or directly on them, by which is pressed on body for better contact or alternatively is electrode 143'a fastened on body, on band 273, through which cable 267"a is stretched , preferably clasp 272 wears monitor 349 alternatively placed near belt, from which cable 267b with electrodes 143'and monitor 349 is interconnected thus preferably cable 267a of double core falls off because cable 267b is, in this case, preferably brought directly to monitor 349 how is drawn also on Fig.17. Analogously more electrodes 143'a are alternatively preferably connected on clasps 272,272'or band 273 accordingly the need.

**Detail 1** is drawing alternative connection of electrodes 143' glued over cable 267 and cable 267a of two cores to monitor 349-349‴ placed preferably in control module 357 in pocket.

**Fig.14** is drawing further example how is on wrist 645 placed monitor 349-349‴ of heart signals preferably placed in control module 357 serving beside the other things as control of chest strap 749-749‴and projection of pieces of information about heart signals and preferably processed in ECG and further values and curves whereas heart signals are sensing wireless or by cable 233 from chest strap 749-749". Further there is drawn the location of electrodes 143 on chest strap 749-749‴ and electrodes 143".

**Fig.15** is showing fastening of clasp 272' on belt 263 and interconnection of electrodes 143'a, 143'.

**Fig.16** is drawing clasp 272" fastened on belt 273 on body in such a way that it is making possible to put on trousers 274b or skirt preferably with belt 263 under it.

**Fig.17** is drawing alternative attaching of electrodes 143a on clasp 272 and interconnection by double core cable 267c to monitor 349-349‴ on wrist band or alternatively on clasp 272 where cable 267b preferably is not necessary.

**Fig.18** is drawing electrode 143'interconnected by cable 267'a and further by body band 273 on belt sideways to electrode 143" a and further in sleeve 268 to monitor 349-349‴, therefore there are not any cables or bands on chest.

**Fig.19** is drawing placing of electrodes 143', 143'a on body 208 without glueing.

**Detail A** is drawing electrode 143'fastened on flexible band 293 preferably of steel, which is pressed by band 273 on body, therefore is pressed also electrode 143'which is connected by cable 267', of one core, which will change itself on cable 267a" of two cores, from electrode 143"a, and is on shoulder pressed by flexible material 286 preferably formed by cord or rubber band, taken from band 273 over shoulder. Electrode 143 "a is preferably fastened to it and to backside on back side of band 273. Cable 267" is preferably taken by band 273 with band of flexible material 286 to electrode 143'and then, as cable 267a in sleeve 268 to monitor 349 on wrist of analogously hung on belt.

**Fig.20** is drawing general time course of value of pulsation frequency HR in dependence on time t in coherence with intensity of physical activity in marked time zones where zone 367 expresses course of actual value of pulsation frequency 366 before starting of training, zone 368 delimited by time interval t1 of progressive warming of muscles in which, due to increase of muscles activity is increased also pulsation frequency from actual value of pulsation frequency 366 on target value of pulsation frequency 365 recommended for training, usually 70 % of individual value of maximum pulsation frequency 364 which is possible to set on maximum performance, zone 370 delimited by time interval t2 delimiting time of intensive training and the last one is the zone 371 in which time interval t3 is decreased by value of pulsation frequency during cooling of muscles due to the decrease of load. Further in graph is drawn value of pulsation frequency 364 of individual maximum of pulsation frequency for person in training.

**Fig. 21** describes new method of monitoring and evaluation of course of pulsation frequency consisting in monitoring of observance of preset limits of pulsation frequency in whole range of training, i.e. in zones 368, 370 and 371. Advantage of this way consists in conducting of sportsmen in all phases of training i.e. since the gradual warming up of muscles over intensive training until cooling of muscles. New is the conduct of training especially in phase of warming up of muscles of sportsman in zone 368 of warming pulsation frequency 381 before larger load in area of target pulsation frequency 382 which is important for prevention of muscles injury and simultaneously for preparation of consequent reach of maximum performance during the time of intensive training. Value of pulsation frequency is measured and evaluated by electronic device preferably with use of monitor of pulsation frequency, usually with using of chest straps and watches for sport or mobile phones. Deviation of values of frequency of heart beats out of set limit is initiating warning, usually acoustic signal. Indicated way is suitable also for monitoring of heart beats frequency of cardiac during the physical activities recommended by doctor which are one of the best preventive provisions against illness of heart and vessels. Primarily it concerns the dynamic sports as run, swimming, biking when monitoring of pulsation frequency takes place simultaneously. Also in case of these activities is the course of pulsation frequency similar as during the training of sportsman although with substantively lower load during the warming of muscles, main part of exercising and also in the end, when is included breathing out and relaxation that follows. Electronic device, which is used for evaluation of pulsation frequency by this new method, continuously is sensing pulsation frequency HR of sportsman usually with using of correct chest straps or electrodes fixed by another way. It works with all parameters or some parameters drawn on Fig.2 the preset values of them can be stored in device and for individual trainings modified and/or newly inserted. It concerns the following parameters:
t1 time interval of gradual warming up of muscles delimiting zone 368
t2 time interval of intensive training delimiting zone 370
t3 time interval of cooling of muscles delimiting zone 371
363 initial value of pulsation frequency at start (optionally put in value or use actually value measured out
365 target value of pulsation frequency recommended for training
372 upper limit of value of pulsation frequency for zone 370 of gradual warming up of muscles
373 upper limit of value of pulsation frequency for zone 370 of intensive training
374 upper limit of value of pulsation frequency for zone of cooling of muscles
375 bottom limit of value pulsation frequency for zone 368 of gradual warming up of muscles
375 bottom limit of value of pulsation frequency for zone 370 of intensive training
376 bottom limit of value of pulsation frequency for zone 371 of cooling of muscles

Above mentioned parameters is possible to insert individually or in identical groups for several limits. Preferably the simplified insertion will be enough, when after insertion of values of time intervals t1. t2, and t3 and target values of pulsation frequency 365 recommended for training is inserted tolerance band of pulsation frequency expressed by number of pulses, par example as +/- pulses per minute or as deviation +/- % of actual value. At the moment of starting of training by pressing button 396 par examples marked as Start the continuous evaluation goes off, indicating whether immediate measured values of pulsation frequency ate inside tolerance band delimited by selected limits. Further is indicated regime of automatic monitoring and evaluation of pulsation frequency, however further eventualities are possible, too. Electronic device is sensing continuously actual values of pulsation frequency 366 , curve of calmness pulsation frequency 400 is not too much changed in calm status and it is therefore drawn by dashed line until the point of start 136 . By pressing button 396 at start as initial value 363 of pulsation frequency will be automatically used actually measured value and all other is going just automatically without intervention of sportsman. Simultaneously is monitored pulsation frequency and its deviation is signalized, of tolerance band including calming pulsation frequency 383 till the end of training. In case of use of dynamic shift of display, Fig. 27, graph on monitor goes to left. Sportsman starts training in zone 368 by activities leading to the warming up of muscles and increase of heart activity showing the growth of pulsation frequency namely until acoustically signalized teaching of target value of pulsation frequency 365 recommended for training. Further training goes on, at the moment of reach of point 380 of end of training is given acoustic signal and sportsman decreases step by step load until reach of calm value of pulsation frequency. Variantly it is possible to adjust the indicated function in such a way that at the moment of pressing of button 396 is used selected value of initial pulsation frequency.

**Fig. 22** is drawing dynamic shift of projected graph to left in direction S on display 178 of electronic device with optional change of setting of time distinguishing of projection. In upper part of figure is drawn situation before start of training with indication of its start by pressing of button 396.

**Fig. 23** is drawing regime of manually started monitoring and consequent automatic evaluation of pulsation frequency during training. Up to the reaching of point 379 signaling possibility of starting of training everything goes accordingly description in precedent figure. However starting of monitoring and evaluation of training in zone 370 comes when sportsman presses button 396 again after passing of any time at the moment indicated by point 397 since which is started measuring of time of training (t2). Ending of training and therefore also measuring of time sportsman is signaling again by pressing of button 396, indicated as point 380 of ending of training since which everything goes identically with precedent example.

**Fig. 24** is drawing regime of measuring of time t1 necessary for to increase of pulsation frequency on level of target value of pulsation frequency 365. In such a case sportsman is not conducted in zone 368, only measured and recorded is time t1 during which the actual pulsation frequency of sportsman reached target value 365 for training and preferably acoustic indication is giving information to sportsman that he has not yet reached target pulsation frequency and therefore operation temperature of muscles also not. When acoustic signal stops, sportsman receives information that he can start performance part of training then preferably by activation of button 396 with functions start and stop measures the active time t2 of training. After finishing of performance phase of training sportsman will stop measuring and recording of time by repeatedly use of button 396.

**Fig. 25** is drawing of example of projection of values of heart pulses frequency during training on display 178 of electronic device, which is mobile phone in this case. Projection in real time is possible, during training and later projection is possible as well, with use of data stored into memory of electronic device during training preferably with possibility of setting of time scale of projection. Floating graph 384 of pulsation frequency is shifted to left in direction of arrow S, from right side of display 178 where begins, on left edge_of display 178 where di disappears. It displays actual value of time. On monitor is placed in upper part of picture and is drawing adjustable time interval preferably of last 30 seconds of course of pulsation frequency in real time. Position of each segment is drawing time moment of origin of corresponding individual heart pulse and height its pulsation frequency. Besides is continuously monitored the eventual occurrence of arrhythmia at detection of arrhythmic pulse which is marked preferably by different thickness and color corresponding to segment 395. Value of arrhythmia is depicted further graphic by column 388 in window 389 and verbal indication of degree of arrhythmia is indicated in line 390 in following degrees: None (arrhythmia undetected), column 388 is not marked, further degrees Low and Trivial marked by green column 388, degree Medium marked by yellow column and degrees High and Very High marked by red color. Further there is in left part of Fig.5 numerically drawn actual value of pulsation frequency 391, for example 82 (pulses per second) and continuously are during training numerically indicated time data on display 392 of time of zone 368, further on display 398 of time of zone 370 and on display 399 of time of zone 371. In example on displays drawn values are expressing that sportsman overcame zone 368 in which to zone was set time equal to 2 minutes and in time of drawing sportsman is training 4 minutes and 37 seconds in zone 370. Evaluation of training is on Fig.30 and following figures is preferably made with large round button 393 which is generally marked on figures by mark 137 and notice of which depicts what will happen after its pressing in dependence on ongoing application preferably Start, Pause, Continue, Stop etc. Fixed graph 385 of pulsation frequency placed in bottom part of figure depicts course of pulsation frequency during the complete training with indication of time intervals which are indicated as zones 368, 370 and 371. Zone 368 is gradually warming on start of training. After starting of training value of pulsation frequency is growing until it will reach optimum setting for training. Zone 370 is load part; zone 371 is area of cooling of muscles. Before start user has chance to choice regime of monitoring of training. Regime of automatic monitoring is using preset parameters without their correction whereas as value 363 of pulsation frequency is inserted at respective moment the actually measured value. Regime of automatic monitoring can be modified in such a way that sportsman before commencement of training will use possibility of change of all or only selected preset parameters before start of training. By pressing of button 393 with function Start (after pressing the notice and thus also function is changed on Pause) on panel of electronic device which is in graph 385 indicated of mark 137 starts training and short beep sounds. Floating graph 384 will be continuously shifted which will enable to project value of pulsation frequency for last 30 seconds with good definition. Fixed graph 385 of pulsation frequency placed in bottom part of figure will draw value of pulsation frequency recorded during the whole training. After beginning of training pulsation frequency will grow up to reaching of end of zone 368. At this moment actual pulsation frequency of user will reach the optimum value of target pulsation frequency and training will go to zone 370 and this state will be signaled by short beep after termination of load part of training and the end of zone 370 user transits into zone 771. Transit is again acoustically marked by short beep In this zone user will slow down of run and actual value of his pulsation frequency will lower until reach of value of original pulsation frequency before start and thus also end of zone 371.

**Fig. 26** is drawing example of graphic depiction of couse of pulsation frequency after finishing of training in case of using of regime of automatic monitoring of training in case that during training came to deviation of values of frequency of heart pulsation out of set limits by exceeding of upper limit 373 of value of pulsation frequency for zone 370 and bottom limit 375 of value of pulsation frequency for zone 370 in points 394 the occurrence of which initiates usually acoustic signal.

**Fig. 27** is drawing the use of dynamic shift of projected graph on display 178 for increase of time difference of projection of graph 385" of course of values of pulsation frequency in all phases of training. Graph 385" of course of values of pulsation frequency is floating; observation of covered areas of graph comes due to its shifting by user.

**Fig. 28** is drawing regime of simple graphic projection of course of pulsation frequency during the whole training. By pressing button 393 with function Start on panel of electronic device which is in graph 385 marked by mark 137 is started projection and record of course of pulsation frequency by fixed graph 385 of pulsation frequency placed in bottom part of picture. Measuring and display is possible whenever stop by pressing button 393 with function Pause and possibly continue by further pressing of button 393 in this case with function Continue or process to finish by further pressing of button 393 with function Stop.

**Fig. 29** is drawing example of curves defining heart activity of monitored person projected by display 1 of holter, which is preferably part of control module 357 or displaying module 358 or evaluation block 359. Example illustrates simultaneous projection of curve of pulsation 6 and curve of frequency of occurrence of arrhythmic and regular pulses 12 expressing number of occurrence of arrhythmic pulses of monitored person. Curve of upper limit of deviation from average pulse 9 and curve of bottom limit of deviation from average pulse 10 are the limiting curves of regular pulse for determination of arrhythmia. Upper bar 2 projected on display 1 is drawing two rows of text messages, lower bar 4 is drawing time axis 14. In upper part of display there is placed curve of pulse 6 created as connecting bar of points indicating values of pulses one by one going, drawn on tops of segment lines of value of pulse 7 the position of which on time axis marks moments in which the value of pulse was set and their height indicates value of pulse. Degree of arrhythmia expressing frequency of occurrence of arrhythmic pulse sis in indicated example stipulated with use of curve of average pulse 25 indicated on Fig.32 which is calculated as average of values of optional number of pulses of precedent pulses, preferably of three. From this curve is derived curve of upper limit of deviation from average pulse 9 corresponding preferably with values of curve of average pulse 25 decreased for optional number of pulses preferably 10 pulses. If actual value of pulse expressed by curve of pulse 6 deviates out of limits set by curve of upper limit of deviation from average pulse 9 and curve of lower limit of deviation from average pulse 10, occurrence of arrhythmia is detected. Curve of frequency of occurrence of arrhythmic and regular pulses 12 has preferably 10 degrees (0-10 pulses) whereas degree No. 10 is the highest. Curve of frequency of occurrence of arrhythmic and regular pulses 12 is connection of degrees of arrhythmia 52 which is not displayed. Initial degree of arrhythmia 52 is 0. At detection of occurrence of arrhythmic pulse curve of frequency of occurrence of arrhythmic and regular pulses 12 preferably is increased with every arrhythmic pulse for one degree. If it is not detected in actual pulse occurrence of arrhythmia curve of frequency of arrhythmic and regular degrees 12 is decreased for one degree or alternatively on 0. Time course of arrhythmia depicts curve of frequency of occurrence of arrhythmic and regular pulses 12 placed in lower part of curves 3. Scale of arrhythmia 13 has division on parts which correspond to percent's namely preferably 10 segments one segment corresponds to value of arrhythmia 10 %. Value of arrhythmia drawn on axe Y, by curve of frequency of occurrence of regular pulses 12 is continuously actualized. Time scale projected on axe X for curve of frequency of occurrence of arrhythmic and regular pulses 12 is common with curve of pulses 6. Value of arrhythmia related to selected time period can by expressed in several ways. Numeric value of arrhythmia 20 for time of 30 seconds is projected in upper part of display where figure preferably 4.2 degrees of arrhythmia taken as 10 % from arrhythmic pulses therefore means occurrence of arrhythmia 42 % which is stipulated of percentage relation of number of pulses with detected arrhythmia of total number of pulses in frame of evaluated time segment. Values of pulsation and time expressed by curve of pulse 6 are deducted on scale of pulsation frequency 8 and on time axe 14 expressed as number of pulses per minute which is placed on bottom bar 4 with marked figure of real time 15 in this example is indicated time preferably in ten minutes intervals par example 09:41:20, 09:41:30 etc. Actual value of pulses is calculated of time distance of lime segments of value of pulse 7 generated in monitor of heart signals 349‴ general, drawing actual pulse in precedent time interval. Time is given by numbers of time interval 11 preferably with distance 10 seconds which are marking time marks 24. For increase of distinguishing are the segments of value of pulse 7 and time marks 24 distinguished by colors. Storing of whole or parts of course of pulse, arrhythmia or ECG in memory of device is possible whenever by using of button preferably touch ones and/or automatically in case of exceeding of preset limit and/or automatically in set time intervals. Setting including choice of actually projected curves and also data way of their projection operates preferably group of touch buttons 5 formed on display number of which, marking and functions are subject of change in dependence on actual setting. Further buttons can have different functions. Volume of acoustic alarm par example for night and day is changed by button D drawn on Fig. 29. Brightness of display 1 is possible to change by button M. Curve of pulse 6 is vertically shifted by buttons ∧ and ∨ button 63 controls the remote change of setting of group of filters in electronic of attached detection device. Stop or restart of continuous projection of curves is controlled by button I>/II. Increase/decrease of horizontal scale of curve of pulse 6 control buttons H+/H-.

**Fig. 30** is drawing easy deduction of coordinates of actual pulse. Initial part of pulse 6 on right side is drawing actual value of pulse in actual time and its placing some millimeters from right edge of display is making the deduction of its coordinates easier. For evaluation of each of following pulses the whole curve of pulse 6 is shifted for one pulse to left and on free place is displayed its value. Simultaneously is shifted and completed also the curve of frequency of occurrence of arrhythmic and regular pulses 12 as described on Fig. 29.

**Fig. 31** is drawing simplified orientation expressing of arrhythmia verbally or by column graph. For orientation expressing is possible to use verbal description of arrhythmia 22 and its value divide in several groups preferably on None, Low, Trivial, Medium, High and Very High and analogously is possible to use also several different heights of orientation column graph of arrhythmia 21.

**Fig. 32** is drawing curve of average of pulse calculated of chosen number of precedent pulses 25 which has not to be displayed and was calculated of curve of pulse 6 how described on Fig. 29. Curve of upper limit of deviation from average pulse 9 and curve of bottom limit of deviation from average pulse 10 are the limiting curves of regular pulses for determination of arrhythmia.

**Fig. 33** is drawing example of offer of parameters of setting of filters of external disturbance and setting of limit value of arrhythmia. Button N how is described on Fig. 29 is example of using for evocation of offer or gradually of group of offers for insertion of parameters of projection and evaluation of curves. Filters 0, 1 and 2 of external disturbance are decreasing external influence on evaluation of pulses. Filters are possible during operation to switch over by button marked F0 on Fig. 29, the marking of which is changed on F1 or F2 in dependence on chosen filter. Degree of filtration is given by parameter marked " Upper Limit" of pulses per minute, the exceeding of which to be taken as failure is caused by external disturbance. Occurrence of alarm status and thus also giving of signal by exceeding of set limit of arrhythmia are determining parameters " Arrhythmia above " expressing limiting value of arrhythmia expressed in % and "for time " for which was the arrhythmia value calculated. Also it is possible to set delay between limits crossing and giving of sound signal of alarm.

**Fig. 34** is drawing example of further offer namely for setting of parameters of detection of arrhythmia and setting of limits of alarm the exceeding of which is activating the alarm status. Detection of arrhythmia is using parameters of calculation of values of average pulse and from them derived curves - curve of upper limit of deviation from average pulse 9 and curve of bottom limit of deviation from average pulsation 10 as drawn on Fig. 29 which set items " Average pulsation per X pulses" par example 3 pulses and "Upper and bottom limit" expressing deviations of curve of upper limit of deviation from average pulsation 9 and curve of bottom limit of deviation from average pulsation 10, how drawn on Fig. 29, from calculated value of pulses expressed by number of pulses par example +/- 10 or percent's. Parameter " Ignoring from multiple of average" is limiting activation of alarm status in case of evaluation of abnormal deviation of pulsation caused by failure of signals. Remaining items of offer are set for setting of further limits of alarm. Limits of alarm of pulsation are setting of parameters " Minimum permitted pulsation", per example drawn by line of bottom limit of pulsation for Alarm 30 how is depicted on Fig. 29 indicating value of pulsation under which the decrease pulsation for more than ,,Alarm at N pulses above/under limit in a row" per example N equal to 3 successively going pulses evacuees status of alarm. Analogously item "Maximum permitted pulsation" per example 80 is setting position of line of upper limit of pulsation for alarm 29 how drawn on Fig. 29, upper limit for alarm indicating value of pulsation the exceeding of which by pulse for N (par example N is equal to 3) or more pulses going in a row, inserted as the second item in marked "Alarm at N of pulses above/under limit in a row" evokes status of alarm.

**Fig. 35** is drawing monitoring of degree of arrhythmia 52 as it is depicted on Fig.29, for individual pulses in optional time limit in respective case 0.5 hour. Degrees of arrhythmia 52 the number of which is indicated in description Fig. 29 are drawn in area of projection of curves 3 and it is possible to switch over them by button S of display of pulsation on Fig. 29. "Projection of values of arrhythmia is using the whole area of drawing of curves 3. Function of buttons N, S and F0, how depicted on Fig. 29, has not been changed by switching over. Simultaneously with switching over of projection has been changed marking of function as well in case of other touch buttons in comparison with precedent example. By pressing of button of limit 53 is possible to set time limit of degrees of arrhythmia 52. Under degrees of arrhythmia 52 is in every of 3 depicted segments marked time at the begun and end of segment as well. For example the second segment begins at 09:30:00 and ends at 09:59:59. In space 56 on right from depiction of degrees of arrhythmia 52 is repeated time of beginning of segment 09:30 including length of segment 0,5 hour (30 minutes) behind slash 09:30/30. In the new row is indicated occurrence of arrhythmia during the whole segment 57 in indicated example 0,38 %. In upper left corner is figured cursor 54 as thicker line segment, the position of which is on curve up to place of interest shifted by buttons ^ v < > . By pressing of button D the cursor 54 is shifted on curve to place of actual time, button Z will increase the time detail in place of position of cursor.

**Fig. 36** is drawing projection of degrees of arrhythmia 52a which is formed by numeric values of arrhythmia 20 which are not connected by curve and in case of choice of longer time segment par example 8 hours are strained into structure which has no gaps between individual number values of arrhythmia 20 and therefore forms glimmering of curve.

**Fig. 37** is drawing example of curve ECG 16 in time limit of 5 seconds. On background of curve ECG 16 is depicted grid of millimeter enabling to deduct values of amplitudes of impulses and also time figures of course. Scales of projection of curve ECG 16 in horizontal and also vertical direction is possible to change by buttons from the group of touch buttons 5 created on display and set also for all further indicated operations. Actual scales, par example 25 mm/s on horizontal axe and 20 mm/mV on vertical axe are indicated on bottom part of ECG graph. Besides it there is at begin of curve ECG 16, depicted calibration impulse 18, the height of which is drawing normalized voltage 20 mm/mV usable in a standard way for evaluation of amplitudes of impulses of curve ECG 16. Above every complex QRS 52 is mark of pulse 50 preferably formed by symbol of star indicating that in monitor of heart signal 349‴ general to every complex QRS 62 handed over pulse depicted in curve of pulse. Touch button of group of touch buttons 5 is enabling switch over of remote movement filters on monitor of heart signals. Moving filters are preferably set in regimes calm, walking, run. Filters for sensing of ECG and pulsation curves in moving are deforming the curves, therefore is difficult to take out of them par example wave "P". For recording of undeformed curve in calm holter 351 is producing warning signals acoustically, by vibrator or display in adjustable periods in which the monitored person brings himself/herself in calmness and by marker button effects record and indicates time which is displayed on display in case of curves preferably of pulses or ECG, by mark. Monitored person can use holter also without warning signal in order to mark time in certain situations or activities par example when is recorded, that person is not feeling well or by higher effort. Simultaneously by button is preferably initiated voice record preferably on control module 347. Record is replayed when required preferably at projection by marker 64. In regime pause preferably is possible to "travel" through record, par example ECG or pulsation only on markers 64 by pressing of button "project marker" preferably always for one marker 64 further. For saving capacity of accumulator is preferably prepared record only by pressing of marker 64.

**Fig. 38** is drawing projection of curve ECG 16 in time limit of 15 seconds. In case of larger time range 15 seconds it is possible to monitor well the marks of pulse 50 and better evaluate that detection of QRS complex 62 was realized correctly and thus will be correctly segment of value of pulse 7 in curve of pulse 6 contained, how depicted on Fig. 29.

**Fig. 39** is drawing example of projection of curve ECG 16 in time range of 15 seconds with missing impulse 58 which shows that detection of complex QRS 62 how is depicted on Fig. 37 was not realized correctly and thus will be not correctly contained value of segment of value of pulse 7_how depicted on Fig. 37 on curve of pulse 6, how depicted on Fig 29, will be gap. In case that complex QRS 62 how depicted on Fig. 37 will be detected extra which we will recognize on marks going one by one, it will be not correctly contained value of segment of value of pulse 7 and on curve of pulsation 6 will be recorded one pulse more how is depicted on Fig. 29.

**Fig. 40** is drawing example of simultaneous projection of curve ECG 16 and curve of pulse 6 whereas time scale of projection of both curves is different. Time scale used for imaging of curve of pulse 6 is considerably bigger which enables to monitor course of curve of pulse 6 in larger time interval and is identical with example on Fig.29. Width of rectangle forming cursor ECG 12 indicates on curve of pulse 6 time segment which does not correspond to length of segment depicted by curve 16. In regime PAUSE when the projection in real time does not take place, it is possible by moving of cursor on recorded curve of pulse 6 to browse in records and therefore to project the corresponding segments of stored curve ECG 16.

**Fig. 41** is drawing example of simultaneous projection of curve ECG 16 and curve of pulse 6 whereas time scale is the same. Correctly detected complex QRS 62 how is drawn on Fig. 37 is depicted with symbol of star. Control buttons and further functions preferably of evaluation of alarm status can be completed by means resulting of Fig. 29.

**Fig. 42** is drawing monitor 349 - 349‴ of heart signals in control module 357 for their sensing, processing and projection of signals of heart activity and examples of its placement on various places whereas it is possible to move them preferably on these places. One of these examples is placing of control module 357 on wrist of hand 938 by wrist band 371. On inside of wrist band 371 is placed contact 940 which is preferably part of wrist band 371 and which has on outside connecting point 978 for connection with the second contact 941 on control module 357. Control module 357 is to wrist band 371 fastened by means of hitches 937. Control module 357 has on outside surface two contacts from which one contact 941 is reserved for connection to contact 940 which is part of wrist band 371 and second contact 936 is destined for sensing of signals of heart activity. Control module 357 is also provided with door 943 for replacement of replaceable accumulator preferably during operation and connector 945 for wire connections to electrodes through which are brought heart signals to processing. As further way of bringing of heart signals is indicated wireless connection 939 which is bringing signals preferably from chest strap. The further way of bringing of heart signals by means of contacts 936 on arm with which is electrically interconnected by means of contact 940 and contact 936 by finger of second hand. Other example of bringing of heart signals is depicted wireless sensor 942 working preferably on principle of screening of finger and sensing of light signals which are transmitted into control module 357 preferably by means of wireless connection 939.

**On Detail 1** is drawn example of using of monitor 349-349‴ in control module 357 placed loosely in space, at which the heart signal is sensed by putting of finger on one of contacts 936 and by putting of finger of the second hand on second contact 941. Control module 357 is preferably provided with touch display 363 for projection and control of functions.

**On Detail 2** is drawn fastening of control module 357 on chest strap 749 which is provided with hitches 937 which preferably form simultaneously connecting contacts extending on contacts of control module 357 preferably on principle of detachable snap fasteners which are connected by wire connection 492 to sensing electrodes of chest strap 749. Heart signal is sensed from electrodes of chest strap or can be connected also external electrodes 957 by connector 945 for sensing of 1-12 leads of ECG.

**On Detail 3** is drawn example of using of monitor 349-349‴ in control module 357 placed in casing 945 on belt to which are connected external electrodes 143'preferably placed under shirt 969 by means of connector 945 on control module 357.

**On Detail 4** is drawn example of fastening of monitor 349-349‴in control module 357, control module 357 fastened on belt 946 of trousers by clasp 947. To control module can be connected external electrodes 957 by connector 945 or another sensors of heart pulsation by means of wireless connection 939.

**On Detail 5** is drawn example of using of monitor 349-349‴in control module 357, hanging of control module on core on chest.

**On Detail 6** is drawn example of using of monitor 349-349‴in control module 357 placed in control module 357 fastened on shortened removable band 971 by means of hitches 937 and interconnected by wire connections 492 with electrodes of chest strap 940 preferably directly by hitches 937 in execution of detachable snap fasteners. To control module are preferably connected external electrodes 957 by connector 945.

Fig. 43 is drawing example of using of monitor 349-349‴in control module 357 with tilting arms of electrodes 973 in position of tilted arms with tilting braces 972. Also the view from below in direction D is drawn.

On Detail 1 is drawn example of using of monitor 349-349‴in control module 357 with tilting arms of electrodes 973 in position of tilted off arms on which are placed the electrodes themselves 950. Tilting off arms of electrodes 973 are stored in hinges of arms of electrodes 951 and in dismantled state are fixed by tilting braces 972 with hinges 974. Tilted braces 972 are in dismantled state fixed into cuts 948 on back side of tilting arms 973. For sensing of heart signals the control module is to be pressed on chest in area of heart. Other possibility is to press one electrode on finger of one hand and second electrode on finger of the second hand.

**Fig. 44** is drawing example of using of monitor 349-349‴ placed in holter 954 clicking in direction of D which is preferably placed as removable, on chest strap 749 or wrist band 371. Hitches of holter 952 fit in hollowing 953 on control module 357 which is fixed in holter clicking from below 954.

**On** **Fig. 45** is drawn holter with skids 956 for insertion of control module 357 from side. Holter with skids is placed on chest strap 749 or wrist band 371.

**Fig. 46** is drawing example of using of monitor 349-349‴in control module 357 and control module itself 357 preferably provided with touch display on which are individual functions operated by mechanic buttons 372 or buttons 965 on touch display 370 preferably in combination with mechanic buttons 372.

**Fig. 47** is drawing basic placing of control module 357 in storing mechanism 979 on wrist of hand with fastening by band 371.

**Detail 1** is drawing basic position of control module 357 in storing mechanism 979 which is fixed by press spring 980 in position of insertion of connector 981. Control module 357 is possible to use also for further functions preferably also for telephone communication. For such a purpose there is on further figures depiction of removal of control module 357 out of storing mechanism 979 for easier manipulation.

**Detail 2** is drawing shifting of control module 357 in direction of arrow D against force of spring 980 until moment of shifting off out of connector 981.

**Detail 3** is drawing tilting off and removing of control module 357 out of holter 962 of storing mechanism 979. For easier removal of control module 357 out of holter is preferably bend of holter 987 made of flexible material.

**On** **Fig. 48** is drawn example of using of monitor 349-349‴in control module 357 in storing mechanism 979 of control module which is preferably equipped by holter 977 with spring and which is preferably placed on wrist band 371. Control module is in this execution preferably inserted against spring on figure right and after is inserted in connector 945 as described in detail in Fig. 47 , by which is reached interconnection with electrodes 143'. At insertion control module 357 is inserted in connector 945 and through wire connection 492 is connected on electrodes 143'external, for sensing of heart signals.

**On** **Fig. 49** is drawn execution when monitor 349-349‴in control module 357 is enlarged for removable accumulator 961 which is stored in holter preferably provided with spring analogously as on figures 47 and 48 whereas this set is adapted for insertion of mobile phone 100 which is after insertion interconnected with control module 357 by connector of mobile phone 981, "female" inserted in "male" in control module 357 which is in holter 977 fixed steadily. Control module is processing signals from electrodes 957 which are preferably projected on mobile phone 100. With preference projection remains active on mobile phone also at its removal out of holter by wire connection 944 with nearby units which is activated preferably automatically. By this connection it is possible as alternative to signals from electrodes 957 to receive signal of chest strap 749' and in case using of maxi control module 357" to use preferably mini chest strap 744'" which does not contain any of monitors 349-349'". In case of insertion of mobile phone 100 in holter are the heart signals transmitted to it by connector 945 of electrodes 143'. Preferably is control module 357 adapted for operation in regime of mobile phone for which is preferably removed out of holter 977 for closing to ear.

**Detail 1** is drawing monitor 349-349‴ in control module 357 with replaceable accumulator 975 preferably during operation where front end 362 and front end 362' of sensors, digitalization unit 366 and communication unit 275'is placed in firm part 986 which remains in holter 977 and secures primary processing and their digital transmission by radio connection 153 to control module 357 in time when is shifted out of holter 977. In time of insertion in holter digital form of heart signals is passed by connector 945.

**Detail 2** is drawing device described in Det.1, where is completed replaceable basic accumulator 129 by which is charged bridging accumulator 120 if control module 357 is inserted in holter 977. If is basic accumulator 129 removed, its operation is taken over by bridging accumulator 120. Radio connection 153 secures alternative connection with chest strap 749 for transmission of heart signals from it instead of electrodes 143'.

**On** **Fig. 50** is drawn execution when monitor 349-349‴in control module 357 and mobile phone 100 is also with storing mechanism 979 on wrist band 371 stored slidingly on skids 965 therefore is enabling insertion par example out of sleeve and then comfortable monitoring of actual projection. Connection to electrodes is by twisted core 964 which will enable comfortable change of position of whole set. Mobile phone 100 is possible to remove out of holter as on Fig. 47.

**On Detail 1** is drawn control module in slightly shifted off position suitable for observation of displayed date on display.

**On Detail 2** is drawn slight shifting off out of mobile phone 100 up to such extending that it is disconnected from connector 967. This arrangement has the advantage that the twisted core is not necessary because control module 357 is not shifted.

**On Detail 3** is drawn complete separation of control module 357 which is not stored in storing mechanism 979, from wrist band 371. To this comes after overcoming of arresting position when protrusion 983 does not fit in arresting groove 982.

## Claims

1. Live Holter
is comprising a holter (351) with a control module (357') and/or with imaging module (358) and optionally an evaluating block (359) wherein the holter (351) comprises a chest belt (749"), a front end unit (362), a monitor of heart signals (349"), a communication unit (275'), and the live holter is adapted for sensing analog heart signals from the chest belt (749") and processing the heart signals in the front end unit (362) into data sent to the monitor of heart signals (349 ") and from the monitor of heart signals (349") to the communication unit (275') for transmission for Holter evaluation in at least one of: the control module (357'), the imaging module (358), the evaluating block (359) and for transmission for displaying live or from record on at least one of: the control module (357'), the imaging module (358), the evaluating block (359), and the evaluating block (359) is comprising server (806) and further comprising operator's PC (839) and/or participant's PC (839'),
wherein the monitor (349") of heart signals comprises a memory for data recording of heart signals, advantageously formed by a SD card (888), and the communication unit (275') communicates locally with the control module (357'), for displaying on a display (363) of control module and/or the communication unit (275') communicates locally with the imaging module (358) and the local displaying is for patient and/or medical staff, wherein the holter (351) is further adapted for long distance transmission by means of the communication unit (275') through a network of mobile operator (898) and/or through a WiFi (131) to evaluation block (359) for displaying to medical staff and for permanent monitoring of monitored data live or monitoring of recorded data.

2. Live Holter according Claim 1 wherein control module (357), imaging module (358) and evaluating block (359) are provided with imaging elements (1) formed by displays and/or screens and are adapted for projection live or/and of record, monitoring of sensed data, values and courses optionally on their projection elements simultaneously or variously, or gradually namely especially arrhythmia, ECG, variability, curves of pulse with limits of arrhythmia.

3. Live Holter according Claim 1 wherein chest strap (749) is provided with monitor (349") of heart signals, general, with control buttons adjusted on it, chest strap (749') general provided also with contact surfaces (224) of chest strap and electrodes (143') external, whereas monitor (349‴) of heart signals is equipped with memory, especially SD card (888), for downloading of data into evaluating device (936).

4. Live Holter according Claim 1 wherein chest strap (749) consisting of monitor (349) of heart signals and sensors with SD card (888), to the inputs of it block front end (362) of ECG with electrodes (143) and block front end (362') with sensors (361) of processing of sensed signals are connected, whereas output of monitor (349") after digitization enters into communication unit (275') of communication with imaging module (378) directly, is placeable on chest strap (749") or out of it and sensing surfaces (224, 224') are in couples.

5. Live Holter according Claim 1 wherein control module (357) on bracelet (371) is adapted for control of monitor (349‴) of heart signals and is provided with display (370), control unit (753) with buttons ( 372), especially reset button (111) and panic button (110).

6. Live Holter according Claim 1 wherein monitor (349) of heart signals of chest strap (749) is connected by wireless link (882) with nearby cooperating units (901) in functions of control module (357) and/or imaging module (358) formed by mobile phone (100') with auxiliary functions adapted and/or mobile phone (100) with auxiliary device (104), whereas there are as backup to two couples of contact surfaces (224,224') assigned in monitor (349) two control units (736, 736') and two nearby cooperating units (901) with respective wireless link (882) in such a way that they are making two independent channels of backup.

7. Live Holter according Claim 1 wherein chest strap (749') general , provided with monitor (349‴), is wireless connected with control module (357) general and with imaging module (358), when control module (357) is formed by control unit (753) general, or mobile phone (100') adapted or mobile phone (100) standard with auxiliary device (104) and imaging module (358) for detail projection is formed by PC (889) and/or tablet (373) and/or mobile phone (100) standard, whereas communication between both modules is by wireless link.

8. Live Holter according Claim 1 wherein evaluating block (359) is formed by server (806), to which is connected by wire and/or wireless through PC (839) of operator and optionally also PC (839) of other remote participants when their connection is viable optionally, by means of internet.

## Patentansprüche

1. Holter-EKG
umfasst einen Holter-EKG (351) mit Steuermodul (357') und/oder Bildgebungsmodul (358) und optional einen Auswerteblock (359), wobei der Holter-EKG (351) einen Brustgurt (749'), eine Frontend-Einheit (362), einen Monitor für Herzsignale (349"), eine Kommunikationseinheit (257') umfasst, und der Holter-EKG angepasst ist, um analoge Herzsignale vom Brustgurt (749") zu erfassen und die Herzsignale in der Frontend-Einheit (362) zu verarbeiten und vom Monitor für Herzsignale (349") an die Kommunikationseinheit (275'), um die Holter-Auswertung zu mindestens einem der Folgenden zu übertragen: einem Steuermodul (357'), dem Bildgebungsmodul (358), dem Auswerteblock (359) und zur Übertragung zur Live-Anzeige oder zur Anzeige einer Aufzeichnung auf mindestens einem der Folgenden: dem Steuermodul (357"), dem Bildgebungsmodul (358), dem Auswerteblock (359) und der Auswerteblock (359) umfasst einen Server (806) und ferner einen Bediener-PC (839) und/oder einen Teilnehmer-PC (839'),
wobei der Monitor (349") für Herzsignale einen Speicher zur Datenaufzeichnung von Herzsignalen umfasst, der vorteilhafterweise durch eine SD-Karte (888) gebildet ist, und die Kommunikationseinheit (275') lokal mit dem Steuermodul (357') kommuniziert, um auf einer Anzeige (363) des Steuermoduls anzuzeigen, und/oder die Kommunikationseinheit (275') lokal mit dem Bildgebungsmodul (358) kommuniziert, um eine lokale Anzeige für Patienten und/oder medizinisches Personal durchzuführen, wobei der Holter-EKG (351) ferner für eine Fernübertragung mittels der Kommunikationseinheit (275') über ein Netzwerk eines Mobilfunkbetreibers (898) und/oder über WiFi (131) an den Auswertungsblock (359) zur Anzeige für medizinisches Personal und zur permanenten Überwachung überwachter Live-Daten oder zur Überwachung aufgezeichneter Daten angepasst ist.

2. Holter-EKG nach Anspruch 1
wobei
Steuermodul (357), Bildgebungsmodul (358) und Auswerteblock (359) mit durch Displays und/oder Bildschirme gebildeten Bildgebungselementen (1) versehen sind und zur Live-Projektion oder/und von Aufzeichnungen erfasster Überwachungsdaten, Werte und Verläufe wahlweise auf ihren Projektionselementen gleichzeitig oder unterschiedlich oder allmählich, nämlich insbesondere Arrhythmie, EKG, Variabilität, Pulskurven mit Grenzen der Arrhythmie, angepasst sind.

3. Holter-EKG nach Anspruch 1
wobei der Brustgurt (749) mit einem Monitor (349") für Herzsignale, allgemein, mit daran angepassten Steuertasten versehen ist, der Brustgurt (749') allgemein auch mit Kontaktflächen (224) des Brustgurts und externen Elektroden (143') versehen ist, während der Monitor (349‴) für Herzsignale mit einem Speicher, insbesondere einer SD-Karte (888), zum Herunterladen von Daten in die Auswertevorrichtung (936) ausgestattet ist.

4. Holter-EKG nach Anspruch 1
wobei der Brustgurt (749), der aus einem Monitor (349) für Herzsignale und Sensoren mit SD-Karte (888) besteht, mit dessen Eingängen das Block-Vorderende (362) des EKG mit Elektroden (143) und das Block-Vorderende (362') mit Sensoren (361) zur Verarbeitung der erfassten Signale verbunden sind, während der Ausgang des Monitors (349") nach der Digitalisierung direkt in die Kommunikationseinheit (275') zur Kommunikation mit dem Bildgebungsmodul (378) eintritt, auf dem Brustgurt (749") oder außerhalb von diesem platzierbar ist und die Erfassungsflächen (224, 224') paarweise vorhanden sind.

5. Holter-EKG nach Anspruch 1
wobei das Steuermodul (357) auf dem Armband (371) zur Steuerung des Monitors (349‴) für Herzsignale angepasst ist und mit einer Anzeige (370), einer Steuereinheit (753) mit Tasten (372), insbesondere einer Reset-Taste (111) und einer Paniktaste (110), ausgestattet ist.

6. Holter-EKG nach Anspruch 1
wobei der Monitor (349) für Herzsignale des Brustgurts (749) durch eine drahtlose Verbindung (882) mit nahegelegenen kooperierenden Einheiten (901) in Funktionen eines Steuermoduls (357) und/oder eines Bildgebungsmoduls (358) verbunden ist, das durch ein Mobiltelefon (100') mit angepassten Hilfsfunktionen und/oder ein Mobiltelefon (100) mit einer Hilfsvorrichtung (104) gebildet ist, während zwei Paare von, dem Monitor (349) zugeordneten Kontaktflächen (224, 224'), zwei Steuereinheiten (736, 736') und zwei nahegelegene kooperierende Einheiten (901) mit einer jeweiligen drahtlosen Verbindung (882) als Backup vorhanden sind, derart, dass sie zwei unabhängige Backup-Kanäle bilden.

7. Holter-EKG nach Anspruch 1
wobei der Brustgurt (749') allgemein, der mit dem Monitor (349‴) versehen ist, drahtlos mit dem Steuermodul (357) allgemein und mit dem Bildgebungsmodul (358) verbunden ist, wenn das Steuermodul (357) durch die Steuereinheit (753) allgemein gebildet ist, oder das angepasste Mobiltelefon (100') oder das Standard-Mobiltelefon (100) mit der Hilfsvorrichtung (104) und das Bildgebungsmodul (358) für die Detailprojektion durch einen PC (889) und/oder ein Tablet (373) und/oder ein Standard-Mobiltelefon (100) gebildet ist, während die Kommunikation zwischen beiden Modulen über eine drahtlose Verbindung erfolgt.

8. Holter-EKG nach Anspruch 1
wobei der Auswerteblock (359) durch den Server (806) gebildet ist, mit dem drahtgebunden und/oder drahtlos über den PC (839) des Bedieners und optional auch den PC (839) anderer entfernter Teilnehmer verbunden ist, wenn deren Verbindung optional mittels Internet möglich ist.

## Revendications

1. Holter en temps réel
comprend un holter (351) avec un module de commande (357') et/ou un module d'imagerie (358) et éventuellement un bloc d'évaluation (359), le holter (351) comprenant une ceinture thoracique (749'), unité frontale (362), un cardiofréquencemètre (349"), une unité de communication (257'), et le holter en temps réel est adapté pour capter les signaux cardiaques analogiques de la ceinture thoracique (749") et traiter les signaux cardiaques dans l'unité frontale (362) et du cardiofréquencemètre (349") à l'unité de communication (275') pour transmission pour évaluation Holter dans au moins l'un des suivants: un module de commande (357'), le module d'imagerie (358), le bloc d'évaluation (359) et pour transmission pour affichage en temps réel ou à partir d'un enregistrement sur au moins l'un des suivants: le module de commande (357"), le module d'imagerie (358), le bloc d'évaluation (359), le bloc d'évaluation (359) comprend un serveur (806) et comprend en outre le PC de l'opérateur (839) et/ou le PC du participant (839'),
le cardiofréquencemètre (349") comprenant une mémoire pour l'enregistrement de données de signaux cardiaques, avantageusement formée par une carte SD (888), et l'unité de communication (275') communique localement avec le module de commande (357'), pour afficher sur un afficheur (363) du module de commande et/ou l'unité de communication (275') communique localement avec le module d'imagerie (358), un afficheur local est destiné au patient et/ou au personnel médical, le holter (351) étant en outre adapté pour la transmission à longue distance au moyen de l'unité de communication (275') à travers un réseau d'opérateur mobile (898) et/ou à travers WiFi (131) au bloc d'évaluation (359) pour afficher pour le personnel médical et pour la surveillance permanente des données surveillées en temps réel ou la surveillance des données enregistrées.

2. Holter en temps réel selon la revendication 1,
le module de contrôle (357), le module d'imagerie (358) et le bloc d'évaluation (359) étant pourvus d'éléments d'imagerie (1) formés d'affichages et/ou d'écrans et sont adaptés pour la projection en temps réel ou/et d'enregistrement, la surveillance des données captées, des valeurs et des cours éventuellement sur leurs éléments de projection simultanément ou diversement, ou progressivement à savoir notamment l'arythmie, l'ECG, la variabilité, les courbes de pouls avec des limites d'arythmie.

3. Holter en temps réel selon la revendication 1,
la ceinture thoracique (749) étant munie d'un cardiofréquencemètre (349"), général, avec boutons de commande réglés dessus, la ceinture thoracique (749') générale est munie également de surfaces de contact (224) de la ceinture thoracique et d'électrodes (143') externes, tandis que le cardiofréquencemètre (349‴) est équipé de mémoire, en particulier de carte SD (888), pour le téléchargement des données dans le dispositif d'évaluation (936).

4. Holter en temps réel selon la revendication 1,
la ceinture thoracique (749) composée d'un cardiofréquencemètre (349) et de capteurs avec la carte SD (888), aux entrées de celui-ci, l'extrémité avant bloc (362) de l'ECG avec des électrodes (143) et l'extrémité avant bloc (362') avec des capteurs (361) de traitement des signaux détectés sont connectés, tandis que la sortie du cardiofréquencemètre (349") après numérisation entre dans l'unité de communication (275') de communication avec le module d'imagerie (378) directement, est plaçable sur la ceinture thoracique (749") ou hors de celle-ci et les surfaces de détection (224, 224') sont en couple.

5. Holter en temps réel selon la revendication 1,
le module de commande (357) sur un bracelet (371) étant adapté pour le contrôle du cardiofréquencemètre (349‴) et est muni d'un affichage (370), d'une unité de commande (753) avec des boutons (372), en particulier un bouton de réinitialisation (111) et un bouton panique (110).

6. Holter en temps réel selon la revendication 1,
le cardiofréquencemètre (349) de la ceinture thoracique (749) étant connecté par liaison sans fil (882) avec des unités coopérantes voisines (901) en fonction du module de commande (357) et/ou du module d'imagerie (358) formé par le téléphone mobile (100') avec des fonctions auxiliaires adaptées et/ou le téléphone mobile (100) avec dispositif auxiliaire (104), alors qu'il y a en secours à deux couples de surfaces de contact (224, 224') assignées dans le cardiofréquencemètre (349) deux unités de commande (736, 736') et deux unités coopérantes voisines (901) avec liaison sans fil respective (882) de telle sorte qu'elles font deux canaux de secours indépendants.

7. Holter en temps réel selon la revendication 1,
la ceinture thoracique (749') générale, munie d'un cardiofréquencemètre (349‴), étant connectée sans fil avec le module de commande (357) général et avec le module d'imagerie (358), lorsque le module de commande (357) est formé par l'unité de commande (753) générale, ou le téléphone mobile (100') adapté ou le téléphone mobile (100) standard avec dispositif auxiliaire (104) et le module d'imagerie (358) pour la projection de détails est formé par le PC (889) et/ou la tablette (373) et/ou le téléphone mobile (100) standard, alors que la communication entre les deux modules se fait par liaison sans fil.

8. Holter en temps réel selon la revendication 1,
le bloc d'évaluation (359) étant formé par le serveur (806), auquel est connecté par fil et/ou sans fil par le PC (839) de l'opérateur et éventuellement aussi le PC (839) d'autres participants distants lorsque leur connexion est viable en option, au moyen d'internet.
